# EUROPEAN PATENT APPLICATION

(11) **EP 4 059 929 A1**
(43) Date of publication of application: **21.09.2022**
(21) Application number: 20886669.9
(22) Date of filing: 13.11.2020
(51) Int. Cl.: C07D 401/14, A61K 31/496, A61K 31/506, A61K 31/5513, C07D 405/14, A61P 3/10

(54) **GLP-1 RECEPTOR AGONIST AND USE THEREOF**

(30) Priority: 15.11.2019 KR 20190146798; 24.02.2020 KR 20200022485
(71) Applicant: Ildong Pharmaceutical Co., Ltd., Seoul 06752 (KR)
(72) Inventor: YOON, Hong Chul, Hwaseong-si Gyeonggi-do 18449 (KR); AN, Kyung Mi, Hwaseong-si Gyeonggi-do 18449 (KR); LEE, Myong Jae, Hwaseong-si Gyeonggi-do 18449 (KR); LEE, Jin Hee, Hwaseong-si Gyeonggi-do 18449 (KR); KIM, Jeong-geun, Hwaseong-si Gyeonggi-do 18449 (KR); IM, A-rang, Hwaseong-si Gyeonggi-do 18449 (KR); JEON, Woo Jin, Hwaseong-si Gyeonggi-do 18449 (KR); JEONG, Jin Ah, Hwaseong-si Gyeonggi-do 18449 (KR); HEO, Jaeho, Hwaseong-si Gyeonggi-do 18449 (KR); HONG, Changhee, Hwaseong-si Gyeonggi-do 18449 (KR); KIM, Kyeojin, Hwaseong-si Gyeonggi-do 18449 (KR); PARK, Jung-eun, Hwaseong-si Gyeonggi-do 18449 (KR); SOHN, Te-ik, Hwaseong-si Gyeonggi-do 18449 (KR); OH, Changmok, Hwaseong-si Gyeonggi-do 18449 (KR); HONG, Da Hae, Hwaseong-si Gyeonggi-do 18449 (KR); KWON, Sung Wook, Hwaseong-si Gyeonggi-do 18449 (KR); KIM, Jung Ho, Hwaseong-si Gyeonggi-do 18449 (KR); SHIN, Jae Eui, Hwaseong-si Gyeonggi-do 18449 (KR); YOO, Yeongran, Hwaseong-si Gyeonggi-do 18449 (KR); CHANG, Min Whan, Hwaseong-si Gyeonggi-do 18449 (KR); JANG, Eun Hye, Hwaseong-si Gyeonggi-do 18449 (KR); JE, In-gyu, Hwaseong-si Gyeonggi-do 18449 (KR); CHOI, Ji Hye, Hwaseong-si Gyeonggi-do 18449 (KR); KIM, Gunhee, Hwaseong-si Gyeonggi-do 18449 (KR); JUN, Yearin, Hwaseong-si Gyeonggi-do 18449 (KR)
(74) Representative: Boult Wade Tennant LLP
(86) International application number: PCT/KR2020/015985
(87) International publication number: WO 2021/096284

(57) **Abstract**

The present invention provides novel compounds of chemical formula 1, optical isomers of the compounds, or a pharmaceutically acceptable salts of the compounds, or the optical isomers. The compounds, isomers, and salts exhibits excellent activity as GLP-1 receptor agonists. Particularly, they, as GLP-1 receptor agonists, exhibit excellent glucose tolerance, thus having a great potential to be used therapeutic agents for metabolic diseases. In addition, they exhibits excellent pharmacological safety for cardiovascular systems.

## Description

### [Technical field]

The present application provides novel GLP-1R agonist compounds and uses of the compounds.

### [Background Art]

Insulin is a peptide secreted by beta cells of pancreas and is a substance that plays a very important role in regulating blood sugar in a body. Diabetes is a metabolic disease in which a concentration of glucose in the blood increases due to insufficient secretion of insulin or normal functioning thereof. A case in which blood sugar rises due to inability to secrete insulin from the pancreas is referred to as type 1 diabetes. Thus, administration of insulin is required to treat the type 1 diabetes.

On the other hand, when insulin secretion is insufficient or the secreted insulin does not work properly and thus the blood sugar in the body is not controlled and rises, this is referred to as type 2 diabetes, which it is treated using a hypoglycemic agent based on a chemical substance.

It is well known based on large-scale clinical studies that strict blood sugar control toward a normal blood sugar level in diabetes treatment is important for preventing various complications caused by the diabetes.

A candidate compound that may lower the blood sugar by strongly stimulating the secretion of insulin includes a hormone referred to as glucagon like peptide-1 (GLP-1). GLP-1 was first discovered in 1985 as an incretin hormone secreted by L-cells in ileum and colon. GLP-1 increases insulin secretion by acting on a receptor referred to as GLP-1R (glucagon like peptide-1 receptor). GLP-1 is secreted via stimulation by absorbed nutrients or blood sugar levels. Diabetes treatment using GLP-1 has advantages that hypoglycemia does not occur because insulin is secreted depending on the glucose concentration. In addition, this hormone is known to be effective in reducing movement of an upper digestive system and suppressing appetite, and to proliferate existing beta cells of the pancreas.

Due to those characteristics thereof, GLP-1 was a candidate compound which was applied for a treatment method for the type 2 diabetes, but it had many obstacles in developing the same as a drug because a half-life in blood thereof was only 2 minutes. To overcome the shortcomings of GLP-1 due to the short action time, recently, therapeutic agents have been developed using a GLP-1 analog and a DPP-4 inhibitor which are resistant to an enzyme referred to as dipeptidyl peptidase IV (DPP-IV) that destroys the GLP-1 in the blood (Oh, SJ "Glucagon-like Peptide-1 Analogue and Dipeptidyl Peptidase-IV Inhibitors" Journal of the Korean Endocrine Society Vol. 21(6), pp. 437-447, 2006; Holst, J. J. "Glucagon like peptide 1: a newly discovered gastrointestinal hormone" Gastroenterology Vol. 107, pp. 1848-1855, 1994).

Among insulin-secreting peptides other than GLP-1, exendin is a peptide found in the salivary secretions of the Mexican beaded lizard *(Heroderma horridum)* and the Gila monster *(Helloderma suspectum)* as endogenous reptiles of Arizona and North Mexico. Exendin-3 is present in the salivary secretions of *Heroderma horridum,* and exendin-4 is present in the salivary secretions of *Helloderma suspectum,* and both have high homology with the GLP-1 sequence (Goke et al., J. Biol. Chem. Vo1. 268, pp. 19650-19655, 1993). Pharmacological research reports state that exendin-4 may act on GLP-1 receptors on specific insulin-secreting cells, dispersed racemose gland cells from the pancreas of guinea pigs and stomach walls cells thereof. This peptide has been reported to stimulate somatostatin release and inhibit gastrin release from the isolated stomach.

Currently, various GLP-1 analogs having resistance to the DPP-4 enzyme that destroys GLP-1 in blood have been developed and are being used as a therapeutic agent for type 2 diabetes. These GLP-1 analogs have a considerably longer half-life compared to GLP-1, and thus they have the advantage of maintaining a hypoglycemic effect for a long time. However, oral administration thereof is not possible, resulting in low medication convenience in that they must be used in a form of an injection. Therefore, recently, research for discovering a small molecule GLP-1R agonist that may be administered orally and may be used as a diabetes treatment agent is being conducted. Recently, in Korea, it has been reported that DA-15864 as a novel small molecule compound that may selectively stimulate the GLP-1 receptor in humans and mice acts as a GLP-1 receptor agonist that may be administered orally to treat diabetes and obesity (Moon, H.-S. et al., "The development of non-peptide glucagon-like peptide 1 receptor agonist for the treatment of type 2 diabetes" Arch. Pharm. Res. Vol. 34(7), pp. 1041 - 1043, 2011). These oral formulations have high development value in that they act as GLP-1R agonists with improved easiness of administration.

Further, regulatory authorities such as US FDA are paying attention to cardiovascular side effects of drugs that may cause sudden death, especially QT prolongation and delayed ventricular repolarization. The pharmacological study of the cardiovascular safety of the novel substance is being emphasized. In this regard, human ether-a-go-go related gene (hERG) is a gene that encodes a subunit of the human potassium channels responsible for the delayed rectifier potassium current (IKr), which seems to have the most influential role in determining the duration of the action potential and thus the QT interval. If the hERG channel is inhibited by drugs, the ventricular repolarization determined by the duration of the cardiac action potential is delayed, an effect that can be measured as prolongation of the QT interval on the ECG. This has been associated with cardiotoxicity such as cardiac arrhythmia including Torsade de pointes (TdP). New pharmaceutical candidates should be assessed in terms of inhibition of the hERG channels which have a significant impact on QT prolongation with regard to cardiovascular adverse events. In this process, most of the drugs have an effect on the inhibition of the hERG channels, and thus a development process thereof may stop.

Particularly, in the development of the diabetes medicines, QT prologation is an essential consideration. In the case of diabetes, the cause of death due to ischemic heart disease increased by 2 to 3 times or more. Women diagnosed with diabetes before age 30 are known to have a significant increased risk of myocardial infarction or fatal coronary artery disease. Thus, if an anti-diabetic drug causes the QT prolongation, it is difficult to develop the drug itself with an inevitable limitation for long-term use, even if it has an excellent effect.

Under this circumstance, novel agonist compounds having an excellent effect of increasing the activity of the GLP-1 receptor were screened from various candidate substances in the present disclosure, and it was confirmed that these compounds exhibit excellent activity as GLP-1 receptor agonists.

### [Disclosure]

### [Technical Purpose]

The present disclosure provides compounds represented by the following Chemical Formula 1, optical isomers of the compounds, or pharmaceutically acceptable salts of the compounds or the optical isomers: 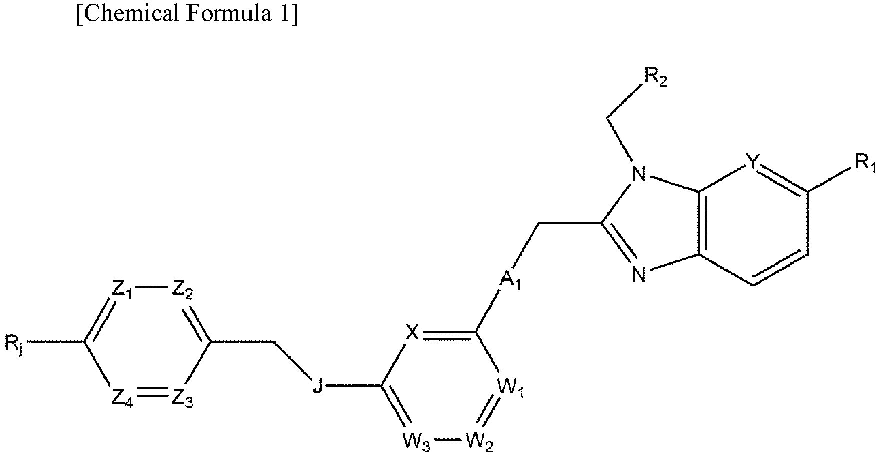
wherein R₁ is -C(=O)Rₐ;
Rₐ is -OH or -O-(C₁-C₄ alkyl);
Y is -CH- or -N-;
R₂ is one selected from the group consisting of substituted or unsubstituted C₆ to C₁₂ aryl, substituted or unsubstituted C₅ to C₁₂ heteroaryl, substituted or unsubstituted C₃ to C₈ heterocycloalkyl, and substituted or unsubstituted C₃ to C₈ cycloalkyl, where the substituted aryl, heteroaryl, heterocycloalkyl, and cycloalkyl include at least one substitution with -OH, -(C₁-C₄ alkyl), halogen, or -CN;
A₁ is 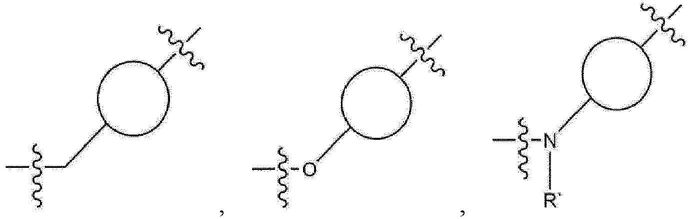 or 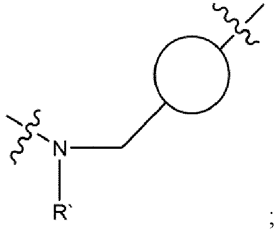 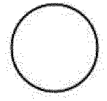 is substituted or unsubstituted C₃ to C₈ heterocycloalkyl containing at least one nitrogen, substituted or unsubstituted C₃ to C₁₂ spiroheterocycloalkyl containing at least one nitrogen, or substituted or unsubstituted C₃ to C₁₂ bridged heterobicycloalkyl containing at least one nitrogen, where the substituted heterocycloalkyl, spiroheterocycloalkyl, and heterobicycloalkyl include at least one substitution with -OH, -(C₁-C₄ alkyl), halogen, or -CN;
R' is hydrogen or -(C₁-C₄ alkyl);
X is -CR_{b}- or -N-;
R_{b} is one selected from the group consisting of -H, halogen, -CN, -OH, -O-(C₁-C₄ alkyl), -NH₂, -NO₂, and -C₁-C₄ haloalkyl;
W₁ is -CR_{c}- or -N-, where R_{c} is one selected from the group consisting of -H, halogen, -CN, -OH, -O-(C₁-C₄ alkyl), -NH₂, -NO₂, and -C₁-C₄ haloalkyl;
W₂ is -CR_{d}- or -N-, where R_{d} is one selected from the group consisting of -H, halogen, -CN, -OH, -O-(C₁-C₄ alkyl), -NH₂, -NO₂, and -C₁-C₄ haloalkyl;
W₃ is -CRₑ- or -N-, where Rₑ is one selected from the group consisting of -H, halogen, -CN, -OH, -O-(C₁-C₄ alkyl), -NH₂, -NO₂, and -C₁-C₄ haloalkyl;
J is -O- or -NR"-;
R" is hydrogen or -(C₁-C₄ alkyl);
Z₁ is -CR_{f}- or -N-, where R_{f} is one selected from the group consisting of -H, halogen, -CN, -OH, -O-(C₁-C₄ alkyl), -NH₂, -NO₂, and -C₁-C₄ haloalkyl;
Z₂ is -CR_{g}- or -N-, where R_{g} is one selected from the group consisting of -H, halogen, -CN, -OH, -O-(C₁-C₄ alkyl), -NH₂, -NO₂, and -C₁-C₄ haloalkyl;
Z₃ is -CRₕ- or -N-, where Rₕ is one selected from the group consisting of -H, halogen, -CN, -OH, -O-(C₁-C₄ alkyl), -NH₂, -NO₂, and -C₁-C₄ haloalkyl;
Z₄ is -CRᵢ- or -N-, where Rᵢ is one selected from the group consisting of -H, halogen, -CN, -OH, -O-(C₁-C₄ alkyl), -NH₂, -NO₂, and -C₁-C₄ haloalkyl; and
Rⱼ is one selected from the group consisting of -H, halogen, -CN, -OH, -O-(C₁-C₄ alkyl), -NH₂, -NO₂, and -C₁-C₄ haloalkyl.

Specifically, in the Chemical Formula 1, Z₁ may be -CR_{f}- or -N-; Z₂ may be -CR_{g}-or -N-; Z₃ may be -CRₕ- or -N-; Z₄ may be -CRᵢ- or -N-, where only one of Z₁ to Z₄ may be -N-.

Specifically, in the Chemical Formula 1, Z₁ may be -CR_{f}-, Z₂ may be -CR_{g}-, Z₃ may be -CRₕ-, and Z₄ may be -CRi-.

More specifically, the compound represented by Chemical Formula 1, the optical isomer of the compound, or the pharmaceutically acceptable salt of the compound or the optical isomer may be a compound represented by the following Chemical Formula 2:

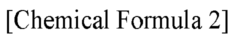

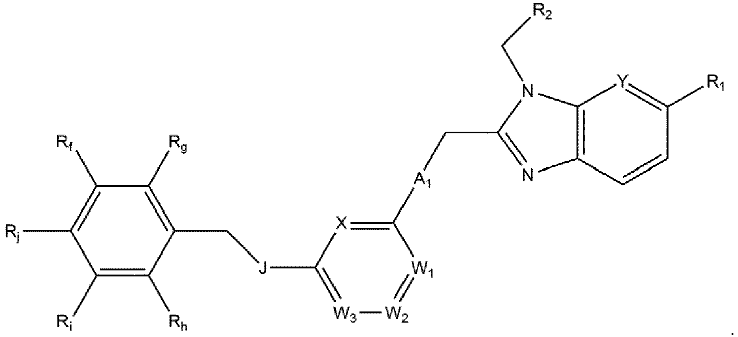

In the compound of the Chemical Formula 2, R_{f}, R_{g}, Rₕ, Rᵢ, Rⱼ, J, X, W₁, W₂, W₃, A₁, R₂, Y, and R₁ are the same as previously defined in the above Chemical Formula 1.

Specifically, in the Chemical Formula 1, Rⱼ may be halogen or -CN.

Specifically, in the Chemical Formula 1, R_{g} may be one selected from the group consisting of -H, halogen and -CN.

Specifically, in the Chemical Formula 1, J may be -O-

More specifically, the compound represented by Chemical Formula 1, the optical isomer of the compound, or the pharmaceutically acceptable salt of the compound or the optical isomer may be a compound represented by the following Chemical Formula 3:

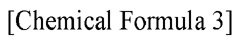

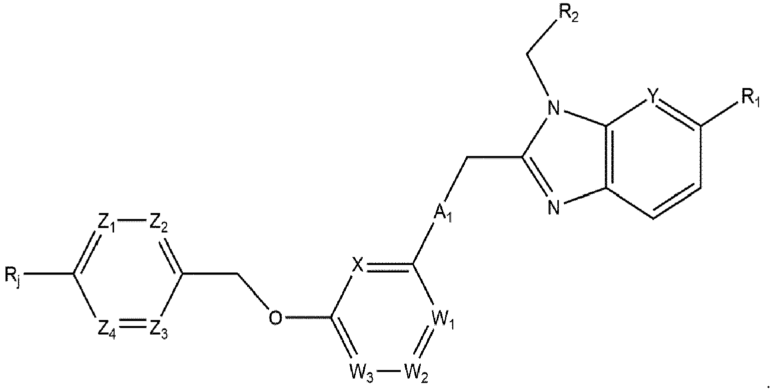

In the compound of the Chemical Formula 3, Z₁, Z₂, Z₃, Z₄, Rⱼ, X, W₁, W₂, W₃, A₁, R₂, Y, and R₁ are the same as previously defined in the above Chemical Formula 1.

Specifically, in the Chemical Formula 1, A₁ may be

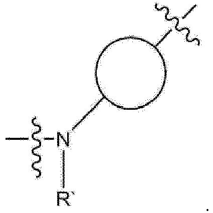

More specifically, the compound represented by Chemical Formula 1, the optical isomer of the compound, or the pharmaceutically acceptable salt of the compound or the optical isomer may be a compound represented by the following Chemical Formula 4:

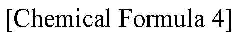

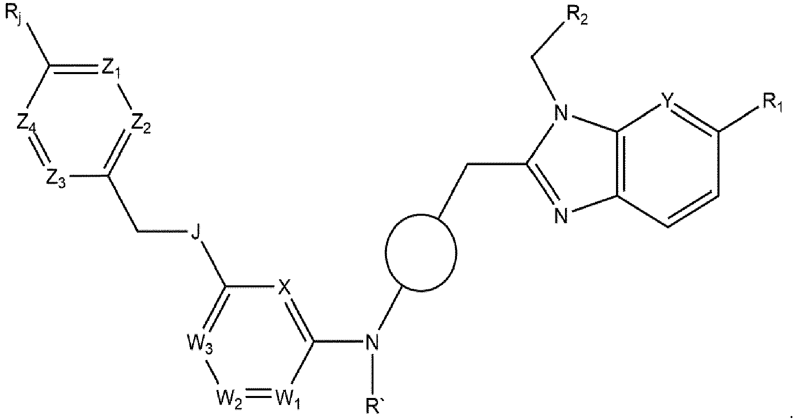

In the compound of the Chemical Formula 4, Z₁, Z₂, Z₃, Z₄, Rⱼ, J, X, W₁, W₂, W₃, R', 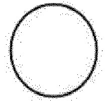 R₂, Y, and R₁ are the same as previously defined in the above Chemical Formula 1. Specifically, in the Chemical Formula 1, 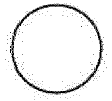 may be substituted or unsubstituted C₃ to C₈ heterocycloalkyl containing at least one nitrogen, where the substituted heterocycloalkyl includes at least one substitution with -OH, -(C₁-C₄ alkyl), halogen, or -CN.

Specifically, in the Chemical Formula 1, 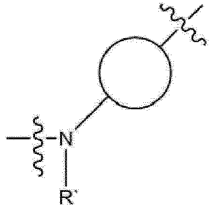 may be one selected from the group consisting of substituted or unsubstituted 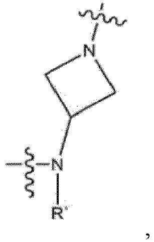 substituted or unsubstituted 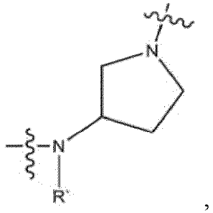 and substituted or unsubstituted 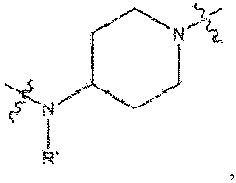 where the substituted 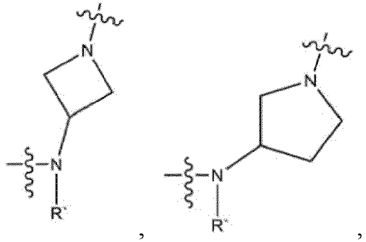 and 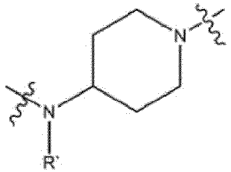 include at least one substitution with -OH, -(C₁-C₄ alkyl), halogen, or -CN.

Specifically, in the Chemical Formula 1, A₁ may be

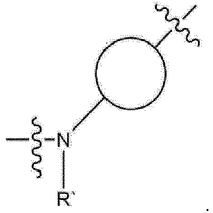

More specifically, the compound of Chemical Formula 1, the optical isomer of the compound, or the pharmaceutically acceptable salt of the compound or the optical isomer may be a compound represented by the following Chemical Formula 5-1 when 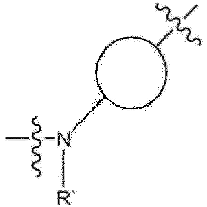 is substituted or unsubstituted

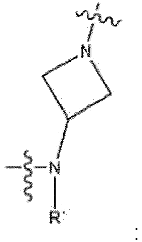

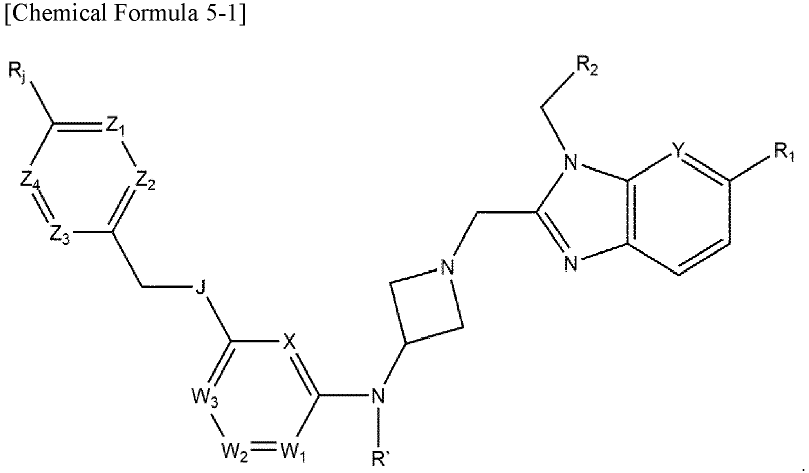

More specifically, the compound of Chemical Formula 1, the optical isomer of the compound, or the pharmaceutically acceptable salt of the compound or the optical isomer may be a compound represented by the following Chemical Formula 5-2 when 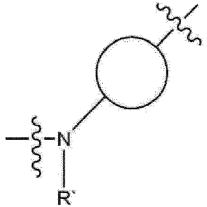 is substituted or unsubstituted

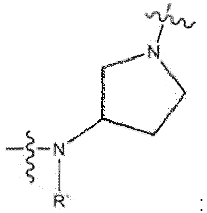

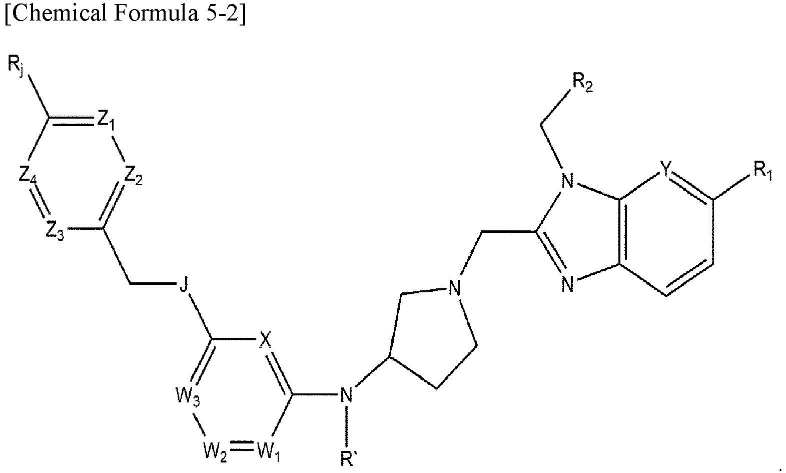

More specifically, the compound of Chemical Formula 1, the optical isomer of the compound, or the pharmaceutically acceptable salt of the compound or the optical isomer may be a compound represented by the following Chemical Formula 5-3 when 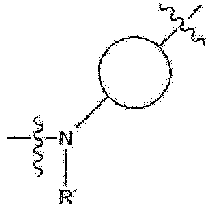 is substituted or unsubstituted

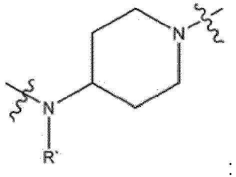

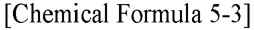

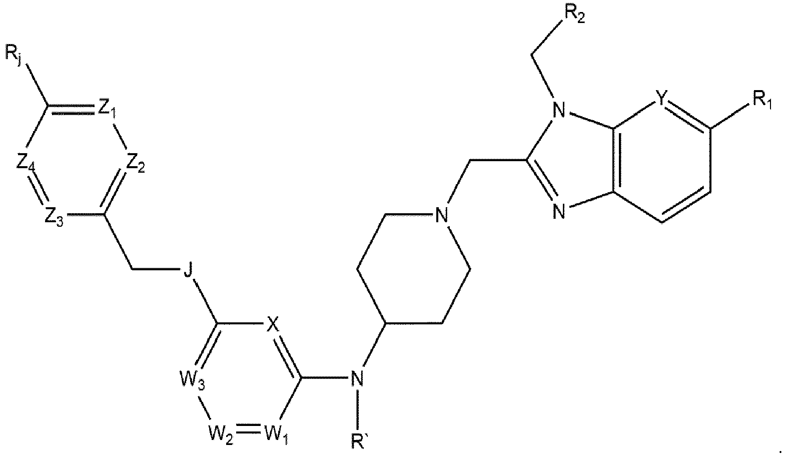

In the compounds of the Chemical Formulas 5-1, 5-2, and 5-3, Z₁, Z₂, Z₃, Z₄, Rⱼ, J, X, W₁, W₂, W₃, R', R₂, Y, and R₁ are the same as previously defined in the above Chemical Formula 1.

Specifically, in the Chemical Formula 1, R₂ may be substituted or unsubstituted C₃ to C₅ heterocycloalkyl or substituted or unsubstituted C₃ to C₅ cycloalkyl, where the substituted heterocycloalkyl and cycloalkyl include at least one substitution with -OH, - (C₁-C₄ alkyl), halogen, or -CN.

Specifically, in the Chemical Formula 1, R₂ may be substituted or unsubstituted 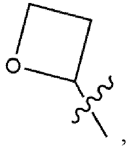 where the substituted 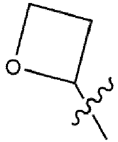 includes at least one substitution with - OH, -(C₁-C₄ alkyl), halogen, or -CN.

More specifically, the compound of Chemical Formula 1, the optical isomer of the compound, or the pharmaceutically acceptable salt of the compound or the optical isomer may be a compound represented by the following Chemical Formula 6:

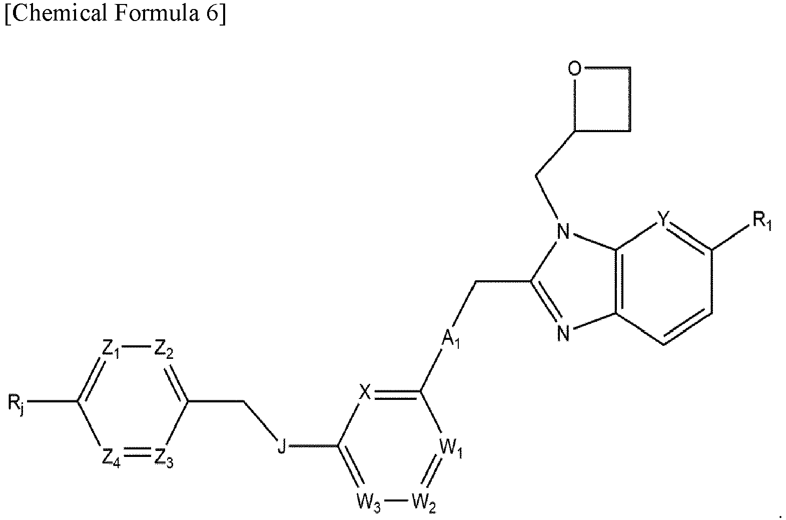

In the compound of the Chemical Formula 6, Z₁, Z₂, Z₃, Z₄, Rⱼ, J, X, W₁, W₂, W₃, A₁, Y, and R₁ are the same as previously defined in the above Chemical Formula 1.

In another embodiment, the present disclosure provides the compound listed below, an optical isomer of the compound, or a pharmaceutically acceptable salt of the compound or the optical isomer:
(S)-2-((4-((6-((4-cyano-2-fluorobenzyl)oxy)pyridin-2-yl)amino)piperidin-1-yl)methyl)-1-(oxetan-2-ylmethyl)-1*H*-benzo[d]imidazole-6-carboxylic acid;
(S)-2-((4-((3-((4-cyano-2-fluorobenzyl)oxy)phenyl)amino)piperidin-1-yl)methyl)-1-(oxetan-2-ylmethyl)-1*H*-benzo[d]imidazole-6-carboxylic acid;
(S)-2-((4-((4-((4-cyano-2-fluorobenzyl)oxy)-5-fluoropyrimidin-2-yl)amino)piperidin-1-yl)methyl)-1-(oxetan-2-ylmethyl)-1*H*-benzo[d]imidazole-6-carboxylic acid;
(S)-2-((4-((2-((4-cyano-2-fluorobenzyl)oxy)pyrimidin-4-yl)amino)piperidin-1-yl)methyl)-1-(oxetan-2-ylmethyl)-1*H*-benzo[d]imidazole-6-carboxylic acid;
(S)-2-((4-((6-((4-chloro-2-fluorobenzyl)amino)pyridin-2-yl)oxy)piperidin-1-yl)methyl)-1-(oxetan-2-ylmethyl)-1*H*-benzo[d]imidazole-6-carboxylic acid;
(S)-2-((4-((3-((5-cyanopyridin-2-yl)methoxy)-4-fluorophenyl)amino)piperidin-1-yl)methyl)-1-(oxetan-2-ylmethyl)-1*H*-benzo[d]imidazole-6-carboxylic acid;
(S)-2-((4-((3-((5-chloropyridin-2-yl)methoxy)phenyl)amino)piperidin-1-yl)methyl)-1-(oxetan-2-ylmethyl)-1*H*-benzo[d]imidazole-6-carboxylic acid;
2-(((R)-3-((3-((5-cyanopyridin-2-yl)methoxy)-4-fluorophenyl)amino)pyrrolidin-1-yl)methyl)-1-(((*S*)-oxetan-2-yl)methyl)-1*H*-benzo[d]imidazole-6-carboxylic acid;
2-(((R)-3-((3-((5-cyanopyridin-2-yl)methoxy)-4-fluorophenyl)amino)pyrrolidin-1-yl)methyl)-3-(((*S*)-oxetan-2-yl)methyl)-3*H*-imidazo[4,5-b]pyridine-5-carboxylicacid;
2-(((R)-3-((3-((4-cyano-2-fluorobenzyl)oxy)-4-fluorophenyl)amino)pyrrolidin-1-yl)methyl)-1-(((*S*)-oxetan-2-yl)methyl)-1*H*-benzo[d]imidazole-6-carboxylic acid;
(*S*)-2-((4-((3-((4-cyano-2-fluorobenzyl)oxy)-4-fluorophenyl)amino)piperidin-1-yl)methyl)-1-(oxetan-2-ylmethyl)-1*H*-benzo[d]imidazole-6-carboxylic acid;
(*S*)-2-((4-((6-((4-chloro-2-fluorobenzyl)oxy)pyridin-2-yl)amino)piperidin-1-yl)methyl)-1-(oxetan-2-ylmethyl)-1*H*-benzo[d]imidazole-6-carboxylic acid;
(*S*)-2-((4-((6-((4-chloro-2-fluorobenzyl)oxy)pyridin-2-yl)amino)piperidin-1-yl)methyl)-3-oxetan-2-ylmethyl)-3*H*-imidazo[4,5-b]pyridine-5-carboxylic acid;
(*S*)-2-((3-(((6-((4-cyano-2-fluorobenzyl)oxy)pyridin-2-yl)amino)methyl)azetidin-1-yl)methyl)-1-(oxetan-2-ylmethyl)-1*H*-benzo[d]imidazole-6-carboxylic acid;
2-(((*R*)-3-((6-((4-chloro-2-fluorobenzyl)oxy)pyridin-2-yl)amino)pyrrolidin-1-yl)methyl)-1-(((*S*)-oxetan-2-yl)methyl)-1*H*-benzo[d]imidazole-6-carboxylic acid;
2-(((*R*)-3-((6-((4-chloro-2-fluorobenzyl)oxy)pyridin-2-yl)amino)pyrrolidin-1-yl)methyl)-3-(((*S*)-oxetan-2-yl)methyl)-3*H*-imidazo[4,5-*b*]pyridine-5-carboxylic acid;
(*S*)-2-((4-((4-((4-cyano-2-fluorobenzyl)oxy)pyrimidin-2-yl)amino)piperidin-1-yl)methyl)-1-(oxetan-2-ylmethyl)-1*H*-benzo[d]imidazole-6-carboxylic acid;
2-((2-(((6-((4-cyano-2-fluorobenzyl)oxy)pyridin-2-yl)amino)methyl)pyrrolidin-1-yl)methyl)-1-(ethyl)-1*H*-benzo[d]imidazole-6-carboxylic acid;
(*S*)-2-((4-((6-((4-cyano-2-fluorobenzyl)oxy)pyrazin-2-yl)amino)piperidin-1-yl)methyl)-1-(oxetan-2-ylmethyl)-1*H*-benzo[d]imidazole-6-carboxylic acid;
2-((3-((6-((4-cyano-2-fluorobenzyl)oxy)pyridin-2-yl)amino)pyrrolidin-1-yl)methyl)-1-(((*S*)-oxetan-2-yl)methyl)-1*H*-benzo[d]imidazole-6-carboxylic acid;
2-(((*S*)-3-((6-((4-cyano-2-fluorobenzyl)oxy)pyridin-2-yl)amino)pyrrolidin-1-yl)methyl)-1-(((*S*)-oxetan-2-yl)methyl)-1*H*-benzo[d]imidazole-6-carboxylic acid;
2-(((*R*)-3-((4-((4-cyano-2-fluorobenzyl)oxy-5-fluoropyrimidin-2-yl)amino)pyrrolidin-1-yl)methyl)-1-(((*S*)-oxetan-2-yl)methyl)-1*H*-benzo[*d*]imidazole-6-carboxylic acid;
2-(((*R*)-3-((2-((4-cyano-2-fluorobenzyl)oxy)pyrimidin-4-yl)amino)pyrrolidin-1-yl)methyl)-1-(((*S*)-oxetan-2-yl)methyl)-1*H*-benzo[d]imidazole-6-carboxylic acid;
2-((3-((6-((4-cyano-2-fluorobenzyl)oxy)pyridin-2-yl)methyl)pyrrolidin-1-yl)methyl)-1-(((*S*)-oxetan-2-yl)methyl)-1*H*-benzo[d]imidazole-6-carboxylic acid;
2-(((*R*)-3-((3-((4-chloro-2-fluorobenzyl)oxy)-4-fluorophenyl)amino)pyrrolidin-1-yl)methyl)-1-(ethyl)-1*H*-benzo[d]imidazole-6-carboxylic acid;
2-(((*R*)-3-((3-((4-chloro-2-fluorobenzyl)oxy)-4-fluorophenyl)amino)pyrrolidin-1-yl)methyl)-3-(((*S*)-oxetan-2-yl)methyl)-3*H*-imidazo [4,5-*b*] pyridine-5-carboxylic acid;
(S)-2-((4-((3-((4-chloro-2-fluorobenzyl)oxy)-4-fluorophenyl)amino)piperidin-1-yl)methyl)-1-(oxetan-2-ylmethyl)-1*H*-benzo[d]imidazole-6-carboxylic acid;
(*S*)-2-((4-((3-((4-chloro-2-fluorobenzyl)oxy)-4-fluorophenyl)amino)piperidin-1-yl)methyl)-3-(oxetan-2-ylmethyl)-3*H*-imidazo[4,5-b]pyridine-5-carboxylic acid;
2-(((*R*)-3-((4-((4-chloro-2-fluorobenzyl)oxy-5-fluoropyrimidin-2-yl)amino)pyrrolidin-1-yl)methyl)-1-(((*S*)-oxetan-2-yl)methyl)-1*H*-benzo[*d*]imidazole-6-carboxylic acid;
2-(((*R*)-3-((6-((4-chloro-2-fluorobenzyl)oxy)pyrazin-2-yl)amino)pyrrolidin-1-yl)methyl)-1-(((*S*)-oxetan-2-yl)methyl)-1*H*-benzo[d]imidazole-6-carboxylic acid;
2-(((R)-3-((2-((4-chloro-2-fluorobenzyl)oxy-5-fluoropyrimidin-4-yl)amino)pyrrolidin-1-yl)methyl)-1-(((*S*)-oxetan-2-yl)methyl)-1*H*-benzo[*d*]imidazole-6-carboxylic acid;
2-(((*R*)-3-((6-((4-cyano-2-fluorobenzyl)oxy)pyridin-2-yl)amino)pyrrolidin-1-yl)methyl)-1-(((*S*)-oxetan-2-yl)methyl)-1*H*-benzo[d]imidazole-6-carboxylic acid;
2-(((*R*)-3-((3-((5-chloropyridin-2-yl)methoxy)phenyl)amino)pyrrolidin-1-yl)methyl)-1-(((*S*)-oxetan-2-yl)methyl)-1*H*-benzo[d]imidazole-6-carboxylic acid;
2-((3-((6-((4-cyano-2-fluorobenzyl)oxy)pyridin-2-yl)amino)piperidin-1-yl)methyl)-1-(((*S*)-oxetan-2-yl)methyl)-1*H*-benzo[d]imidazole-6-carboxylic acid;
2-(((*R*)-3-((6-((4-cyano-2-fluorobenzyl)oxy)pyrazin-2-yl)amino)pyrrolidin-1-yl)methyl)-1-(((*S*)-oxetan-2-yl)methyl)-1*H*-benzo[d]imidazole-6-carboxylic acid;
(*S*)-2-((4-((6-((4-cyano-2-fluorobenzyl)oxy)pyridin-2-yl)(methyl)amino)piperidin-1-yl)methyl)-1-(oxetan-2-ylmethyl)-1*H*-benzo[*d*]imidazole-6-carboxylic acid;
2-(((*R*)-3-((3-((4-cyano-2-fluorobenzyl)oxy)phenyl)amino)pyrrolidin-1-yl)methyl)-1-(((*S*)-oxetan-2-yl)methyl)-1*H*-benzo[d]imidazole-6-carboxylic acid;
2-((3-((6-((4-chloro-2-fluorobenzyl)amino)pyridin-2-yl)oxy)pyrrolidin-1-yl)methyl)-1-(((*S*)-oxetan-2-yl)methyl)-1*H*-benzo[d]imidazole-6-carboxylic acid;
(*S*)-2-((4-((6-((5-chloropyridin-2-yl)methoxy)pyridin-2-yl)amino)piperidin-1-yl)methyl)-1-(oxetan-2-ylmethyl)-1*H*-benzo[d]imidazole-6-carboxylic acid;
(*S*)-2-((4-((6-((5-cyanopyridin-2-yl)methoxy)pyridin-2-yl)amino)pipeiidin-1-yl)methyl)-1-(oxetan-2-ylmethyl)-1*H*-benzo[d]imidazole-6-carboxylic acid;
2-(((*R*)-3-((6-((5-cyanopyridin-2-yl)methoxy)pyridin-2-yl)amino)pyrrolidin-1-yl)methyl)-1-(((*S*)-oxetan-2-yl)methyl)-1*H*-benzo[d]imidazole-6-carboxylic acid;
(*S*)-2-((3-((6-((4-cyano-2-fluorobenzyl)oxy)pyridin-2-yl)amino)azetidin-1-yl)methyl)-1-(oxetan-2-ylmethyl)-1*H*-benzo[d]imidazole-6-carboxylic acid;
2-(((*R*)-3-((4-((4-cyano-2-fluorobenzyl)oxy)pyrimidin-2-yl)amino)pyrrolidin-1-yl)methyl)-1-(((*S*)-oxetan-2-yl)methyl)-1*H*-benzo[d]imidazole-6-carboxylic acid; and
2-(((*R*)-3-((6-((5-chloropyridin-2-yl)methoxy)pyridin-2-yl)amino)pyrrolidin-1-yl)methyl)-1-(((*S*)-oxetan-2-yl)methyl)-1*H*-benzo[d]imidazole-6-carboxylic acid.

In another embodiment of the present disclosure, the present disclosure provides pharmaceutical compositions comprising the compounds of the Chemical Formula 1, 2, 3, 4, 5-1, 5-2, 5-3 or 6, optical isomers of the compounds, pharmaceutically acceptable salts of the compounds or the optical isomers as defined in any of the embodiments described herein.

The present disclosure also includes the following embodiments:
The present disclosure provides pharmaceutical compositions comprising the compounds of the Chemical Formula 1, 2, 3, 4, 5-1, 5-2, 5-3 or 6, optical isomers of the compounds pharmaceutically acceptable salts of the compouds or the optical isomers as defined in any of the embodiments described herein, and a pharmaceutically acceptable carrier.

The present disclosure provides the compounds of the Chemical Formula 1, 2, 3, 4, 5-1, 5-2, 5-3 or 6, optical isomers of the compounds, pharmaceutically acceptable salts of the compounds or the optical isomers as defined in any of the embodiments described herein for use in treatment and/or prevention of metabolic diseases.

The present disclosure provides methods for treating metabolic diseases, the method comprising administering to a subject in need the compounds of the Chemical Formula 1, 2, 3, 4, 5-1, 5-2, 5-3 or 6, optical isomers of the compounds, pharmaceutically acceptable salts of the compounds or the optical isomers as defined in any of the embodiments described herein.

The present disclosure provides uses of the compounds of the Chemical Formula 1, 2, 3, 4, 5-1, 5-2, 5-3 or 6, optical isomers of the compounds, pharmaceutically acceptable salts of the compounds or the optical isomers as defined in any of the embodiments described herein for prevention or treatment of metabolic diseases.

The present disclosure provides uses of the compounds of the Chemical Formula 1, 2, 3, 4, 5-1, 5-2, 5-3 or 6, optical isomers of the compounds, pharmaceutically acceptable salts of the compounds or the optical isomers as defined in any of the embodiments described herein for preparation of a medicament for prevention or treatment of metabolic diseases.

The present disclosure provides pharmaceutical compositions for prevention or treatment of metabolic diseases comprising the compounds of the Chemical Formula 1, 2, 3, 4, 5-1, 5-2, 5-3 or 6, optical isomers of the compounds, pharmaceutically acceptable salts of the compounds or the optical isomers as defined in any of the embodiments described herein.

The present disclosure provides GLP-1R agonists comprising the compounds of the Chemical Formula 1, 2, 3, 4, 5-1, 5-2, 5-3 or 6, optical isomers of the compounds , pharmaceutically acceptable salts of the compounds or the optical isomers as defined in any of the embodiments described herein.

According to the specific embodiments of the present disclosure, the compounds of the present disclosure exhibit excellent effects as GLP-1 agonists. Specifically, a result of performing a competitive immunoassay between an intrinsic cAMP generated in a cell and a foreign cAMP labeled with a dye shows that the compounds of the present disclosure have excellent effects as GLP-1 agonists.

In addition, a glucose tolerance test shows that the compounds, optical isomers, and pharmaceutically acceptable salts of the present disclosure have excellent glucose tolerance in both intravenous and oral administrations as well as have excellent pharmacokinetic properties. In addition, the compounds, the optical isomers, and the pharmaceutically acceptable salts of the present disclosure exhibit excellent pharmacological safety. For example, since it exhibits excellent safety for the cardiovascular system, the possibility of cardiotoxicity such as arrhythmia even when taken for a long period of time is expected to be very low.

All examples of compounds, optical isomers of the compounds or pharmaceutically acceptable salts of the compounds or the optical isomers, may be claimed individually or as a group together in any combination with any number of each and every embodiment described herein.

The present disclosure also relates to a pharmaceutical composition for use in the treatment and/or prevention of the metabolic diseases discussed herein comprising the compounds of the Chemical formulas 1, 2, 3, 4, 5-1, 5-2, 5 or 6, an optical isomer of the compounds, or a pharmaceutically acceptable salt of the compounds or the optical isomers as defined in any of the embodiments described herein.

Specifically, the metabolic disease used herein includes, for example, diabetes (T1D and/or T2DM, such as prediabetes), idiopathic T1D (type 1b), latent autoimmune diabetes in adults (LADA), early onset T2DM (EOD), younger onset atypical diabetes (YOAD), maturity onset diabetes in young (MODY), malnutrition-related diabetes, gestational diabetes, hyperglycemia, insulin resistance, liver insulin resistance, impaired glucose tolerance, diabetic neuropathy, diabetic nephropathy, kidney disease (e.g., acute kidney failure, tubular dysfunction, pro-inflammatory changes to proximal tubule), diabetic retinopathy, adipocyte dysfunction, visceral fat accumulation, sleep apnea, obesity (e.g., hypothalamic obesity and monogenic obesity) and associated comorbidities (e.g. osteoarthritis and urinary incontinence), eating disorders (e.g., binge eating syndrome, anorexia nervosa, and syndrome of obesity, such as Prader-Willi syndrome and Barde-Biedl syndrome), weight gain due to use of other drugs (e.g. from use of steroids and antipsychotics), excessive sugar intake, dyslipidemia (including hyperlipidemia, hypertriglyceridemia, increased total cholesterol, high LDL cholesterol, and low HDL cholesterol), hyperinsulinemia, Non-alcoholic fatty liver disease (NAFLD) (including related diseases such as steatosis, NASH, fibrosis, cirrhosis, and hepatocellular carcinoma), cardiovascular disease, atherosclerosis (including coronary artery disease), peripheral vascular disease, hypertension, endothelial dysfunction, impaired vascular compliance, congestive heart failure, myocardial infarction (e.g., necrosis and apoptosis), stroke, hemorrhagic stroke, ischemic stroke, traumatic brain injury, pulmonary hypertension, restenosis after angioplasty, intermittent claudication, postprandial lipidemia, metabolic acidosis, ketosis, arthritis, osteoporosis, Parkinson's disease, left ventricular hypertrophy, peripheral arterial disease, loss of vision, cataracts, glomerulosclerosis, chronic renal failure, metabolic syndrome, X syndrome, premenstrual syndrome, angina, thrombosis, atherosclerosis, transient ischemic attack, vascular restenosis, impaired glucose metabolism, symptoms of impaired fasting blood sugar, hyperuricemia, gout, erectile dysfunction, skin and connective tissue disorders, psoriasis, foot ulcers, ulcerative colitis, hyper-apo B lipoproteinemia, Alzheimer's disease, schizophrenia, cognitive impairment, inflammatory bowel disease, short bowel syndrome, Crohn's disease, colitis, irritable bowel syndrome, polycystic ovary syndrome, and addiction (e.g., alcohol and/or drug abuse).

The meanings of terms and symbols used herein are as follows:
As used herein, the term "alkyl" refers to a straight or branched chain monovalent hydrocarbon group of a structural formula -CₙH(₂ₙ₊₁). Non-limiting examples thereof include methyl, ethyl, propyl, isopropyl, butyl, 2-methyl-propyl, 1,1-dimethylethyl, pentyl and hexyl, and the like. For example, "C₁ to C₄ alkyl" may refer to alkyl such as methyl, ethyl, propyl, butyl, 2-methyl-propyl, or isopropyl.

As used herein, the term "C₆ to C₁₂ aryl" refers to an aromatic hydrocarbon containing 6 or 12 carbon atoms. The term "C₆ to C₁₂ aryl" refers to, for example, a ring system such as monocyclic (e.g., phenyl) or bicyclic (e.g., indenyl, naphthalenyl, tetrahydronaphthyl, tetrahydroindenyl).

As used herein, the term "C₅ to C₁₂ heteroaryl" refers to an aromatic hydrocarbon containing 5 to 12 carbon atoms in which at least one of ring carbon atoms is replaced with a heteroatom selected from oxygen, nitrogen and sulfur. The heteroaryl group may be attached via a ring carbon atom or, if valency permits, via a ring nitrogen atom or the like. The heteroaryl group includes fused ring system having 2 to 3 rings.

As used herein, the term "C₃ to C₈ cycloalkyl" refers to a cyclic monovalent hydrocarbon group of a structural formula -CₙH(₂ₙ₋₁) containing 3 to 8 carbon atoms. Non-limiting examples thereof include cyclopropyl, cyclobutyl, cyclopentyl, cyclohexyl, cycloheptyl, and cyclooctyl.

As used herein, the term "C₃ to C₈ heterocycloalkyl" refers to a cycloalkyl group containing 3 to 8 carbon atoms in which at least one of ring methylene groups (-CH₂-) is replaced with a group selected from -O-, -S- and nitrogen. In this case, nitrogen may provide an attachment point or may be substituted based on embodiments.

As used herein, the term "unsubstituted" means a state that hydrogen is not substituted with any substituent.

As used herein, the term "substituted aryl, heteroaryl, heterocycloalkyl, cycloalkyl, spiroheterocycloalkyl and heterobicycloalkyl" may include, for example, at least one substitution, that is, 1, 2, 3, 4, 5, 6 or more substitutions with -OH, -(C₁-C₄ alkyl), halogen, or -CN. Each of these substitutions may be made independently.

The term "C₃ to C₁₂ spiroheterocycloalkyl" as used herein means a non-aromatic hydrocarbon residue containing at least one hetero atom and containing at least two rings fused at a single carbon atom containing 3 to 12 carbon atoms. That is, the "spiroheterocycloalkyl" refers to a polycyclic hydrocarbon containing one or more heteroatoms, which share an atom (referred to as a spiro atom) between monocyclic rings. They may comprise one or more double bonds, but no atom of the ring has a fully conjugated pi-electron system.

As used herein, the term C₃ to C₁₂ bridged heterobicycloalkyl is a bridged multiple cyclic ring assembly and is a bridged multiple cyclic ring assembly containing at least one hetero atom, preferably one or more nitrogen atoms.

As used herein, the term "halogen" refers to fluoride, chloride, bromide, or iodide.

As used herein, the term "haloalkyl" refers to an alkyl group in which hydrogen is substituted with one or more halogens (e.g., fluoride, chloride, bromide, or iodide).

Following abbreviations in the present disclosure represent following corresponding terms:
EA: ethyl acetate
BINAP: 2,2'-bis(diphenylphosphino)-1,1'-binapthyl
DCM: dichloromethane
DMF: dimethylformamide
THF: tetrahydrofuran
TBD: triazabicyclodecene
DMSO: dimethyl sulfoxide
Pd₂(dba)₃: tris(dibenzylideneacetone)dipalladium(0)
MeOH: methanol
KOtBu: potassium tert-butoxide
A wavy line 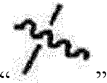 herein indicates a point of attachment of a substituent to another group.

In the Chemical Formula 1, R₁ is -C(=O)Rₐ, and Rₐ is -OH or -O-(C₁-C₄ alkyl). Preferably, R₁ may be -C(=O)OH.

In the Chemical Formula 1, R₂ is any one selected from the group consisting of substituted or unsubstituted C₆ to C₁₂ aryl, substituted or unsubstituted C₅ to C₁₂ heteroaryl, substituted or unsubstituted C₃ to C₈ heterocycloalkyl, and substituted or unsubstituted C₃ to C₈ cycloalkyl, where the substituted aryl, heteroaryl, heterocycloalkyl, and cycloalkyl include at least one substitution with -OH, -(C₁-C₄ alkyl), halogen, or -CN.

R₂ is preferably substituted or unsubstituted C₃ to C₅ heterocycloalkyl or substituted or unsubstituted C₃ to C₅ cycloalkyl, where the substituted heterocycloalkyl and cycloalkyl include at least one substitution with -OH, -(C₁-C₄ alkyl), halogen, or -CN. More preferably, R₂ is an oxacyclobutane containing a substituted or unsubstituted chiral central carbon, where the substituted oxacyclobutane includes at least one substitution with -OH, -(C₁-C₄ alkyl), halogen, or -CN.

More preferably, R₂ may be substituted or unsubstituted 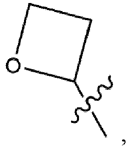 where the substituted 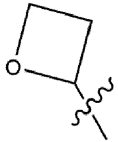 includes at least one substitution with -OH, -(C₁-C₄ alkyl), halogen, or -CN.

In another embodiment of the present disclosure, Y may be -CH- or -N-. More specifically, Y may be -CH-.

In the Chemical Formula 1, A₁ is 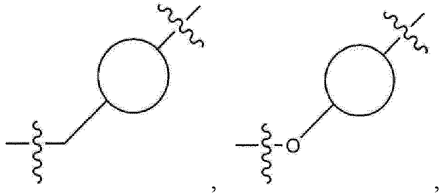 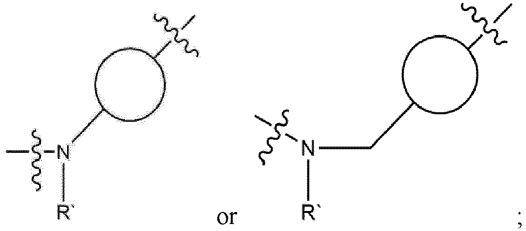 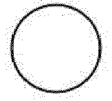 is substituted or unsubstituted C₃ to C₈ heterocycloalkyl containing at least one nitrogen, substituted or unsubstituted C₃ to C₁₂ spiroheterocycloalkyl containing at least one nitrogen, or substituted or unsubstituted C₃ to C₁₂ bridged heterobicycloalkyl containing at least one nitrogen, where the substituted heterocycloalkyl, spiroheterocycloalkyl, and heterobicycloalkyl include at least one substitution with -OH, -(C₁-C₄ alkyl), halogen, or -CN; and
R' is hydrogen or -(C₁-C₄ alkyl).

Specifically, 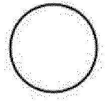 may be substituted or unsubstituted C₃ to C₈ heterocycloalkyl containing at least one nitrogen, where the substituted heterocycloalkyl includes at least one substitution with -OH, -(C₁-C₄ alkyl), halogen, or -CN.

Specifically, it may be one selected from the group consisting of

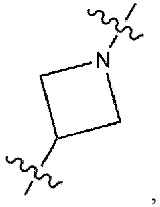

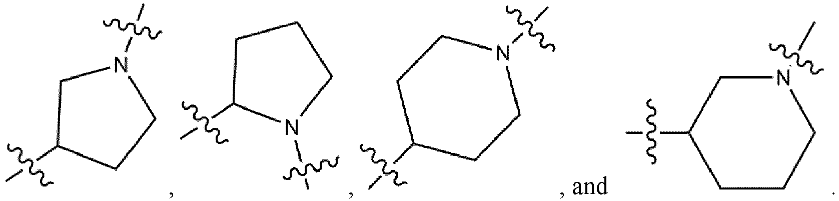

In another embodiment of the present disclosure, A₁ is

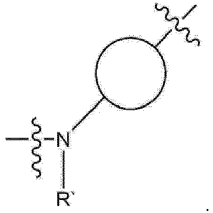

Specifically, 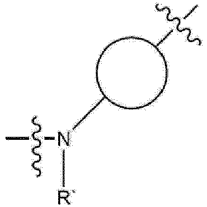 may be one selected from the group consisting of substituted or unsubstituted 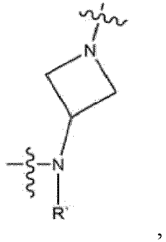 substituted or unsubstituted 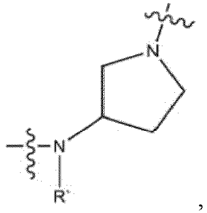 and substituted or unsubstituted 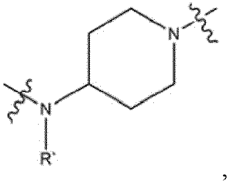 where the substituted 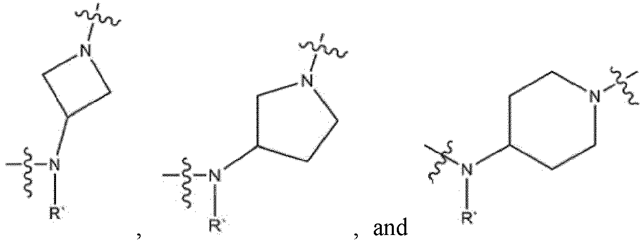 include at least one substitution with -OH, -(C₁-C₄ alkyl), halogen, or -CN.

Specifically, in the Chemical Formula 1, A₁ may be one selected from the group consisting of the following compounds:

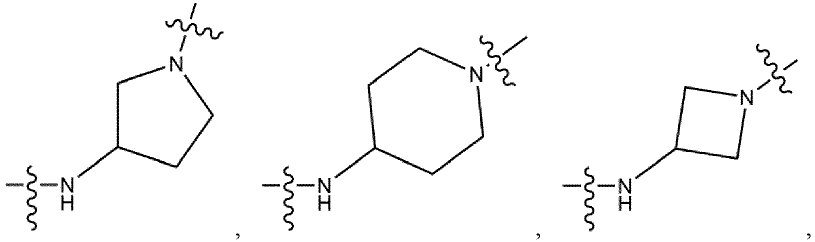

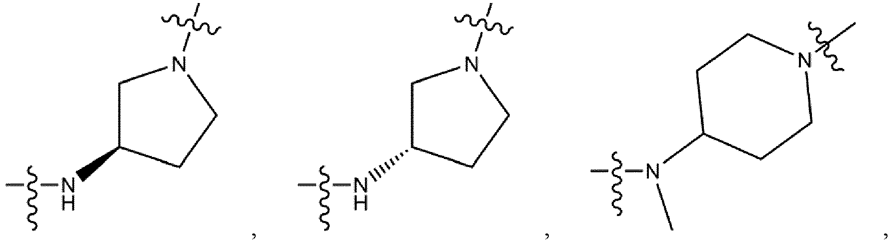

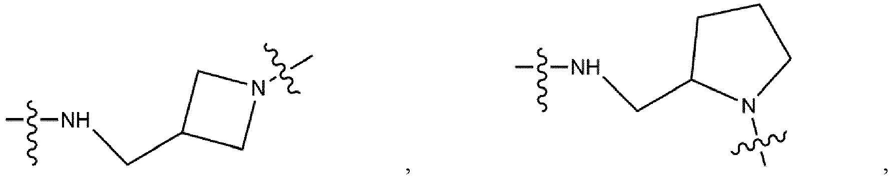

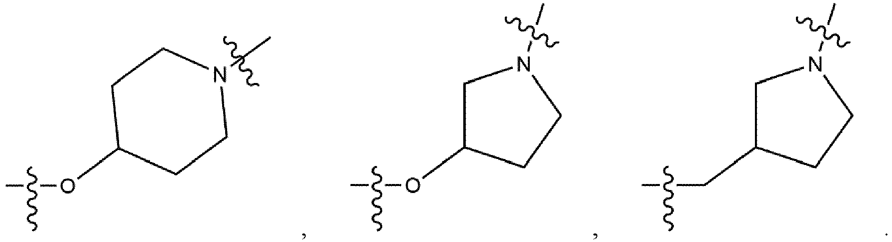

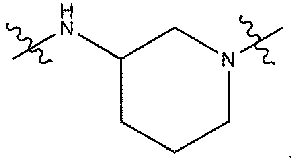

In another embodiment of the present disclosure, J may be -O- or -NR"-, and more specifically, J may be -O-.

In still another embodiment of the present disclosure, only one of Z₁ to Z₄ may be -N-.

In still another embodiment of the present disclosure, Z₁ may be -CR_{f}-, Z₂ may be -CR_{g}-, Z₃ may be -CRₕ-, and Z₄ may be -CRᵢ-.

In still another embodiment of the present disclosure, Rⱼ may be halogen or -CN.

Specifically, R_{g} may be any one selected from the group consisting of -H, halogen and -CN.

Preferably, 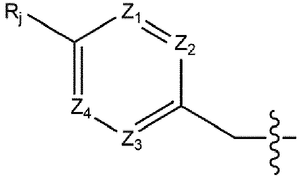 in the Chemical Formula 1 may be substituted or unsubstituted phenyl or substituted or unsubstituted pyridinyl.

Preferably, 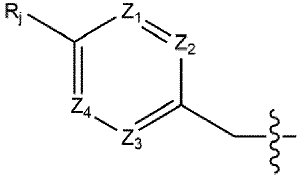 in the Chemical Formula 1 may be one selected from the group consisting of the following compounds:

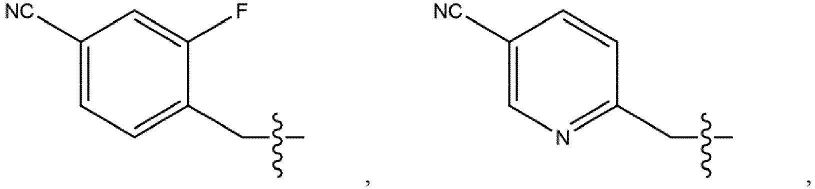

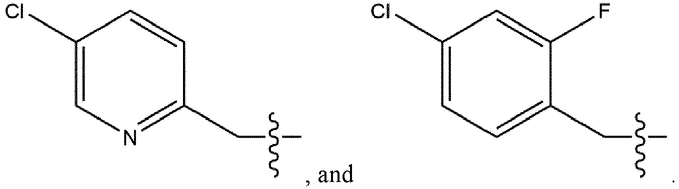

Preferably, 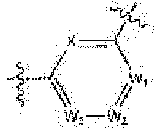 in the Chemical Formula 1 may be substituted or unsubstituted phenyl, substituted or unsubstituted pyridinyl, substituted or unsubstituted pyrimidinyl, or substituted or unsubstituted pyrazinyl.

Preferably, 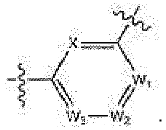 in the Chemical Formula 1 may be one selected from the group consisting of the following compounds:

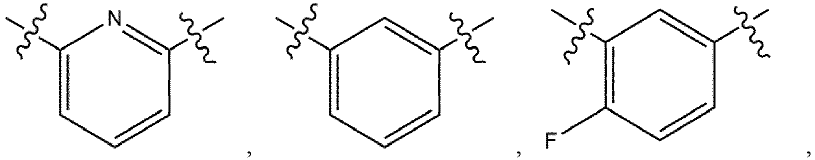

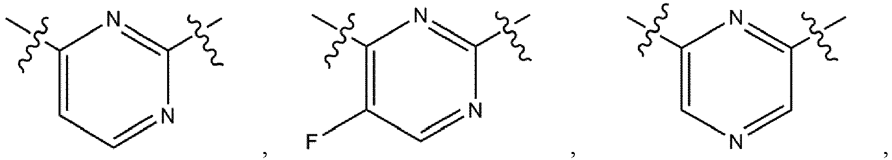

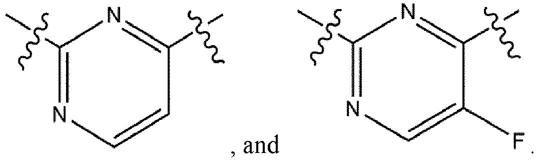

Compounds and intermediates as described below were named using the naming convention provided from ChemBioDraw Ultra. The naming convention is generally consistent with the International Union for Pure and Applied Chemistry (IUPAC) recommendations for nomenclature of organic chemistry and the CAS index rules. It will be appreciated that chemical names may have only parentheses, or both parentheses and brackets. A stereochemical description may be placed at different locations within a name itself, depending on the naming convention. Those of skill in the art will be aware of such formatting variations and may appreciate that they provide the same chemical structure.

The compounds, the optical isomers of the compounds, or pharmaceutically acceptable salts of the compounds or the optical isomers of the present disclosure may comprise acid addition salts and base addition salts.

Suitable acid addition salts are formed from acids that form non-toxic salts. Examples thereof may include acetate, adipate, aspartate, benzoate, besylate, bicarbonate/carbonate, bisulfate/sulfate, borate, camsylate, citrate, cyclamate, ediselate, esylate, formate, fumarate, gluceptate, gluconate, glucuronate, hexafluorophosphate, hybenzate, hydrochloride/chloride, hydrobromide/bromide, hydroiodide/iodide, isethionate, lactate, malate, maleate, malonate, mesylate, methyl sulfate, naphthylate, 2-naphsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate, 1,5-naphthalenedisulfonic acid, and xinafoate salts.

Suitable base addition salts are formed from bases forming non-toxic salts. Examples thereof may include aluminum, arginine, benzathine, calcium, choline, diethylamine, bis(2-hydroxyethyl)amine (diolamine), glycine, lysine, magnesium, meglumine, 2-aminoethanol (olamine), potassium, sodium, 2-amino-2-(hydroxymethyl) propane-1,3-diol (tris or trimethamine), and zinc salts.

In addition, hemi salts of acids and bases such as hemisulfate and hemicalcium salts may be formed.

The compounds, the optical isomers of the compounds, or pharmaceutically acceptable salts of the compounds or the optical isomers of the present disclosure may exist in unsolvated and solvated forms. As used herein, the term 'solvate' refers to a molecular complex comprising one or more pharmaceutically acceptable solvent molecules (e.g., ethanol) as well as a compound of the Chemical Formula 1, an optical isomer of the compound, or a pharmaceutically acceptable salt of the compound or the optical isomer. The term 'hydrate' refers to a solvate when the solvent is water.

A multi-component complex (in addition to salts and solvates) is also included within the scope of the present disclosure. In this connection, a medicament and one or more other components are then present in a stoichiometric or non-stoichiometric amount. The complex of this type includes inclusion compounds (drug-host inclusion complexes) and co-crystals. Co-crystals are typically defined as crystalline complexes of neutral molecular components that are bonded to each other via non-covalent interactions, but co-crystals may be complexes of neutral molecules with salts. Co-crystals may be prepared by melt crystallization, by recrystallization from a solvent, or by physically grinding the components together.

The compounds of the present disclosure may exist as a solid state continuum ranging from completely amorphous to fully crystalline. The term 'amorphous' refers to a state in which a substance loses a long-distance arrangement regularity at a molecular level and the physical properties of a solid or liquid may be exhibited depending on temperature. Typically, the substance does not provide a unique X-ray diffraction pattern, and exhibits properties of a solid, and is more formally described as a liquid. Upon heating thereof, a change thereof from solid to liquid properties thereof occurs. The substance is characterized by a state change (typically secondary) ('glass transition'). The term 'crystalline' refers to a solid phase in which a substance has arrangement regularity at the molecular level and provides an X-ray diffraction pattern with defined peaks. The substance will also exhibit the properties of a liquid when heated sufficiently, but the change thereof from solid to liquid is characterized by a phase change (typically primary) ('melting point').

The compounds of the present disclosure that contain one or more asymmetric carbon atoms may exist as two or more stereoisomers. When structural isomers are convertible to each other through a low energy barrier, tautomeric isomerism or tautomerism may occur. This may, for example, take a form of proton tautomerism in the compound of the Chemical Formula 1 containing imino, keto, or oxime groups, or take a form of valence tautomerism in the compound thereof containing aromatic residues. As a result, a single compound may exhibit at least two types of isomerism.

The pharmaceutically acceptable salts of the compounds of the present disclosure may contain counter-ions that are optically active or racemic.

Conventional techniques for the preparation/isolation of individual enantiomers include chiral synthesis from suitable optically pure precursors, for example, resolution of racemic bodies (or racemic bodies of salts or derivatives) using chiral high pressure liquid chromatography (HPLC). Alternatively, the racemic body (or racemic precursor) may react with base or acid (e.g. 1-phenylethylamine or tartaric acid), when a suitable optically active compound, e.g., an alcohol, or the compound of the Chemical Formula 1 contains acidic or basic residues. The resulting diastereomeric mixture may be separated using chromatography and/or fractional crystallization, and one or both of the diastereomers may be converted to the corresponding pure enantiomer(s) using means well known to those skilled in the art. A chiral compound of the Chemical Formula 1 (and a chiral precursor thereof) may be obtained in an enantiomer-enriched form using chromatography, typically HPLC, on asymmetric resins using a mobile phase composed of hydrocarbons, typically heptane or hexane, containing 0 to 50% by volume, typically 2% to 20% by volume of isopropanol, and 0 to 5% by volume of alkylamine, typically 0.1% by volume of diethylamine. Concentration of the eluent results in an enriched mixture. Chiral chromatography using subcritical and supercritical fluids may be used. Chiral chromatography methods useful in some embodiments of the present disclosure are known in the art.

When any racemic body crystallizes, two different types of crystals are possible. A first type is the above mentioned racemic compound (intrinsic racemic body) in which crystal of one homogeneous form containing both enantiomers in an equimolar amount is formed. A second type is a racemic mixture or conglomerate in which crystals of two forms, each comprising a single enantiomer, are produced in an equimolar amount. Although both the crystal forms present in the racemic mixture have the same physical properties, they may have different physical properties from those of the true racemic body. The racemic mixture may be separated using conventional techniques known to those skilled in the art.

The compound of Chemical Formula 1, preferably the compounds of Chemical Formulas 2 to 6 described herein comprise the compounds itself and prodrugs thereof. The present disclosure comprises the compound of Chemical Formula 1, as well as a pharmaceutically acceptable salt of the compound, and a pharmaceutically acceptable solvate of the compound and the salt.

### Administration and Dosage

Typically, the compounds of the present disclosure may be administered in an amount effective to treat the symptoms described herein. Compounds of the present disclosure may be administered as the compounds itself or, alternatively, as pharmaceutically acceptable salts of the compounds. For administration and dosage purposes, the compounds or the pharmaceutically acceptable salts of the compounds of present disclosure may, for the sake of simplicity, be referred to as the compound or compounds according to the present disclosure.

The compound according to the present disclosure is administered via any suitable route, in a form of a pharmaceutical composition suitable for the route, and in a dosage effective for intended treatment. The compound according to the present disclosure may be administered orally, or in rectal, vaginal, parenteral, or topical manner.

The compound according to the present disclosure may preferably be administered orally. Oral administration may involve swallowing to allow the compound to enter the gastrointestinal tract, or it may include buccal or sublingual administration to allow the compound to enter the bloodstream directly from the oral cavity.

In some embodiments, the compound according to the present disclosure may be administered directly to the bloodstream, muscle or internal organs. Suitable means for parenteral administration include intravenous, intraarterial, intraperitoneal, intrathecal, intraventricular, intraurethral, intrasternal, intracranial, intramuscular and subcutaneous administration. Devices suitable for parenteral administration include needle (including microneedle) syringes, needleless syringes and infusion techniques.

In other embodiments, the compound according to the present disclosure may be administered topically (that is, in epidermal or transdermal manner) to the skin or mucous membrane. In still another embodiment, the compound according to the present disclosure may be administered intranasally or by inhalation. In still other embodiments, the compound according to the present disclosure may be administered rectally or intravaginally. In yet other embodiments, the compound according to the present disclosure may be administered directly to the eye or ear.

The compound according to the present disclosure and/or the composition containing the compound may be administered based on various factors including a type, age, weight, sex and medical symptom of the patient; severity of symptoms; route of administration; and activity of a particular compound as used. Thus, the administration scheme may vary widely. In some embodiments, a total daily dose of the compound according to the present disclosure may be typically about 0.001 to about 100 mg/kg (i.e., mg of the compound according to the present disclosure per kg body weight) for the treatment of the symptoms discussed herein. In other embodiments, the total daily dose of the compound according to the present disclosure may be about 0.01 to about 30 mg/kg, about 0.03 to about 10 mg/kg, or about 0.1 to about 3 mg/kg. It is not unusual for the administration of the compound according to the present disclosure to be repeated several times a day (typically no more than 4 times a day). The multiple doses per day may typically be used to increase the total daily dose, if necessary.

In the oral administration, the composition may be provided in the form of tablets, capsules, liquids, etc. for symptom-related control of the dosage to the patient. The medicament typically contains from about 0.01 mg to about 500 mg of the active ingredient.

Suitable subjects according to the present disclosure include mammalians. In some embodiments, humans are suitable subjects. Human subjects may be male or female and may be at any stage of growth.

### Pharmaceutical Compositions

In another embodiments, the present disclosure provides pharmaceutical compositions.

The present disclosure provides pharmaceutical compositions for preventing and treating metabolic diseases comprising the compounds represented by Chemical Formula 1, optical isomers of the compounds, or pharmaceutically acceptable salts of the compounds or the optical isomers.

The pharmaceutical compositions may comprise pharmaceutically acceptable carriers with the compounds of the present disclosure.

Other active pharmaceutical ingredients may also be present. As used herein, the term "pharmaceutically acceptable carrier" includes any and all of physiologically compatible solvents, dispersion media, coatings, antibacterial and antifungal agents, isotonic and absorption delaying agents, and the like. Examples of the pharmaceutically acceptable carriers include one or more of water, saline, phosphate buffered saline, dextrose, glycerol, ethanol, as well as combinations thereof. Isotonic agents such as sugar, sodium chloride, or polyalcohols such as mannitol or sorbitol may be contained in the composition. For example, a pharmaceutically acceptable ingredient (e.g., wetting agent) or a small amount of an auxiliary ingredient (e.g., wetting agent, emulsifying agent, preservative, or buffer) that can enhance the shelf life or effectiveness of an antibody or a portion thereof may be contained in the composition.

The composition according to the present disclosure may be in various forms. The composition according to the present disclosure may be in a form of, for example, liquid, semi-solid and solid dosage, such as liquid solutions (e.g., injectable and injectable solutions), dispersions or suspensions, tablets, pills, powders, liposomes and suppositories. The form depends on the intended route of administration and therapeutic purpose thereof.

A typical composition is in the form of injectable and infusible solutions. One mode of administration is a parenteral mode (e.g., intravenous, subcutaneous, intraperitoneal, intramuscular mode). In some embodiments, a drug may be administered via intravenous infusion or injection. In some other embodiments, a drug may be administered via intramuscular or subcutaneous injection.

Oral administration of a solid formulation may be achieved, for example, based on hard or soft capsules, pills, cachets, lozenges or tablets, each containing a predetermined amount of one or more compounds according to the present disclosure. In some embodiments, oral administration may be achieved based on powder or granular form. In some other embodiments, the oral dosage form may be sublingual form, for example, lozenge. In the solid dosage form, the compound of the Chemical Formula 1 is usually combined with one or more excipients. The capsules or tablets may contain controlled release formulations. The capsules, tablets and pills may also contain a buffering agent or may be prepared into an enteric coating.

In still other embodiments, oral administration may be achieved in a liquid dosage form. The liquid dosage forms for oral administration include, for example, pharmaceutically acceptable emulsions, solutions, suspensions, syrups, and elixirs containing inert diluents (e.g., water) commonly used in the art. The composition may contain excipients such as wetting agents, emulsifying agents, suspending agents, flavoring agents (e.g., sweetening agents), and/or fragrances.

In some embodiments, the present disclosure provides parenteral dosage forms of the composition. As used herein, the term "parenteral administration" includes, for example, subcutaneous injection, intravenous injection, intraperitoneal injection, intramuscular injection, intrasternal injection, and infusion. Injectable preparations (i.e., sterile injectable aqueous or oleaginous suspensions) may be formulated according to known techniques using suitable dispersing, wetting and/or suspending agents.

Other carrier substances and modes of administration known in the pharmaceutical art may be used. The pharmaceutical composition according to the present disclosure may be prepared by any well-known pharmaceutical technique, such as effective formulation and administration procedures. The considerations related to the effective formulation and administration procedures are well known in the art, and they are described in standard textbooks.

### Kits

In another embodiments of the present disclosure, the present disclosure provides kits containing the compounds of the Chemical Formula 1, 2, 3, 4, 5-1, 5-2, 5-3 or 6, or the pharmaceutical compositions comprising the compounds of the Chemical Formula 1, 2, 3, 4, 5-1, 5-2, 5-3 or 6. The kit may contain a diagnostic agent or a therapeutic agent in addition to the compound of the Chemical Formula 1, 2, 3, 4, 5-1, 5-2, 5-3 or 6, or the pharmaceutical compositions comprsing thereof. The kit includes instructions for use in a diagnostic or therapeutic method. In some embodiments, the kit comprises the compound of the Chemical Formula 1, 2, 3, 4, 5-1, 5-2, 5-3 or 6, or the pharmaceutical composition containing the compound, and a diagnostic agent. In some other embodiments, the kit comprises the compound of the Chemical Formula 1, 2, 3, 4, 5-1, 5-2, 5-3 or 6, or the pharmaceutical composition comprising the compound.

In still other embodiments, the present disclosure provides kits each suitable for use in carrying out the treatment methods described herein. In some embodiments, the kit contains a first dosage formulation comprising one or more compounds according to the present disclosure in an amount sufficient to carry out the method according to the present disclosure. In some other embodiments, the kit contains one or more compounds according to the present disclosure in an amount sufficient to carry out the method according to the present disclosure, and a container for administration thereof.

### Preparation

Reaction Formulas as described below are intended to provide a general description of the methodology used in the preparation of the compounds, optical isomers, or pharmaceutical acceptable salts according to the present disclosure. Some of the compounds according to the present disclosure may contain single or multiple chiral centers with stereochemical designations (R) or (S). It will be apparent to those skilled in the art that whether the substance is enantiomer-enriched or is a racemic body, all synthetic conversions may be carried out in a similar manner. In addition, separation of an optically active target substance may be carried out at any desired point in a sequence using known methods as described herein and in the chemical literature.

In following Reaction formulas, the variables X, Y, W₁, W₂, W₃, J, A₁, Z₁, Z₂, Z₃, Z₄, R₁, R₂, Rₐ, R_{b}, R_{c}, R_{d}, Rₑ, R_{f}, R_{g}, Rₕ, Rᵢ, and Rⱼ are the same as described herein with reference to the compound of the Chemical Formula 1, 2, 3, 4, 5-1, 5-2, 5-3 or 6, unless otherwise stated.

The compounds of the Chemical Formula 1, 2, 3, 4, 5-1, 5-2, 5-3 or 6, according to the present disclosure include compounds of the following Examples as prepared below. The compounds of the Examples may be prepared or provided based on various methods described in the literature and common technical knowledge known to those skilled in the art based on two or more selected from following intermediate compounds. The intermediate compounds may be prepared or provided based on various methods described in the literature and common technical knowledge known to those skilled in the art, in addition to following descriptions.

Methods of preparing the intermediates used to prepare the compounds of the Chemical Formula 1, 2, 3, 4, 5-1, 5-2, 5-3 or 6, are described in the Preparation Methods 1 to 6 to be described below.

### [Advantageous Effects]

The novel compounds according to the present disclosure exhibit excellent activity as GLP-1 receptor agonists. In addition, the compounds according to the present disclosure, as GLP-1 receptor agonists, exhibit excellent glucose tolerance, thus exhibiting a remarkable effect as a therapeutic agent for metabolic diseases. Moreover, the novel compounds according to the present disclosure exhibit excellent pharmacological safety.

### [Modes of the Invention]

Hereinafter, preferred examples are set forth to aid in understanding the present disclosure. However, the following examples are provided for easier understanding of the present disclosure, and the present disclosure is not limited thereto.

Reagents and solvents as mentioned below were purchased from Sigma-Aldrich, TCI, etc., unless otherwise noted. Waters Alliance high-performance liquid chromatography (HPLC) system was used. Biotage Flash purification system was used as a silica gel used for column chromatography. ¹H NMR spectra was recorded using Bruker 400 MHz Ascend^{™} system. Waters Masslynx mass spectrum system was used.

All of ¹H nuclear magnetic resonance (NMR) spectra were consistent with the proposed chemical structures. Characteristic chemical shifts (δ) are given in parts-permillion (ppm) relative to the residual proton signal in the deuterated solvent (CDCl₃: 7.27 ppm; CD₃OD: 3.31 ppm; DMSO-*d*₆: 2.50 ppm) and are reported using conventional abbreviations for designation of major peaks: for example, s: singlet; d: doublet; t: triplet; q: quartet; m: multiplet; and br: broad.

### Synthesis Examples

### Synthesis Example 1: Synthesis of intermediates 1 to 39

Exemplary methods of preparing intermediates 1 to 39 are described in detail below. Using the Preparation Methods 1 to 7 described below, those skilled in the art may prepare the compounds listed as intermediates 1 to 39 from appropriate starting materials which are available commercially or may be prepared by methods known in the art.

### 1. Preparation Method 1

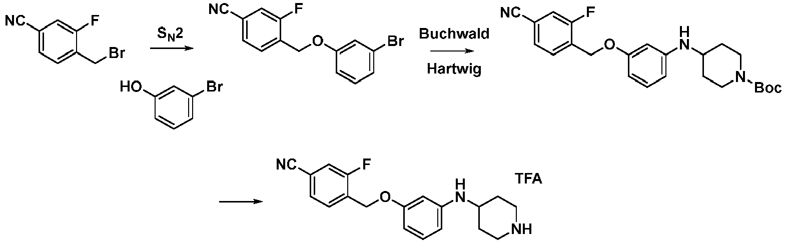

### (1) Synthesis of Intermediate 1: 3-fluoro-4-((3-(piperidin-4-ylamino)phenoxy)methyl)benzonitrile trifluoroacetic acid salt

### 1) Synthesis of 4-((3-bromophenoxy)methyl)-3-fluorobenzonitrile

4-(Bromomethyl)-3-fluorobenzonitrile (10 g), 3-bromophenol (5.46 mL) and potassium carbonate (9.68 g) were dissolved in CH₃CN (100 mL), which was stirred at room temperature for 16 hours. After completion of the reaction as indicated by TLC, 1*N* NaOH was added and the mixture was extracted twice with ethyl acetate to separate the compound into an organic layer. The resulting organic layer was dried over anhydrous Na₂SO₄, filtered and concentrated under reduced pressure. Then, the resulting product was separated by silica gel column chromatography eluting with hexane/ethyl acetate, followed by solidification under DCM/hexane conditions to obtain the target compound (11.88 g) as a white solid at a yield of 83%. LC-MS(ES⁺): 307 (M+H)⁺ .

### 2) Synthesis of tert-butyl 4-((3-((4-cyano-2-fluorobenzyl)oxy)phenyl)amino)piperidine-1-carboxylate

The compound (674 mg) synthesized in the step 1), tert-butyl 4-aminopiperidine-1-carboxylate (529 mg), Pd₂(dba)₃ (201 mg), NaOtBu (423 mg), and DavePhos (173 mg) were placed in a round bottom flask and stirred in toluene (4 mL). The air inside the reactor was replaced with nitrogen using a nitrogen balloon. Then, the resulting mixture was heated to 120°C under N₂ atmosphere and stirred for one day. After completion of the reaction as indicated by TLC, the solution was diluted with ethyl acetate and inorganic substances were filtered under reduced pressure on a Celite pad to obtain a filtrate. The filtrate was concentrated under reduced pressure, which was subjected to silica gel column chromatography eluting with hexane/ethyl acetate to obtain the target compound (177 mg) as a yellow syrup at a yield of 19%. LC-MS(ES⁺): 426 (M+H)⁺ .

### 3) Synthesis of 3-fluoro-4-((3-(piperidin-4-ylamino)phenoxy)methyl)benzonitrile trifluoroacetic acid salt

The compound (177 mg) synthesized in the step 2) was placed in a round bottom flask, dissolved in DCM (4 mL) and stirred. TFA (4 mL) was added to the mixture, which was stirred at room temperature for 30 minutes. After completion of the reaction as indicated by TLC, the resulting product was filtered under reduced pressure and solidified by adding ether to obtain the target compound. LC-MS(ES⁺): 326 (M+H)⁺.

### 2. Preparation Method 2

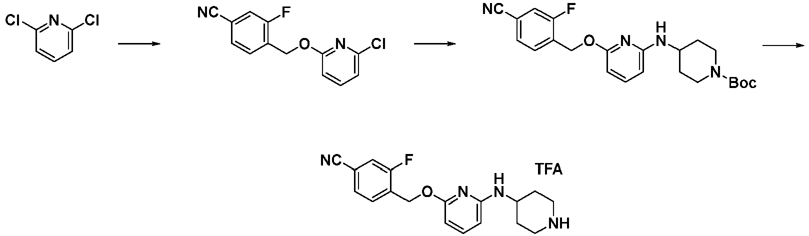

### (1) Synthesis of Intermediate 2: 3-fluoro-4-(((6-(piperidin-4-ylamino)pyridin-2-yl)oxy)methyl)benzonitrile trifluoroacetic acid salt

### 1) Synthesis of 4-(((6-chloropyridin-2-yl)oxy)methyl)-3-fluorobenzonitrile

4-Cyano-2-fluorobenzyl alcohol (3.81 g) was added dropwise at 15°C to a solution of potassium tert-butoxide (4.24 g) in THF (54 mL) and the mixture was stirred for 45 minutes. 2,4-Dichloropyridine (3.1 g) was added dropwise to the reaction mixture, and the resulting mixture was stirred at 15°C for 18 hours. The reaction was quenched by adding aqueous NH₄Cl to the reaction mixture. Then, ethyl acetate was added and the mixture was stirred for 15 minutes. The resulting mixture was filtered using a Celite pad to obtain a filtrate. The filtrate was extracted with ethyl acetate, and the organic layer was dried over anhydrous magnesium sulfate, filtered, and concentrated. The concentrated residue was subjected to silica gel column chromatography eluting with hexane/ethyl acetate to obtain the target compound (4.85 g) as a white solid at a yield of 88%. LC-MS(ES⁺): 263 (M+H)⁺.

### 2) Synthesis of tert-butyl 3-((6-((4-cyano-2-fluorobenzyl)oxy)pyridine-2-yl)amino)piperidine-1-carboxylate

The compound (300.0 mg) synthesized in the step 1), 4-amino-*N*-Boc-piperidine (274.5 mg), Pd₂(dba)₃ (52.3 mg), BINAP (71.1 mg), and Cs₂CO₃ (558.2 mg) were dissolved in toluene (3.0 mL). The resulting mixture was stirred at 100°C for 16 hours under nitrogen atmosphere. The reaction product was cooled to room temperature and filtered through a Celite pad to obtain a filtrate, which was concentrated under reduced pressure. The concentrated residue was subjected to silica gel column chromatography eluting with hexane/ethyl acetate to obtain the target compound (228.2 mg) as a pale yellow solid at a yield of 34.2%. LC-MS(ES⁺): 427 (M+H)⁺.

### 3) Synthesis of 3-fluoro-4-(((6-(piperidin-3-ylamino)pyridin-2-yl)oxy)methyl)benzonitrile trifluoroacetic acid salt

The compound (220.0 mg) synthesized in the step 2) was dissolved in DCM (3.0 mL). TFA (5.0 mL) was added dropwise and the resulting mixture was stirred at room temperature for 1 hour. The reaction product was concentrated, then isopropyl ether was slowly added dropwise. The resulting product was filtered to obtain the target compound (258 mg) as a bright pale yellow solid at a yield of 95.9%. LC-MS(ES⁺): 327 (M+H)⁺.

### 3. Preparation Method 3

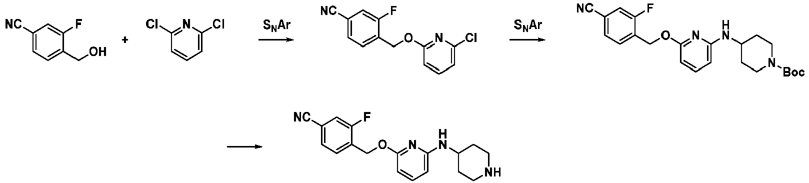

### (1) Synthesis of Intermediate 3: 3-fluoro-4-(((5-fluoro-2-(piperidin-4-ylamino)pyrimidin-4-yl)oxy)methyl)benzonitrile

### 1) Synthesis of 4-(((6-chloropyridin-2-yl)oxy)methyl)-3-fluorobenzonitrile

To a stirred solution of 2,4-dichloropyridine (1.0 g) in toluene (50 mL), was added sodium tert-butoxide (724 mg) at 0°C and the reaction mixture was stirred for 30 minutes. 4-cyano-2-fluorobenzyl alcohol (760 mg) was added dropwise to the reaction mixture, which was stirred at room temperature for 15 hours. The reaction was quenched by adding aqueous NH₄Cl to the reaction mixture, and the resulting mixture was extracted twice with ethyl acetate. The extracted organic layer was dried over anhydrous magnesium sulfate, filtered, and concentrated. The concentrated residue was subjected to silica gel column chromatography eluting with hexane/ethyl acetate to obtain the target compound (900 mg) as a white solid at a yield of 64%. LC-MS(ES⁺): 263 (M+H)⁺

### 2) Synthesis of tert-butyl 4-((6-((4-cyano-2-fluorobenzyl)oxy)pyridin-2-yl)amino)piperidine-1-carboxylate

The compound (200 mg) synthesized in the step 1) was placed in a round bottom flask and dissolved in DMSO (1.4 mL). Then, triethylamine (0.25 mL) and 4-amino-1-Boc-piperidine (285 mg) were added and the resulting mixture was stirred at room temperature. After the air inside the reactor was replaced with nitrogen using a nitrogen balloon, the resulting mixture was stirred at 120°C for 1 hour. The reaction was quenched by adding distilled water and the resulting mixture was extracted twice with ethyl acetate. The inorganic substances were filtered on a Celite pad under reduced pressure to obtain a filtrate, which was concentrated under reduced pressure, then subjected to silica gel column chromatography eluting with hexane/acetone to obtain the target compound (55 mg) at a yield of 17%. LC-MS(ES⁺): 427 (M+H)⁺

### 3) Synthesis of 3-fluoro-4-(((5-fluoro-2-(piperidin-4-ylamino)pyrimidin-4-yl)oxy)methyl)benzonitrile

To a stirred solution of the compound (110 mg) synthesized in the step 2) in DCM (1.2 mL), was added TFA (0.6 mL) dropwise at 0°C. The reaction mixture was stirred at room temperature for 1 hour. After completion of the reaction as indicated by TLC, the mixture was concentrated under reduced pressure and dissolved in DCM. The organic layer was washed with saturated aqueous solution of NaHCO₃, dried over anhydrous magnesium sulfate, filtered, and concentrated under reduced pressure to obtain the target compound (85 mg) at a yield of 99%. LC-MS(ES⁺): 327 (M+H)⁺

### 4. Preparation Method 4

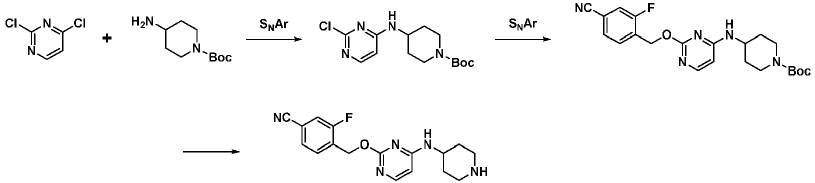

### (1) Synthesis of Intermediate 4: 3-fluoro-4-(((4-(piperidin-4-ylamino)pyrimidin-2-yl)oxy)methyl)benzonitrile

### 1) Synthesis of tert-butyl 4-((2-chloropyrimidin-4-yl)amino)piperidine-1-carboxylate

To a stirred solution of 2,4-dichloropyridine (2.0 g) in DMF (22 mL), triethylamine (2.8 mL) and 4-amino-1-Boc-piperidine (2.9 g) were added at room temperature, which was stirred for 15 hours. The reaction was quenched by adding distilled water to the reaction mixture and the resulting mixture was extracted twice with ethyl acetate. The extracted organic layer was dried over anhydrous magnesium sulfate, filtered, and concentrated. The concentrated residue was subjected to silica gel column chromatography eluting with hexane/ethyl acetate to obtain the target compound (2.6 g) as a white solid at a yield of 62%. LC-MS(ES⁺): 313 (M+H)⁺

### 2) Synthesis of tert-butyl 4-((2-((4-cyano-2-fluorobenzyl)oxy)pyrimidin-4-yl)amino)piperidine-1-carboxylate

The compound (1 g) synthesized in the step 1) was dissolved in THF (16 mL) and sodium hydride (192 mg) was added at room temperature. After stirring at room temperature for 15 minutes, 4-cyano-2-fluorobenzyl alcohol (726 mg) was added and the mixture was stirred at 80°C for 2 days. After completion of the reaction as indicated by TLC, distilled water was added to terminate the reaction and the resulting mixture was extracted twice with ethyl acetate. The inorganic substances were filtered on a Celite pad under reduced pressure to obtain a filtrate, which was concentrated under reduced pressure, then subjected to silica gel column chromatography eluting with hexane/ethyl acetate to obtain the target compound (380 mg) as a white solid at a yield of 28%. LC-MS(ES⁺): 428 (M+H)⁺

### 3) Synthesis of 3-fluoro-4-(((4-(piperidin-4-ylamino)pyrimidin-2-yl)oxy)methyl)benzonitrile

The compound (378 mg) synthesized in the step 2) was placed in a round bottom flask, dissolved in DCM (4.4 mL) and stirred. TFA (2.2 mL) was added at room temperature and the resulting mixture was stirred at room temperature for 1.5 hours. After completion of the reaction as indicated by TLC, the mixture was concentrated under reduced pressure and dissolved in DCM. The organic layer was washed with saturated aqueous solution of NaHCO₃, dried over anhydrous magnesium sulfate, filtered and concentrated under reduced pressure to obtain the target compound (140 mg) at a yield of 48%. LC-MS(ES⁺): 328 (M+H)⁺

### 5. Preparation Method 5

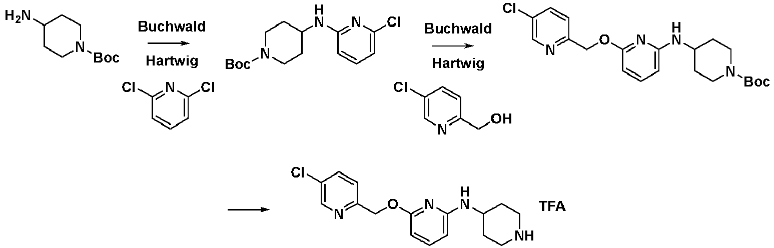

### (1) Synthesis of Intermediate 5: 6-((5-chloropyridin-2-yl)methoxy)-N-(piperidin-4-yl)pyridin-2-amine trifluoroacetic acid salt

### 1) Synthesis of tert-butyl 4-((6-chloropyridin-2-yl)amino)piperidine-1-carboxylate

2,6-Dichloropyridine (200 mg), tert-butyl 4-aminopiperidine-1-carboxylate (300 mg), Pd₂(dba)₃ (63 mg), Cs₂CO₃ (880 mg), and BINAP (88 mg) were placed in a round bottom flask and stirred in toluene (10 mL). The air inside the reactor was replaced with nitrogen using a nitrogen balloon, then the resulting product was heated to 100°C under N₂ atmosphere, which was stirred for one day. After completion of the reaction as indicated by TLC, the solution was diluted with ethyl acetate and inorganic substances were filtered under reduced pressure on a Celite pad to obtain a filtrate. The filtrate was concentrated under reduced pressure, which was subjected to silica gel column chromatography eluting with hexane/ethyl acetate to obtain the target compound (295 mg) as a yellow solid at a yield of 70%.

### 2) Synthesis of tert-butyl 4-((6-((5-chloropyridin-2-yl)methoxy)pyridin-2-yl)amino)piperidine-1-carboxylate

The compound (256 mg) synthesized in the step 1), (5-chloropyridin-2-yl)methanol (160 mg), Pd₂(dba)₃ (75.1 mg), sodium tert-butoxide (205 mg), and BINAP (76.6 mg) were placed in a round bottom flask and stirred in toluene (10 mL). The air inside the reactor was replaced with nitrogen using a nitrogen balloon, then the resulting product was heated to 100°C under N₂ atmosphere, which was stirred for one day. After completion of the reaction as indicated by TLC, the solution was diluted with ethyl acetate and inorganic substances were filtered under reduced pressure on a Celite pad to obtain a filtrate. The filtrate was concentrated under reduced pressure, which was subjected to silica gel column chromatography eluting with hexane/ethyl acetate to obtain the target compound (68 mg) as a yellow oil at a yield of 20%.

### 3) Synthesis of 6-(((5-(chloropyridin-2-yl)methoxy)-N-(piperidin-4-yl)pyridin-2-amine trifluoroacetic acid salt

To a stirred solution of the compound (68 mg) synthesized in the step 2) in DCM (10 mL), was added TFA (1 mL) dropwise at 0°C. The reaction mixture was stirred at room temperature for 1 hour. After completion of the reaction as indicated by TLC, the mixture was concentrated under reduced pressure to obtain the target compound, which was used in the next reaction without further purification.

### 6. Preparation Method 6

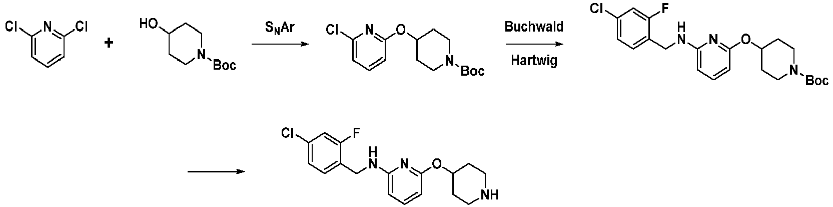

### (1) Synthesis of Intermediate 6: N-(4-chloro-2-fluorobenzyl)-6-(piperidin-4-yloxy)pyridin-2-amine

### 1) Synthesis of tert-butyl 4-((6-chloropyridin-2-yl)oxy)piperidine-1-carboxylate

To a stirred solution of 2,4-dichloropyridine (1.0 g) in DMF (33 mL), sodium hydride (810 mg) and tert-butyl-4-hydroxy-1-piperidine carboxylate (2.0 g) were added at room temperature. The reaction mixture was stirred at room temperature for 4 hours. The reaction was quenched by adding distilled water to the reaction mixture and the resulting mixture was extracted twice with ethyl acetate. The extracted organic layer was dried over anhydrous magnesium sulfate, filtered, and concentrated. The concentrated residue was subjected to silica gel column chromatography eluting with hexane/ethyl acetate to obtain the target compound (1.3 g) as a transparent oil at a yield of 62%. LC-MS(ES⁺): 313 (M+H)⁺ .

### 2) Synthesis of tert-butyl 4-((6-((4-chloro-2-fluorobenzyl)amino)pyridin-2-yl)oxy)piperidine-1-carboxylate

The compound (200 mg) synthesized in the step 1), Pd₂(dba)₃ (29 mg), BINAP (40 mg), sodium tert-butoxide (105 mg), and (4-chloro-2-fluorophenyl)methanamine (122 mg) were placed in a round bottom flask and stirred in toluene (3.2 mL) at 70°C for 15 hours. The inorganic substances were filtered on a Celite pad under reduced pressure to obtain a filtrate, which was concentrated under reduced pressure, then subjected to silica gel column chromatography eluting with hexane/ethyl acetate to obtain the target compound (245 mg) as a white solid at a yield of 88%. LC-MS(ES⁺): 436 (M+H)⁺.

### 3) Synthesis of N-(4-chloro-2-fluorobenzyl)-6-(piperidin-4-yloxy)pyridin-2-amine

The compound (240 mg) synthesized in the step 2) was placed in a round bottom flask, dissolved in DCM (2.7 mL) and stirred. TFA (1.4 mL) was added at room temperature and the resulting mixture was stirred at room temperature for 2 hours. After completion of the reaction as indicated by TLC, the mixture was concentrated under reduced pressure and dissolved in DCM. The organic layer was washed with a saturated aqueous solution of NaHCO₃, dried over anhydrous magnesium sulfate, filtered, and concentrated under reduced pressure to obtain the target compound (179 mg) as a yellow oil at a yield of 97%. LC-MS(ES⁺): 336 (M+H)⁺.

### 7. Preparation Method 7

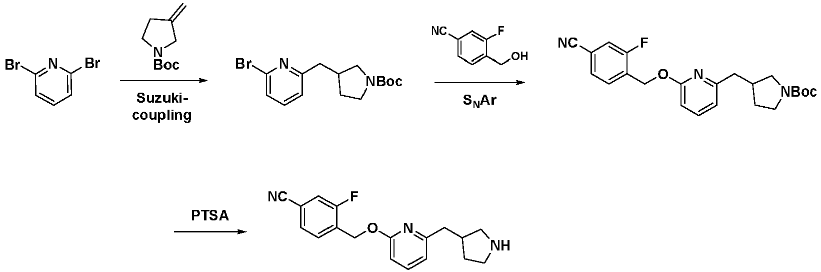

### (1) Synthesis of Intermediate 7: tert-butyl 3-((6-bromopyridin-2-yl)methyl)pyrrolidine-1-carboxylate

### 1) Synthesis of tert-butyl 3-((6-bromopyridin-2-yl)methyl)pyrrolidine-1-carboxylate

Tert-butyl 3-methylenepyrrolidine-1-carboxylate (0.77 g) was dissolved in THF (20 mL) and the solution was degassed at room temperature for 15 minutes with nitrogen. The air inside the reactor was replaced with nitrogen using a nitrogen balloon, then 9-borabicyclo[3.3.1]nonane (8.4 mL, 0.5 M in THF) was added. The reaction mixture was stirred at 60°C for 3 hours, cooled to room temperature, which was then added to a mixture of 2,6-dibromopyridine (1 g), potassium carbonate (0.75 g), PdCl₂(dppf)CH₂Cl₂ complex (0.1 g, 0.03 eq) in DMF/water (=10 mL: 1 mL), prepared and degassed beforehand. The reaction mixture was stirred at 85°C for 16 hours. After completion of the reaction, the resulting product was basified to pH 11 with 1*N* NaOH aqueous solution. The aqueous layer was extracted twice with ethyl acetate. The combined organic layer was washed with brine, dried over anhydrous sodium sulfate, filtered, and concentrated under reduced pressure. The concentrated residue was subjected to silica gel column chromatography to obtain the target compound (0.72 g) as a transparent oil at a yield of 47%. LC-MS(ES⁺): 341 (M+H)⁺

### 2) Synthesis of tert-butyl 3-((6-((4-cyano-2-fluorobenzyl)oxy)pyridin-2-yl)methyl)pyrrolidine-1-carboxylate

The compound (0.8 g, 2.344 mmol, 1.0 eq.) synthesized in the step 1) was placed in a sealed tube and dissolved in toluene (25 mL). Cesium carbonate (2.28 g), 3-fluoro-4-(hydroxymethyl)benzonitrile (425 mg) and 2-(di-tert-butylphosphino)biphenyl (0.02 g, 0.09 mmol, 0.04 eq.) were added to the solution at room temperature, which was degassed for 15 minutes with nitrogen. After the air inside the reactor was replaced with nitrogen using a nitrogen balloon, tris(dibenzylideneacetone)dipalladium(0) (0.042 g) was added to the reaction mixture at room temperature. The reaction mixture was heated to 110°C and stirred for 16 hours. After completion of the reaction, the resulting product was cooled to room temperature, diluted with distilled water, and extracted twice with ethyl acetate. The combined organic layers were washed sequentially with water and brine, dried over anhydrous sodium sulfate, filtered, and concentrated under reduced pressure. The obtained concentrated residue was subjected to silica gel column chromatography to obtain the target compound (0.5 g) as a brown oil at a yield of 51%. LC-MS(ES⁺): 412 (M+H)⁺

### 3) Synthesis of 3-fluoro-4-(((6-(pyrrolidin-3-ylmethyl)pyridin-2-yl)oxy)methyl)benzonitrile

The compound (1.1 g, 2.67 mmol, 1.0 eq.) synthesized in the step 1) was dissolved in ethyl acetate (35 mL), and para-toluenesulfonic acid (p-TSA, 1.01 g, 5.34 mmol, 2.0 eq.) was added at room temperature. The reaction mixture was stirred at 65°C for 4 hours. After completion of the reaction, the reaction mixture was concentrated under reduced pressure, diluted with saturated aqueous NaHCO₃ solution, and extracted twice with DCM. The combined organic layer was washed with brine, dried over anhydrous sodium sulfate, filtered, and concentrated to obtain the target compound (0.8 g, crude) as a brown oil. The obtained compound was used in the next reaction without further purification. LC-MS (M+H)⁺: 312.31

### 8. Synthesis of intermediates 8 to 41

The compounds listed as intermediates 8 to 41 in Table 1 below were prepared by using procedures identical or analogous to the synthesis methods of Preparation Methods 1 to 7 from appropriate starting materials that are available commercially or prepared by preparation methods well known to those skilled in the art, or prepared in a similar manner to the route described above for other intermediates. The compounds were purified using methods well known to those skilled in the art, which may include silica gel chromatography, HPLC, or recrystallization from the reaction mixture. The final compounds could be isolated as neutrals or as acid addition or base addition salts and the trifluoroacetic acid (TFA) salt may exist as at least one or more TFA salts. The compound names and LC-MS data of the prepared intermediates are shown in Table 1 below.

**Table 1**

| Interme diate No. | Preparati on Method | Structure | Compound name | LC/MS Data (ES+) |
|---|---|---|---|---|
| 8 | 1 | 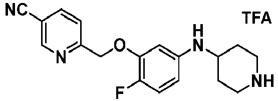 | 6-((2-fluoro-5-(piperidin-4-ylamino)phenoxy)methyl)nic otinonitrile trifluoroacetic acid salt | 327 (M+H)⁺ |
| 9 | 1 | 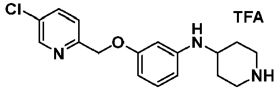 | *N*-(3-((5-chloropyridin-2-yl)methoxy)phenyl)piperidin -4-amine trifluoroacetic acid salt | 318 (M+H)⁺ |
| 10 | 1 | 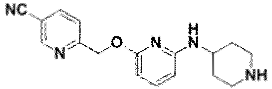 | 6-(((6-(piperidin-4-ylamino)pyridin-2-yl)oxy)methyl)nicotinonitrile | 310 (M+H)⁺ |
| 11 | 1 | 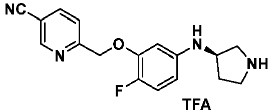 | (R)-6-((2-fluoro-5-(pyrrolidin-3-ylamino)phenoxy)methyl)nic otinonitrile trifluoroacetic acid salt | 313 (M+H)⁺ |
| 12 | 1 | 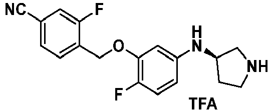 | (*R*)-3-fluoro-4-((2-fluoro-5-(pyrrolidin-3-ylamino)phenoxy)methyl)be nzonitrile trifluoroacetic acid salt | 330 (M+H)⁺ |
| 13 | 1 | 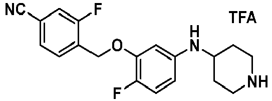 | 3-fluoro-4-((2-fluoro-5-(piperidin-4-ylamino)phenoxy)methyl)be nzonitrile trifluoroacetic acid salt | 344 (M+H)⁺ |
| 14 | 1 | 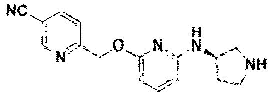 | (*R*)-6-(((6-(pyrrolidin-3-ylamino)pyridin-2-yl)oxy)methyl)nicotinonitrile | 296 (M+H)⁺ |
| 15 | 1 | 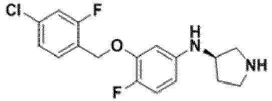 | (*R*)-*N*-(3-((4-chloro-2-fluorobenzyl)oxy)-4-fluorophenyl)pyrrolidin-3-amine | 339 (M+H)⁺ |
| 16 | 1 | 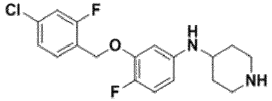 | *N*-(3-((4-chloro-2-fluorobenzyl)oxy)-4-fluorophenyl)piperidin-4-amine | 353 (M+H)⁺ |
| 17 | 2 | 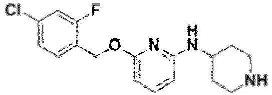 | 6-((4-chloro-2-fluorobenzyl)oxy)-*N-*(piperidin-4-yl)pyridin-2-amine | 336 (M+H)⁺ |
| 18 | 2 | 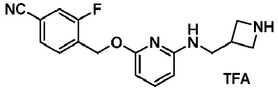 | 4-(((6-((azetidin-3-ylmethyl)amino)pyridin-2-yl)oxy)methyl)-3-fluorobenzonitrile trifluoroacetic acid salt | 313 (M+H)⁺ |
| 19 | 2 | 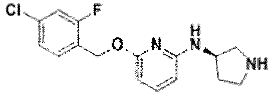 | (*R*)-6-((4-chloro-2-fluorobenzyl)oxy)-*N-*(pyrrolidin-3-yl)pyridin-2-amine | 322 (M+H)⁺ |
| 20 | 2 | 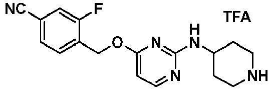 | 3-fluoro-4-(((2-(piperidin-4-ylamino)pyrimidin-4-yl)oxy)methyl)benzonitrile trifluoroacetic acid salt | 328 (M+H)⁺ |
| 21 | 2 | 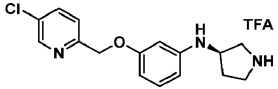 | (*R*)-*N*-(3-((5-chloropyridin-2-yl)methoxy)phenyl)pyrroli din-3-amine trifluoroacetic acid salt | 304 (M+H)⁺ |
| 22 | 2 | 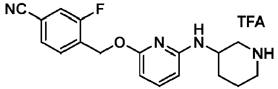 | 3-fluoro-4-(((6-(piperidin-3-ylamino)pyridin-2-yl)oxy)methyl)benzonitrile trifluoroacetic acid salt | 327 (M+H)⁺ |
| 23 | 2 | 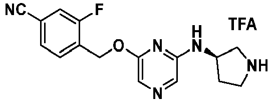 | (*R*)-3-fluoro-4-(((6-(pyrrolidin-3-ylamino)pyrazin-2-yl)oxy)methyl)benzonitrile trifluoroacetic acid salt | 314 (M+H)⁺ |
| 24 | 2 | 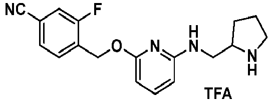 | 3-fluoro-4-(((6-((pyrrolidin-2-ylmethyl)amino)pyridin-2-yl)oxy)methyl)benzonitrile trifluoroacetic acid salt | 327 (M+H)⁺ |
| 25 | 2 | 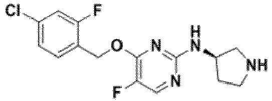 | (*R*)-4-((4-chloro-2-fluorobenzyl)oxy)-5-fluoro-*N*-(pyrrolidin-3-yl)pyrimidin-2-amine | 341 (M+H)⁺ |
| 26 | 2 | 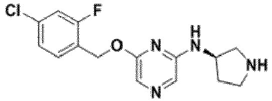 | (*R*)-6-((4-chloro-2-fluorobenzyl)oxy)-*N-*(pyrrolidin-3-yl)pyrazin-2-amine | 323 (M+H)⁺ |
| 27 | 2 | 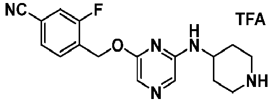 | 3-fluoro-4-(((6-(piperidin-4-ylamino)pyrazin-2-yl)oxy)methyl)benzonitrile trifluoroacetic acid salt | 328 (M+H)⁺ |
| 28 | 2 | 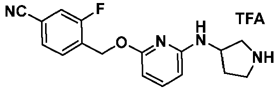 | 3-fluoro-4-(((6-(pyrrolidin-3-ylamino)pyridin-2-yl)oxy)methyl)benzonitrile trifluoroacetic acid salt | 313 (M+H)⁺ |
| 29 | 2 | 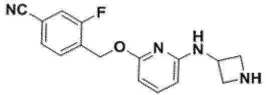 | 4-(((6-(azetidin-3-ylamino)pyridin-2-yl)oxy)methyl)-3-fluorobenzonitrile | 299 (M+H)⁺ |
| 30 | 2 | 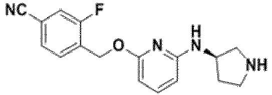 | (*R*)-3-fluoro-4-(((6-(pyrrolidin-3-ylamino)pyridin-2-yl)oxy)methyl)benzonitrile | 313 (M+H)⁺ |
| 31 | 2 | 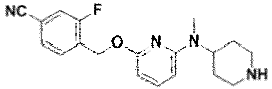 | 3-fluoro-4-(((6-(methyl(piperidin-4-yl)amino)pyridin-2-yl)oxy)methyl)benzonitrile | 341 (M+H)⁺ |
| 32 | 2 | 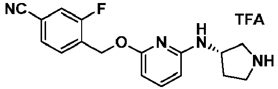 | (*S*)-3-fluoro-4-(((6-(pyrrolidin-3-ylamino)pyridin-2-yl)oxy)methyl)benzonitrile trifluoroacetic acid salt | 313 (M+H)⁺ |
| 33 | 2 | 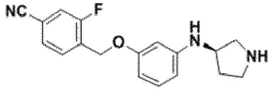 | (*R*)-3-fluoro-4-((3-(pyrrolidin-3-ylamino)phenoxy)methyl)be nzonitrile | 312 (M+H)⁺ |
| 34 | 3 | 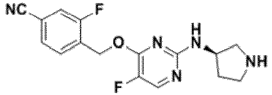 | (*R*)-3-fluoro-4-(((5-fluoro-2-(pyrrolidin-3-ylamino)pyrimidin-4-yl)oxy)methyl)benzonitrile | 332 (M+H)⁺ |
| 35 | 3 | 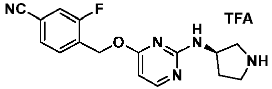 | (*R*)-3-fluoro-4-(((2-(pyrrolidin-3-ylamino)pyrimidin-4-yl)oxy)methyl)benzonitrile trifluoroacetic acid salt | 314 (M+H)⁺ |
| 36 | 4 | 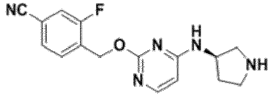 | (*R*)-3-fluoro-4-(((4-(pyrrolidin-3-ylamino)pyrimidin-2-yl)oxy)methyl)benzonitrile | 314 (M+H)⁺ |
| 37 | 4 | 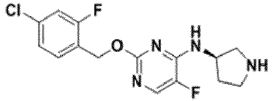 | (*R*)-2-((4-chloro-2-fluorobenzyl)oxy)-5-fluoro-*N*-(pyrrolidin-3-yl)pyrimidin -4-amine | 341 (M+H)⁺ |
| 38 | 5 | 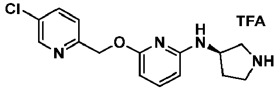 | (R)-6-((5-chloropyridin-2-yl)methoxy)-*N*-(pyrrolidin-3-yl)pyridin-2-amine trifluoroacetic acid salt | 305 (M+H)⁺ |
| 39 | 6 | 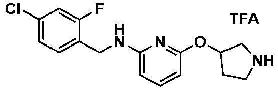 | *N*-(4-chloro-2-fluorobenzyl)-6-(pyrrolidin-3-yloxy)pyridin-2-amine trifluoroacetic acid salt | 322 (M+H)⁺ |
| 40 | Synthesis method described in WO2018 109607 | 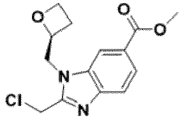 | methyl (*S*)-2-(chloromethyl)-1-(oxetan-2-ylmethyl)-1*H-*benzo[d]imidazole-6-carboxylate | 295 (M+H)⁺ |
| 41 | Synthesis method described in WO2018 109607 | 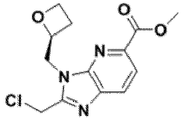 | methyl (*S*)-2-(chloromethyl)-3-(oxetan-2-ylmethyl)-3*H-*imidazo[4,5-*b*]pyridine-5-carboxylate | 296 (M+H)⁺ |

### Example

Synthesis methods of Examples 1 to 44 using the intermediates were described in detail below. The following Preparation Examples A, B, and C show specific examples of the synthesis methods of Examples 1 to 44 using the intermediates above. Those skilled in the art may synthesize the compounds of Examples 1 to 44 according to the present disclosure with reference to the specific examples of Preparation Examples A, B, and C.

### 1. Preparation Example A

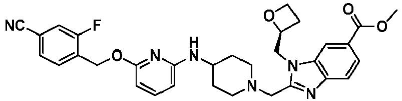

### (1) Synthesis of Example 1: (S)-2-((4-((6-((4-cyano-2-fluorobenzyl)oxy)pyridin-2-yl)amino)piperidin-1-yl)methyl)-1-(oxetan-2-ylmethyl)-1H-benzo[d]imidazole-6-carboxylic acid

### 1) Synthesis of methyl (S)-2-((4-((6-((4-cyano-2-fluorobenzyl)oxy)pyridin-2-yl)amino)piperidin-1-yl)methyl)-1-(oxetan-2-ylmethyl)-1H-benzo[d]imidazole-6-carboxylate

Intermediate 2 (250.0 mg), potassium carbonate (198.8 mg), and Intermediate 40 (155.4 mg) were dissolved in CH₃CN (4.0 mL), which was stirred at 80 to 90°C for 4 hours. The reaction mixture was cooled to room temperature, filtered through a Celite pad to obtain a filtrate. The filtrate was concentrated under reduced pressure, which was then subjected to silica gel column chromatography eluting with hexane/ethyl acetate to obtain the target compound (246.8 mg) as a light brown solid at a yield of 88.1%. LC-MS(ES⁺): 585 [M+H]⁺.

### 2) Synthesis of final compound

The compound (230 mg) obtained in the step 1) was dissolved in 1,4-dioxane/H₂O (4:1, 3 mL), then LiOH (33 mg) was added thereto and the mixture was stirred at 60°C for 5 hours. The reaction mixture was cooled to room temperature, neutralized with IN HCl, and extracted with ethyl acetate. The extracted organic layer was dried over anhydrous magnesium sulfate, filtered, and concentrated. The concentrated residue was subjected to silica gel column chromatography eluting with DCM/MeOH to obtain the final compound (92 mg) as a bright yellow solid at a yield of 41.1%. ¹H NMR (400 MHz, DMSO-*d*₆): δ 12.81(brs, 1H), 8.26 (s, 1H), 7.89 (d, *J*=1.2 Hz, 1H), 7.80 (dd, *J*= 6.8 Hz, *J*= 1.6 Hz, 1H), 7.69 (dd, *J* = 8.0 Hz, *J* = 1.6, 1H), 7.64 (d, *J* = 8.4 Hz, 1H), 7.59 (t, *J* = 7.2 Hz, 1H), 7.29 (t, *J* = 7.6 Hz, 1H), 6.47 (d, *J* = 7.2 Hz, 1H), 6.01 (d, *J* = 8.0 Hz, 1H), 5.94 (d, *J* = 7.6 Hz, 1H), 5.40 (s, 2H), 5.11-5.06 (m, 1H), 4.48 (dd, *J* = 15.2 Hz, *J* = 7.6 Hz, 1H), 4.65-4.61 (m, 1H), 4.52-4.47 (m, 2H), 4.40-4.36(m, 1H), 3.93 (d, *J* = 13.6 Hz, 1H), 3.74 (d, *J* = 13.6 Hz, 1H), 3.48-3.47(m, 1H), 2.85-2.83 (m, 1H), 2.76-2.67 (m, 2H), 2.46-2.39 (m, 1H), 2.18-2.07 (m, 2H), 1.73 (brs, 2H), 1.36-1.28 (m, 2H); LC-MS(ES⁺): 585 [M+H]⁺.

### (2) Synthesis of Examples 2 to 31

The compounds listed as Examples 2 to 31 in Table 2 below were prepared by using procedures identical or analogous to the synthesis method (Preparation Example A) of Example 1 from appropriate starting materials which are available commercially or prepared by preparation methods well known to those skilled in the art. The compounds were purified using methods well known to those skilled in the art, which may include silica gel chromatography, HPLC, or recrystallization from the reaction mixture. The final compounds could be isolated as neutrals or as acid addition or base addition salts. The compound names, NMR data, and LC-MS data of the Examples prepared above are shown in Table 2 below.

**Table 2**

| Example No. | No. of Intermediate A | No. of Intermediate B | Compound name | NMR data | LC/MS data (ES+) |
|---|---|---|---|---|---|
| 2 | 1 | 40 | (*S*)-2-((4-((3-((4-cyano-2-fluorobenzyl)ox y)phenyl)amino) piperidin-1-yl)methyl)-1-(oxetan-2-ylmethyl)-1*H-*benzo[d]imidazo le-6-carboxylic acid | ¹H NMR (400 MHz, DMSOd₆): *δ* 13.78 (brs, 1H), 8.26 (s, 1H), 7.92 (d, *J* =8.8 Hz, 1H), 7.80 (dd, *J* =8.4 Hz, *J* =1.2 Hz, 1H), 7.72-7.75 (m, 2H), 7.63 (d, J =8.4 Hz, 1H), 6.96 (t, *J* =8.2 Hz, 1H), 6.16-6.22 (m, 3H), 5.53 (d, *J* =8.0 Hz, 1H), 5.15 (s, 2H), 5.08-5.14 (m, 1H), 4.73-4.81 (m, 1H), 4.61-4.68 (m, 1H), 4.43-4.51 (m, 1H), 4.37-4.41 (m, 1H), 4.12 (brs, 2H), 3.93 (d, *J* =13.6 Hz, 1H), 3.75 (d, *J* =13.6 Hz, 1H), 3.28-3.39 (m, 1H), 3.15-3.26 (m, 1H), 2.72-2.74 (m, 1H), 2.69-2.79 (m, 2H), 2.40-2.49 (m, 1H), 2.17-2.29 (m, 2H), 1.81-1.90 (m, 2H), 1.28-1.41 (m, 2H). | 570 (M+H)⁺ |
| 3 | 3 | 40 | (*S*)-2-((4-((4-((4-cyano-2-fluorobenzyl)ox y)-5-fluoropyrimidin-2-yl)amino)piperid in-1-yl)methyl)-1-(oxetan-2-ylmethyl)-1*H-*benzo[d]imidazo le-6-carboxylic acid | ¹H NMR (400 MHz, DMSO-d₆): *δ* 8.22 (s, 1H), 8.14 (d, J =2.8 Hz, 1H), 7.94 (d, *J* =10.0 Hz, 1H), 7.81 (d, *J* =8.4 Hz, 1H), 7.76-7.74 (m, 2H), 7.58 (d, *J* =8.4 Hz, 1H), 7.15-7.13 (m, 1H), 5.54 (s, 2H), 5.11-5.06 (m, 1H), 4.75 (dd, *J* =15.2, 7.2 Hz, 1H), 4.63-4.60 (m, 1H), 4.52-4.47 (m, 1H), 4.39 (ddd, *J* =12.0, 8.8, 6.0 Hz, 1H), 3.91 (d, *J* =13.2 Hz, 1H), 3.74 (d, *J* =13.6 Hz, 1H), 2.90-2.87 (m, 1H), 2.77-2.68 (m, 2H), 2.47-2.41 (m, 1H), 2.20-2.08 (m, 2H), 1.76 (m, 2H), 1.43 (m, 2H). | 591 (M+H) |
| 4 | 4 | 40 | (*S*)-2-((4-((2-((4-cyano-2-fluorobenzyl)ox y)pyrimidin-4-yl)amino)piperid in-1-yl)methyl)-1-(oxetan-2-ylmethyl)-1*H*-benzo[d]imidazo le-6-carboxylic acid | ¹H NMR (400 MHz, DMSOd₆): *δ* 8.24 (s, 1H), 7.91 (d, *J* =9.2 Hz, 1H), 7.94 (d, *J* =10.0 Hz, 1H), 7.80 (d, *J* =8.4 Hz, 2H), 7.72 (d, *J* =6.8 Hz, 1H), 7.65-7.62 (m, 2H), 7.49-7.46 (m, 1H), 6.11 (d, *J* =5.2 Hz, 1H), 5.38 (s, 2H), 5.10-5.08 (m, 1H), 4.79-4.74 (m, 1H), 4.64-4.61 (m, 1H), 4.52-4.47 (m, 1H), 4.39 (ddd, *J* =9.2, 6.0, 6.0 Hz, 2H), 3.92 (d, *J* =13.6 Hz, 1H), 3.77-3.74 (m, 2H), 2.87-2.86 (m, 1H), 2.76-2.68 (m, 2H), 2.24-2.13 (m, 2H), 1.78 (m, 2H), 1.40-1.37 (m, 2H). | 572 (M+H)⁺ |
| 5 | 6 | 40 | (*S*)-2-((4-((6-((4-chloro-2-fluorobenzyl)am ino)pyridin-2-yl)oxy)piperidin -1-yl)methyl)-1-(oxetan-2-ylmethyl)-1*H-*benzo[d]imidazo le-6-carboxylic acid | ¹H NMR (400 MHz, DMSOd₆): δ 8.26 (d, *J* =1.2 Hz, 1H), 7.80 (dd, *J* =8.4, 1.2 Hz, 1H), 7.64 (d, *J* =8.4 Hz, 1H), 7.37 (dd, *J* =10.0, 2.0 Hz, 1H), 7.33-7.26 (m, 2H), 7.21 (dd, *J* =8.0, 2.0 Hz, 1H), 7.15 (dd, *J* =5.6, 5.6 Hz, 1H), 6.08 (d, *J* =8.0 Hz, 1H), 5.83 *(d, J =7.6* Hz, 1H), 5.09-5.07 (m, 1H), 4.78 (dd, *J* =15.2, 7.2 Hz, 1H), 4.71-4.67 (m, 1H), 4.63 (dd, *J* =15.2, 2.4 Hz, 1H), 4.52-4.47 (m, 1H), 4.43-4.35 (m, 3H), 3.92 (d, *J* =13.2 Hz, 1H), 3.75 (d, *J* =13.2 Hz, 1H),2.74-2.64 (m, 3H), 2.47-2.40 (m, 1H), 2.21-2.14 (m, 2H), 1.77-1.75 (m, 2H), 1.50-1.44 (m, 2H). | 580 (M+H)⁺ |
| 6 | 8 | 40 | (*S*)-2-((4-((3-((5-cyanopyridin-2-yl)methoxy)-4-fluorophenyl)am ino)piperidin-1-yl)methyl)-1-(oxetan-2-ylmethyl)-1*H-*benzo[d]imidazo le-6-carboxylic acid | ¹H NMR (400 MHz, DMSOd₆): *δ* 12.73 (brs, 1H), 9.06 (s, 1H), 8.38 (d, *J* =8.2, 2.2 Hz, 1H), 8.27 (d, *J* =0.8 Hz, 1H), 7.80 (dd, *J* =8.4, 1.6 Hz, 1H), 7.66 (dd, *J* =20.0, 8.4 Hz, 2H), 6.93 (dd, *J* =11.6, 8.8 Hz, 1H), 6.34 (dd. *J* =7.4, 2.6 Hz, 1H), 6.13-6.10 (m, 1H), 5.41-5.36 (m, 1H), 5.28 (s, 2H), 5.10-5.07 (m, 1H), 4.81-4.75 (m, 1H), 4.63 (d, *J* =12.8 Hz, 1H), 4.51-4.49 (m, 1H), 4.41-4.37 (m, 1H), 3.93 (d, *J* =13.6 Hz, 1H), 3.75 (d, *J* =13.6 Hz, 1H), 3.64-3.57 (m, 1H), 3.19-3.10 (m, 1H), 2.88-2.82 (m, 1H), 2.74-2.69 (m, 2H), 2.46-2.42 (m, 1H), 2.23-2.17 (m, 2H), 1.88-1.76 (m, 2H), 1.33-1.26 (m, 1H). | 571 (M+H)⁺ |
| 7 | 9 | 40 | (*S*)-2-((4-((3-((5-chloropyridin-2-yl)methoxy)phe nyl)amino)piperi din-1-yl)methyl)-1-(oxetan-2-ylmethyl)-1*H-*benzo[d]imidazo le-6-carboxylic acid | ¹H NMR (400 MHz, DMSOd₆): *δ* 12.75 (brs, 1H), 8.63 (d, *J* =2.4 Hz, 1H), 8.26 (s, 1H), 7.96 (dd, *J* =8.6, 2.6 Hz, 1H), 7.81 (dd, *J* =8.6, 1.4 Hz, 1H), 7.63 (d, *J* =8.4 Hz, 1H), 7.52 (d, *J* =8.4 Hz, 1H), 6.94 (t, *J* =8.0 Hz, 1H), 6.20-6.13 (m, 3H), 5.51 (d, *J* =8.0 Hz, 1H), 5.15-5.06 (m, 3H), 4.81-4.75 (m, 1H), 4.64 (dd, *J* =15.0, 2.6 Hz, 1H), 4.51-4.47 (m, 1H), 4.42-4.38 (m, 1H), 3.93 (d, *J* =13.6 Hz, 1H), 3.75 (d, *J* =13.2 Hz, 1H), 3.26-3.12 (m, 1H), 2.87 (d, *J* =11.2 Hz, 1H), 2.76-2.67 (m, 2H), 2.46-2.41 (m, 1H), 2.25-2.19 (m, 2H), 1.86-1.82 (m, 2H), 1.37-1.24 (m, 2H). | 562 (M+H)⁺ |
| 8 | 11 | 40 | 2-(((*R*)-3-((3-((5-cyanopyridin-2-yl)methoxy)-4-fluorophenyl)am ino)pyrrolidin-1-yl)methyl)-1-(((*S*)-oxetan-2-yl)methyl)-1*H-*benzo[d]imidazo le-6-carboxylic acid | ¹H NMR (400 MHz, DMSOd₆): *δ* 12.79 (brs, 1H), 9.05 (t, *J* =1.2 Hz, 1H), 8.37 (dd, *J* =8.2, 2.2 Hz, 1H), 8.25 (d, *J* =0.8 Hz, 1H), 7.80 (dd, *J* =8.6, 1.4 Hz, 1H), 7.65 (dd, *J* =17.4, 8.2 Hz, 2H), 6.94 (dd, *J* =11.6, 8.8 Hz, 1H), 6.31 (dd, *J* =7.4, 2.6 Hz, 1H), 6.08-6.05 (m, 1H), 5.68 (d, *J* =6.4 Hz, 1H), 5.27 (s, 2H), 5.07-5.04 (m, 1H), 4.78-4.71 (m, 1H), 4.61 (dd, *J* =15.2, 2.4 Hz, 1H), 4.48-4.45 (m, 1H), 4.35-4.30 (m, 1H), 4.03 (d, *J* =13.6 Hz, 1H), 3.88 (d, *J* =13.2 Hz, 1H), 3.84-3.79 (m, 1H), 3.40-3.37 (m, 1H), 2.85-2.80 (m, 1H), 2.68-2.64 (m, 1H), 2.56-2.53 (m, 1H), 2.45-2.33 (m, 2H), 2.20-2.11 (m, 1H), 1.55-1.51 (m, 1H). | 557 (M+H)⁺ |
| 9 | 11 | 41 | 2-(((R)-3-((3-((5-cyanopyridin-2-yl)methoxy)-4-fluorophenyl)amino)pyrrolidin-1-yl)methyl)-3-(((*S*)-oxetan-2-yl)methyl)-3*H-*imidazo[4,5-*b*]pyridine-5-carboxylic acid | ¹H NMR (400 MHz, DMSOd₆): *δ* 9.05 (d, *J* =1.2 Hz, 1H), 8.38 (dd, *J* =8.4, 2.0 Hz, 1H), 8.12-8.01 (m, 1H), 7.69 (d, *J* =8.0 Hz, 1H), 7.12-6.95 (m, 2H), 6.41-6.31 (m, 1H), 6.19-6.08 (m, 1H), 5.30 (s, 2H), 5.12-5.06 (m, 1H), 4.79-4.70 (m, 1H), 4.69-4.60 (m, 1H), 4.49-4.40 (m, 1H), 4.31-4.24 (m, 1H), 3.39-3.23 (m, 4H), 2.69-2.62 (m, 2H), 2.51-2.38 (m, 4H), 2.35-2.30 (m, 2H). | 558 (M+H)⁺ |
| 10 | 12 | 40 | 2-(((*R*)-3-((3-((4-cyano-2-fluorobenzyl)ox y)-4-fluorophenyl)am ino)pyrrolidin-1-yl)methyl)-1-(((*S*)-oxetan-2-yl)methyl)-1*H-*benzo[d]imidazo le-6-carboxylic acid | ¹H NMR (400 MHz, DMSOd₆): *δ* 12.74 (brs, 1H), 8.25 (s, 1H), 7.93 (d, *J* =9.6 Hz, 1H), 7.82-7.69 (m, 3H), 7.64 (d, *J* =8.4 Hz, 1H), 6.92 (dd, *J* =11.2, 8.8 Hz, 1H), 6.37 (dd, J =7.6, 2.4 Hz, 1H), 6.09-6.06 (m, 1H), 5.70 (d, *J* =6.8 Hz, 1H), 5.22 (s, 2H), 5.08-5.04 (m, 1H), 4.79-4.72 (m, 1H), 4.62 (dd, *J* =15.0, 2.6 Hz, 1H), 4.48-4.45 (m, 1H), 4.34-4.30 (m, 1H), 4.05 (d, *J* =13.6 Hz, 1H), 3.89 (d, *J* =13.2 Hz, 1H), 3.87-3.83 (m, 1H), 2.85 (dd, *J* =9.4, 6.6 Hz, 1H), 2.70-2.65 (m, 2H), 2.57-2.54 (m, 1H), 2.50-2.34 (m, 2H), 2.24-2.15 (m, 1H), 1.60-1.51 (m, 1H). | 574 (M+H)⁺ |
| 11 | 13 | 40 | (*S*)-2-((4-((3-((4-cyano-2-fluorobenzyl)ox y)-4-fluorophenyl)am ino)piperidin-1-yl)methyl)-1-(oxetan-2-ylmethyl)-1*H-*benzo[d]imidazo le-6-carboxylic acid | ¹H NMR (400 MHz, DMSOd₆): *δ* 12.79 (brs, 1H), 8.27 (s, 1H), 7.94 (dd, *J* =10.0, 1.2 Hz, 1H), 7.82-7.71 (m, 3H), 7.63 (d, *J* =8.8 Hz, 1H), 6.91 (dd, *J* =11.2, 8.8 Hz, 1H), 6.40 (dd, *J* =7.4, 2.6 Hz, 1H), 6.14-6.11 (m, 1H), 5.41 (d, *J* =8.0 Hz, 1H), 5.23 (s, 2H), 5.12-5.08 (m, 1H), 4.78 (dd, *J* =15.2, 7.2 Hz, 1H), 4.64 (d, *J* =12.8 Hz, 1H), 4.51-4.47 (m, 1H), 4.42-4.38 (m, 1H), 3.94 (d, *J* =13.6 Hz, 1H), 3.76 (d, *J* =13.6 Hz, 1H), 3.19-3.16 (m, 1H), 2.90-2.83 (m, 1H), 2.76-2.72 (m, 2H), 2.49-2.39 (m, 1H), 2.25-2.19 (m, 2H), 1.90-1.80 (m, 2H), 1.36-1.24 (m, 2H). | 588 (M+H) ⁺ |
| 12 | 17 | 40 | (*S*)-2-((4-((6-((4-chloro-2-fluorobenzyl)ox y)pyridin-2-yl)amino)piperid in-1-yl)methyl)-1-(oxetan-2-ylmethyl)-1*H-*benzo[d]imidazo le-6-carboxylic acid | ¹H NMR (400 MHz, DMSO-d₆): *δ* 8.27 (d, *J* =1.2 Hz, 1H), 7.80 (dd, *J* =8.4, 1.2 Hz, 1H), 7.64 (d, *J* =8.4 Hz, 1H), 7.49-7.44 (m, 2H), 7.30-7.26 (m, 2H), 6.44 (d, *J* =3.6 Hz, 1H), 6.01 (d, *J* =8.0 Hz, 1H), 5.90 (d, *J* =7.6 Hz, 1H), 5.30 (s, 2H), 5.13-5.07 (m, 1H), 4.78 (dd, *J* =15.2, 7.2 Hz, 1H), 4.64 (dd, *J* =15.2, 2.4 Hz, 1H), 4.53-4.47 (m, 1H), 4.39 (ddd, J =8.8, 5.6, 5.6 Hz, 1H), 3.93 (d, *J* =13.6 Hz, 1H), 3.75 (d, *J* =13.6 Hz, 1H), 3.57 (m, 1H), 2.88-2.85 (m, 1H), 2.76-2.68 (m, 2H), 2.46-2.41 (m, 1H), 2.23-2.12 (m, 2H), 1.80-1.77 (m, 2H), 1.38-1.32 (m, 2H). | 580 (M+H)⁺ |
| 13 | 17 | 41 | (*S*)-2-((4-((6-((4-chloro-2-fluorobenzyl)ox y)pyridin-2-yl)amino)piperid in-1-yl)methyl)-3-oxetan-2-ylmethyl)-3*H-*imidazo[4,5-*b*]pyridine-5-carboxylic acid | ¹H NMR (400 MHz, DMSO-d₆): *δ* 8.07 (d, *J* =8.4 Hz, 1H), 7.99 (d, *J* =8.0 Hz, 1H), 7.48-7.44 (m, 2H), 7.30-7.26 (m, 2H), 6.45 (d, *J* =7.2 Hz, 1H), 6.01 (d, *J* =7.6 Hz, 1H), 5.90 (d, *J* =7.6 Hz, 1H), 5.30 (s, 2H), 5.19 (brs, 1H), 4.77-4.71 (m, 2H), 4.50-4.48 (m, 1H), 4.29 (m, 1H), 3.89 (ddd, J=28.4, 13.6, 13.6 Hz, 1H), 3.57 (m, 2H), 2.86-2.64 (m, 4H), 2.23-2.15 (m, 2H), 1.80 (m, 2H), 1.39-1.34 (m, 2H). | 581 (M+H) ⁺ |
| 14 | 18 | 40 | (*S*)-2-((3-(((6-((4-cyano-2-fluorobenzyl)ox y)pyridin-2-yl)amino)methyl )azetidin-1-yl)methyl)-1-(oxetan-2-ylmethyl)-1*H-*benzo[d]imidazo le-6-carboxylic acid | ¹H NMR (400 MHz, CDCl₃): *δ* 7.96 (d, *J* =8.4 Hz, 1H), 7.74 (d, *J* =8.4, 1H), 7.69 (s, 1H), 7.62 (t, *J* =7.40Hz, 1H), 7.46 (dd, *J =8.0* Hz, 1.2 Hz, 2H), 7.39-7.34(m, 2H), 6.12(d, *J* =7.6 Hz, 1H), 6.01 (d, *J* =8.0 Hz, 1H), 5.42 (s, 2H), 5.07-5.06 (m, 1H), 4.56-4.44 (m, 4H), 4.32-4.2 (m, 3H), 3.90-3.86 (m, 2H), 3.69-3.65 (m, 2H), 3.55 (d, *J* =6.0 Hz, 2H), 2.69-2.60(m, 1H). | 557 (M+H) ⁺ |
| 15 | 19 | 40 | 2-(((*R*)-3-((6-((4-chloro-2-fluorobenzyl)ox y)pyridin-2-yl)amino)pyrroli din-1-yl)methyl)-1-(((*S*)-oxetan-2-yl)methyl)-1*H-*benzo[d]imidazo le-6-carboxylic acid | ¹H NMR (400 MHz, DMSO-d₆): *δ* 8.24 (s, 1H), 7.79 (dd, *J* =8.4, 1.2 Hz, 1H), 7.62 (d, *J* =8.4 Hz, 1H), 7.48-7.41 (m, 2H), 7.31-7.25 (m, 2H), 6.70 (d, *J* =6.4 Hz, 1H), 6.03 (d, *J* =8.0 Hz, 1H), 5.91 *(d, J =7.6* Hz, 1H), 5.27 (s, 2H), 5.08-5.03 (m, 1H), 4.76 (dd, *J* =15.6, 7.6 Hz, 1H), 4.62 (dd, J =15.2, 2.8 Hz, 1H), 4.49-4.43 (m, 1H), 4.32 (ddd, J =8.8, 6.0, 6.0 Hz, 1H), 4.19 (m, 1H), 4.04 (d, *J* =13.2 Hz, 1H), 3.86 (d, *J* =13.2 Hz, 1H), 2.85 (dd, *J* =9.6, 7.2 Hz, 1H), 2.70-2.64 (m, 2H), 2.58-2.54 (m, 1H), 2.42-2.34 (m, 2H), 2.19-2.08 (m, 1H), 1.65-1.60 (m, 1H). | 566 (M+H) ⁺ |
| 16 | 19 | 41 | 2-(((*R*)-3-((6-((4-chloro-2-fluorobenzyl)ox y)pyridin-2-yl)amino)pyrroli din-1-yl)methyl)-3-(((S)-oxetan-2-yl)methyl)-3*H*-imidazo[4,5-*b*]pyridine-5-carboxylic acid | ¹H NMR (400 MHz, DMSO-d₆): *δ* 8.07 (d, *J =8.0* Hz, 1H), 7.97 (d, *J* =8.4 Hz, 1H), 7.47 (dd, *J* =16.4, 16.4 Hz, 1H), 7.42 (dd, J=10.0, 2.0 Hz, 1H), 7.29 *(dd, J* =7.6, 7.6 Hz, 1H), 7.27 (d, *J* =8.0, 1.6 Hz, 1H), 6.72 (d, *J* =6.4 Hz, 1H), 6.03 (d, J =8.0 Hz, 1H), 5.91 (d, *J* =7.6 Hz, 1H), 5.31-5.24 (m, 2H), 5.18-5.12 (m, 1H), 4.81-4.68 (m, 2H), 4.45 (ddd, *J* =13.6, 7.6, 7.6 Hz, 1H), 4.28-4.20 (m, 2H), 4.02 (ddd, *J* =30.4, 13.6, 13.6 Hz, 1H), 2.88 (dd, *J* =9.2, 7.2 Hz, 1H), 2.74-2.56 (m, 3H), 2.20-2.15 (m, 1H), 1.66-1.62 (m, 1H). | 567 (M+H) ⁺ |
| 17 | 20 | 40 | (*S*)-2-((4-((4-((4-cyano-2-fluorobenzyl)ox y)pyrimidin-2-yl)amino)piperid in-1-yl)methyl)-1-(oxetan-2-ylmethyl)-1*H-*benzo[d]imidazo le-6-carboxylic acid | ¹H NMR (400 MHz, DMSO-d₆): *δ* 12.75 (brs, 1H), 8.26 (s, 1H), 8.08-7.98 (m, 1H), 7.90 (dd, *J* =21.0, 9.0 Hz, 1H), 7.81 (dd, *J* =8.6, 1.4 Hz, 1H), 7.76-7.63 (m, 1H), 7.63 (d, *J* =8.4 Hz, 1H), 7.15-7.08 (m, 1H), 6.09 (d, *J* =5.2 Hz, 1H), 5.46 (s, 2H), 5.14-5.07 (m, 1H), 4.77 (dd, *J* =15.2, 7.2 Hz, 1H), 4.63 (d, *J* =12.8 Hz, 1H), 4.51-4.47 (m, 1H), 4.41-4.38 (m, 1H), 3.93 (d, *J* =13.6 Hz, 1H), 3.74 (d, *J* = 13.2 Hz, 1H), 3.58-3.41 (m, 2H), 2.93-2.82 (m, 1H), 2.76-2.67 (m, 2H), 2.48-2.40 (m, 1H), 2.22-2.11 (m, 2H), 1.88-1.63 (m, 2H), 1.56-1.33 (m, 2H). | 572 (M+H)⁺ |
| 18 | 24 | 40 | 2-((2-(((6-((4-cyano-2-fluorobenzyl)ox y)pyridin-2-yl)amino)methyl )pyrrolidin-1-yl)methyl)-1-(((*S*)-oxetan-2-yl)methyl)-1*H-*benzo[d]imidazo le-6-carboxylic acid | ¹H NMR (400 MHz, DMSO-d₆): *δ* 8.21 (d, *J* =2.8 Hz, 1H), 7.89-7.84 (m, 1H), 7.79 (dd, *J* =8.4, 1.6 Hz, 1H), 7.71-7.60 (m, 2H), 7.31 (td, *J* =12.3, 7.9 Hz, 1H), 6.61-6.48 (m, 1H), 6.07 (dd, *J* =30.4, 8.0 Hz, 1H), 5.95 (dd, *J* =11.6, 7.6 Hz, 1H), 5.42-5.37 (m, 2H), 5.09-5.05 (m, 1H), 4.72-4.67 (m, 1H), 4.50-4.28 (m, 3H), 3.55-3.45 (m, 1H), 3.13-3.05 (m, 1H), 2.99-2.91 (m, 1H), 2.84-2.75 (m, 2H), 2.73-2.66 (m, 1H), 2.65-2.53 (m, 1H), 2.42-2.21 (m, 2H), 1.91-1.80 (m, 1H), 1.68-1.50 (m, 3H), 1.31-1.25 (m, 1H). | 571 (M+H)⁺ |
| | | | | | |
| 19 | 27 | 40 | (*S*)-2-((4-((6-((4-cyano-2- fluorobenzyl)ox y)pyrazin-2-yl)amino)piperid in-1-yl)methyl)-1-(oxetan-2-ylmethyl)-1*H-*benzo[d]imidazo le-6-carboxylic acid | ¹H NMR (400 MHz, DMSO-d₆): *δ* 12.80 (brs, 1H), 8.27 (d, J =0.8 Hz, 1H), 7.92 (dd, *J* =9.8, 1.4 Hz, 1H), 7.81 (dd, J =8.4, 1.6 Hz, 1H), 7.71 (dd, J =7.8, 1.4 Hz, 1H), 7.64 (t, *J* =8.0 Hz, 2H), 7.47 (s, 1H), 7.35 (s, 1H), 7.06 (d, *J* =7.2 Hz, 1H), 5.43 (s, 2H), 5.11-5.08 (m, 1H), 4.78 (dd, *J* =15.2, 7.2 Hz, 1H), 4.64 (dd, *J* =15.2, 2.4 Hz, 1H), 4.53-4.47 (m, 1H), 4.39 (td, J =9.1, 5.9 Hz, 1H), 3.94 (d, *J* =13.6 Hz, 1H), 3.77 (d, *J* =13.6 Hz, 1H), 3.53-3.50 (m, 1H), 2.88-2.84 (m, 1H), 2.76-2.71 (m, 2H), 2.47-2.41 (m, 1H), 2.23-2.14 (m, 2H), 1.79-1.73 (m, 2H), 1.39-1.28 (m, 2H). | 572 (M+H)⁺ |
| 20 | 28 | 40 | 2-((3-((6-((4-cyano-2-fluorobenzyl)ox y)pyridin-2-yl)amino)pyrroli din-1-yl)methyl)-1-(((*S*)-oxetan-2-yl)methyl)-1*H-*benzo[d]imidazo le-6-carboxylic acid | ¹H NMR (400 MHz, CDCl₃): *δ* 8.02 (d, *J* =8.8 Hz, 1H), 7.97 (s, 1H), 7.80 (d, *J* = 8.8 Hz, 1H), 7.63 (t, *J* = 7.6 Hz, 1H), 7.45 (d, *J* = 7.6 Hz), 7.37 (d, *J* = 9.2 Hz, 2H), 6.11 (d, *J=* 8.0 Hz, 1H), 5.99 (d, *J* = 8.0 Hz, 1H), 5.42 (s, 2H), 5.14 (brs, 1H), 4.62-4.51 (m, 4H), 4.34-4.4.21 (m, 2H), 4.15(brs, 1H), 2.71(brs, 1H), 2.39-2.35 (m, 4H), 1.92-1.78 (m, 2H) | 557 (M+H) ⁺ |
| 21 | 32 | 40 | 2-(((*S*)-3-((6-((4-cyano-2-fluorobenzyl)ox y)pyridin-2-yl)amino)pyrroli din-1-yl)methyl)-1-(((*S*)-oxetan-2-yl)methyl)-1*H-*benzo[*d*]imidazo le-6-carboxylic acid | ¹H NMR (400 MHz, CDCl₃): *δ* 8.04 (d, *J=* 9.6 Hz, 1H), 7.96 (s, 1H), 7.81 (d, *J* = 8.4 Hz, 1H), 7.63 (t, *J* = 7.6 Hz, 1H), 7.46 (d, *J=* 8.8 Hz, 1H), 7.38-7.32 (m, 2H), 6.11 (d, *J* = 8.0 Hz, 1H), 5.98 (d, *J* = 8.0 Hz, 1H), 5.42 (s, 2H), 5.14 (brs, 1H), 4.64-4.54 (m, 4H), 4.37-4.33 (m, 2H), 4.19 (s, 2H), 3.18-3.09 (m, 1H), 2.97-2.92 (m, 3H), 2.76-2.70 (m, 4H), 2.47-2.34 (m, 4H) | 557 (M+H)⁺ |
| 22 | 34 | 40 | 2-(((*R*)-3-((4-((4-cyano-2-fluorobenzyl)ox y-5-fluoropyrimidin-2-yl)amino)pyrroli din-1-yl)methyl)-1-(((*S*)-oxetan-2-yl)methyl)-1*H-*benzo[d]imidazo le-6-carboxylic acid | ¹H NMR (400 MHz, DMSO-d₆): *δ* 8.24 (d, *J =0.8* Hz, 1H), 8.14 (d, *J* =3.2 Hz, 1H), 7.92 (d, *J* =9.6 Hz, 1H), 7.79 (dd, *J* =8.4, 1.6 Hz, 1H), 7.74 (m, 2H), 7.62 (d, *J* =8.4 Hz, 1H), 7.39 (s, 1H), 5.53 (m, 2H), 5.09-5.03 (m, 1H), 4.76 (dd, *J* =15.2, 7.2 Hz, 1H), 4.61 (dd, *J* =15.2, 2.8 Hz, 1H), 4.49-4.44 (m, 1H), 4.33 (ddd, *J* =8.8, 5.6, 5.6 Hz, 1H), 4.18 (m, 1H), 4.04 (d, *J* =13.6 Hz, 1H), 3.87 (d, *J* =13.2 Hz, 1H), 2.85-2.81 (m, 1H), 2.73-2.66 (m, 2H), 2.42-2.34 (m, 1H), 2.16-2.08 (m, 1H), 1.72 (m, 1H). | 576 (M+H)⁺ |
| 23 | 36 | 40 | 2-(((*R*)-3-((2-((4-cyano-2-fluorobenzyl)ox y)pyrimidin-4-yl)amino)pyrroli din-1-yl)methyl)-1-(((*S*)-oxetan-2-yl)methyl)-1*H-*benzo[d]imidazo le-6-carboxylic acid | ¹H NMR (400 MHz, DMSO-d₆): *δ* 8.24 (s, 1H), 7.89 (d, *J* =10.0 Hz, 1H), 7.81-7.78 (m, 2H), 7.72-7.70 (m, 2H), 7.65-7.61 (m, 2H), 6.16 (d, *J* =6.0 Hz, 1H), 5.37 (s, 2H), 5.08-5.03 (m, 1H), 4.75 (dd, *J* =15.2, 7.2 Hz, 1H), 4.61 (dd, *J* =15.2, 2.4 Hz, 1H), 4.49-4.44 (m, 1H), 4.32 (ddd, *J* =8.8, 5.6, 5.6 Hz, 1H), 4.04 (d, *J* =13.6 Hz, 1H), 3.88 (d, *J* =13.6 Hz, 1H), 2.83-2.79 (m, 1H), 2.74-2.65 (m, 2H), 2.41-2.33 (m, 1H), 2.20-2.15 (m, 1H), 1.63 (m, 1H). | 558 (M+H)⁺ |
| 24 | 7 | 40 | 2-((3-((6-((4-cyano-2-fluorobenzyl)ox y)pyridin-2-yl)methyl)pyrrol idin-1-yl)methyl)-1-(((*S*)-oxetan-2-yl)methyl)-1*H-*benzo[d]imidazo le-6-carboxylic acid | ¹H NMR (400 MHz, DMSO-d₆): *δ* 8.20 (s, 1H), 7.87 (d, *J* =6.8 Hz, 1H), 7.75 (dd, *J* =6.8, 1.6 Hz, 1H), 7.69-7.57 (m, 4H), 6.85 (d, *J* =4.8 Hz, 1H), 6.69 (dd, *J* =6.4, 2.4 Hz, 1H), 5.38-5.35 (m, 2H), 5.07-5.04 (m, 1H), 4.74-4.73 (m, 1H), 4.62-4.56 (m, 1H), 4.48-4.45 (m, 1H), 4.35-4.32 (m, 1H), 3.99 (dd, *J* =10.8, 2.8 Hz, 1H), 3.82 (d, *J* =10.8 Hz, 1H), 2.65-2.55 (m, 7H), 2.36 (m, 1H), 2.29 (m, 1H), 1.80 (m, 1H), 1.41 (m, 1H) | 556 (M+H)⁺ |
| 25 | 15 | 40 | 2-(((*R*)-3-((3-((4-chloro-2-fluorobenzyl)ox y)-4-fluorophenyl)amino)pyrrolidin-1-yl)methyl)-1-(((*S*)-oxetan-2-yl)methyl)-1*H-*benzo[d]imidazo le-6-carboxylic acid | ¹H NMR (400 MHz, DMSO-d₆): *δ* 12.75 (brs, 1H), 8.26 (s, 1H), 7.80 (dd, *J* =8.4, 1.6 Hz, 1H), 7.65 (d, *J* =8.4 Hz, 1H), 7.54 (t, *J* =8.2 Hz, 1H), 7.48 (dd, *J* =10.0, 2.0 Hz, 1H), 7.33 (dd, *J* =8.2, 1.8 Hz, 1H), 6.91 (dd, *J* =11.4, 9.0 Hz, 1H), 6.38 (dd, *J* =7.4, 2.6 Hz, 1H), 6.08-6.05 (m, 1H), 5.68 (d, *J* =4.8 Hz, 1H), 5.11 (s, 2H), 5.06-5.03 (m, 1H), 4.75 (dd, *J* =15.4, 7.4 Hz, 1H), 4.62 (dd, *J* =15.0, 2.6 Hz, 1H), 4.47-4.43 (m, 1H), 4.35-4.29 (m, 1H), 4.17-3.98 (m, 1H), 3.92-3.83 (m, 1H), 3.01-2.73 (m, 2H), 2.72-2.58 (m, 4H), 2.40-2.31 (m, 1H), 2.29-2.16 (m, 1H), 1.68-1.55 (m, 1H). | 583 (M+H)⁺ |
| 26 | 15 | 41 | 2-(((R)-3-((3-((4-chloro-2-fluorobenzyl)ox y)-4-fluorophenyl)am ino)pyrrolidin-1-yl)methyl)-3-(((*S*)-oxetan-2-yl)methyl)-3*H*-imidazo[4,5-b]pyridine-5-carboxylic acid | ¹H NMR (400 MHz, DMSO-d₆): *δ* 13.01 (brs, 1H), 8.13 (d, *J* =8.0 Hz, 1H), 7.99 (d, *J* =8.4 Hz, 1H), 7.54 (t, *J* =8.2 Hz, 1H), 7.48 (dd, *J* =10.0, 2.0 Hz, 1H), 7.33 (dd, *J* =8.2, 1.8 Hz, 1H), 6.89 (dd, *J* =11.6, 8.8 Hz, 1H), 6.38 (dd, *J* =7.4, 2.6 Hz, 1H), 6.07 (td, *J* =8.8, 3.0 Hz, 1H), 5.66 (d, J =6.8 Hz, 1H), 5.15-5.10 (m, 3H), 4.79 (dd, *J* =14.6, 6.2 Hz, 1H), 4.68 (dd, J =14.8, 4.0 Hz, 1H), 4.49-4.43 (m, 1H), 4.30 (td, J =9.1, 6.1 Hz, 1H), 4.06 (q, J =12.8 Hz, 2H), 3.91-3.78 (m, 1H), 2.92-2.88 (m, 1H), 2.75-2.58 (m, 3H), 2.51-2.44 (m, 2H), 2.23-2.18 (m, 1H), 1.60-1.55 (m, 1H). | 584 (M+H) ⁺ |
| 27 | 16 | 40 | (S)-2-((4-((3-((4-chloro-2-fluorobenzyl)ox y)-4-fluorophenyl)am ino)piperidin-1-yl)methyl)-1-(oxetan-2-ylmethyl)-1H-benzo[d]imidazo le-6-carboxylic acid | ¹H NMR (400 MHz, DMSO-d₆): δ 12.32 (brs, 1H), 8.27 (brs, 1H), 7.81 (m, 1H), 7.65 (m, 1H), 7.56 (dd, J =8.0, 8.0 Hz, 1H), 7.50 (d, J =10.0 Hz, 1H), 7.34 (d, J =8.0 Hz, 1H), 6.89 (m, 1H), 6.40 (m, 1H), 6.12 (m, 1H), 5.36 (m, 1H), 5.12 (m, 3H), 4.79 (dd, J =15.2, 6.8 Hz, 1H) ,4.66-4.62, 4.49 (ddd, J =13.6, 7.2, 7.2 Hz, 1H), 4.38 (m, 1H), 3.95-3.77 (m, 2H), 3.18 (m, 1H), 2.86 (m, 1H), 2.74-2.67 (m, 2H), 2.22 (m, 2H), 1.85 (m, 2H), 1.36-1.24 (m, 2H). | 597 (M+H)⁺ |
| 28 | 16 | 41 | (S)-2-((4-((3-((4-chloro-2-fluorobenzyl)ox y)-4-fluorophenyl)am ino)piperidin-1-yl)methyl)-3-(oxetan-2-ylmethyl)-3H-imidazo[4,5-b]pyridine-5-carboxylic acid | ¹H NMR (400 MHz, DMSO-d₆): δ 12.92 (brs, 1H), 8.15 (d, J=6.0 Hz, 1H), 8.00 (d, J=6.4 Hz, 1H), 7.56 (t, J =6.4 Hz, 1H), 7.50 (dd, J=8.0, 1.2 Hz, 1H), 7.34 (d, J =6.4 Hz, 1H), 6.89 (dd, J =8.8, 7.2 Hz, 1H), 6.40 (d, J =4.4 Hz, 1H), 6.11 (d, J =6.8 Hz, 1H), 5.39 (brs, 1H), 5.16-5.11 (m, 3H), 4.84 (dd, J=11.6, 5.2 Hz, 1H), 4.72 (dd, J=11.6, 3.6 Hz, 1H), 4.49 (q, J =5.6 Hz, 1H), 4.40-4.35 (m, 1H), 4.03-3.93 (m, 2H), 3.26-3.12 (m, 1H), 2.89-2.73 (m, 2H), 2.71-2.64 (m, 1H), 2.32-2.21 (m, 2H), 1.94-1.81 (m, 2H), 1.37-1.23 (m, 3H). | 598 (M+H) ⁺ |
| 29 | 25 | 40 | 2-(((R)-3-((4-((4-chloro-2-fluorobenzyl)oxy)-5-fluoropyrimidin-2-yl)amino)pyrroli din-1-yl)methyl)-l-(((S)-oxetan-2-yl)methyl)-1H-benzo[d]imidazo le-6-carboxylic acid | ¹H NMR (400 MHz, DMSO-d₆): δ 8.24 (s, 1H), 8.11 (d, J =3.2 Hz, 1H), 7.79 (dd, J =8.4, 1.2 Hz, 1H), 7.64-7.57 (m, 2H), 7.48 (d, *J =9.6* Hz, 1H), 7.33-7.31 (m, 2H), 5.43 (s, 2H), 5.08-5.03 (m, 1H), 4.79-4.73 (m, 1H), 4.64-4.60 (m, 1H), 4.49-4.44 (m, 1H), 4.36-4.31 (m, 1H), 4.22-4.21 (m, 1H), 4.05 (d, *J* =13.6 Hz, 1H), 3.87 (d, J =13.2 Hz, 1H), 2.88-2.84 (m, 1H), 2.71-2.65 (m, 2H), 2.60-2.55 (m, 1H), 2.41-2.36 (m, 1H), 2.19-2.12 (m, 1H), 1.73 (m, 1H). | 585 (M+H) ⁺ |
| 30 | 26 | 40 | 2-(((R)-3-((6-((4-chloro-2-fluorobenzyl)ox y)pyrazin-2-yl)amino)pyrroli din-1-yl)methyl)-1-(((S)-oxetan-2-yl)methyl)-1H-benzo[d]imidazo le-6-carboxylic acid | ¹H NMR (400 MHz, DMSO-d₆): *δ* 8.23 (s, 1H), 7.80-7.77 (m, 1H), 7.61 (d, *J* =8.4 Hz, 1H), 7.51-7.47 (m, 2H), 7.43 (dd, *J* =10.0, 2.0 Hz, 1H), 7.31-7.26 (m, 3H), 5.34-5.30 (m, 2H), 5.07-5.05 (m, 1H), 4.78-4.73 (m, 1H), 4.64-4.60 (m, 1H), 4.46 (ddd, *J* =13.6, 7.6, 7.6 Hz, 1H), 4.34-4.29 (m, 1H), 4.22 (m, 1H), 4.05 (d, J =13.6 Hz, 1H), 3.89 (d, J =13.6 Hz, 1H), 2.89-2.85 (m, 1H), 2.74-2.63 (m, 2H), 2.59-2.56 (m, 1H), 2.41-2.33 (m, 2H), 2.23-2.07 (m, 1H), 1.67-1.63 (m, 1H). | 567 (M+H)⁺ |
| 31 | 37 | 40 | 2-(((R)-3-((2-((4-chloro-2-fluorobenzyl)ox y)-5-fluoropyrimidin-4-yl)amino)pyrroli din-1-yl)methyl)-1-(((S)-oxetan-2-yl)methyl)-1H-benzo[d]imidazo le-6-carboxylic acid | ¹H NMR (400 MHz, DMSO-d₆): *δ* 8.23 (s, 1H), 7.93 (d, J =3.6 Hz, 1H), 7.82 (d, J =6.4 Hz, 1H), 7.79 (dd, J =8.4, 1.6 Hz, 1H), 7.61 (d, J =8.4 Hz, 1H), 7.49 (dd, J =8.4, 8.4 Hz, 1H), 7.44 (dd, 7=10.0, 2.0 Hz, 1H), 7.29 (dd, J=8.4, 1.6 Hz, 1H), 5.25 (m, 2H), 5.07-5.04 (m, 1H), 4.73-4.71 (m, 1H), 4.64-4.60 (m, 1H), 4.47-4.31 (m, 3H), 4.05 (d, J =13.6 Hz, 1H), 3.90 (d, J=13.6 Hz, 1H), 2.90-2.86 (m, 1H), 2.71-2.65 (m, 2H), 2.59-2.55 (m, 2H), 2.48 (m, 1H), 2.16 (m, 1H), 1.84-1.82 (m, 1H). | 585 (M+H) ⁺ |

### 2. Preparation Example B

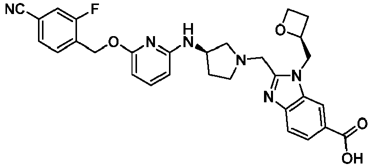

### (1) Synthesis of Example 32: 2-(((R)-3-((6-((4-cyano-2-fluorobenzyl)oxy)pyridin-2-yl)amino)pyrrolidin-1-yl)methyl)-1-(((S)-oxetan-2-yl)methyl)-1H-benzo[d]imidazole-6-carboxylic acid

### 1) Synthesis of methyl 2-(((R)-3-((6-((4-cyano-2-fluorobenzyl)oxy)pyridin-2-yl)amino)pyrrolidin-1-yl)methyl)-1-(((S)-oxetan-2-yl)methyl)-1H-benzo[d]imidazole-6-carboxylic acid

Intermediate 30 (0.35 mmol), potassium carbonate (152 mg), and Intermediate 40 (118 mg) were dissolved in CH₃CN (5.0 mL), which was stirred at 80 to 90°C for 4 hours. The reaction product was cooled to room temperature. Then, the obtained mixture was filtered through a Celite pad and the filtrate was concentrated. The concentrated residue was separated and purified by silica gel column chromatography (12 g SiO₂, 50% EA-> 100% EA) to obtain the target compound (130 mg) as a white solid at a yield of 66%. LC-MS(ES⁺): 571 [M+H]⁺.

### 2) Synthesis of final compound

The compound (120 mg) obtained in the step 1) was dissolved in 1,4-dioxane/H₂O (4/1, 2.0 mL). Then, 1*N* aqueous NaOH solution (0.3 mL) was added dropwise and the mixture was stirred at room temperature for 24 hours. The reaction product was cooled to room temperature, neutralized with 1*N* HCl and extracted with 5% DCM/MeOH solution. The extracted organic layer was dried over anhydrous magnesium sulfate, filtered, concentrated, separated, and purified by silica gel column chromatography (DCM: MeOH: AcOH = 10: 1: 0.01) to obtain the final compound (72 mg) as a white solid at a yield of 62%. ¹H NMR (400 MHz, CDCl₃): *δ* 8.01 (d, *J* =8.0 Hz, 1H), 7.89 (s, 1H), 7.80 (d, *J=* 7.4 Hz, 1H), 7.63 (d, *J=* 8.0 Hz, 1H), 7.38 (m, 2H), 6.11 (d, *J=* 8.0 Hz, 1H), 6.00 (d, *J =* 8.0 Hz, 1H), 5.42 (s, 2H), 5.13 (brs, 1H), 4.69-4.59 (m, 4H), 4.42-4.33 (m, 5H), 4.05(brs, 1H), 3.13-2.35 (m, 4H), 1.92-1.78 (m, 2H); LC-MS(ES⁺): 557 [M+H]⁺.

### (2) Synthesis of Examples 33 to 38

The compounds listed as Examples 33 to 38 in Table 3 below were prepared by using procedures identical or analogous to the synthesis method (Preparation Example B) of Example 32 from appropriate starting materials which are available commercially or prepared by preparation methods well known to those skilled in the art. The compounds were purified using methods well known to those skilled in the art, which may include silica gel column chromatography, HPLC, or recrystallization from the reaction mixture. The final compounds could be isolated as neutrals or as acid addition or base addition salts. The compound names, NMR data, and LC-MS data of the Examples prepared above are shown in Table 3 below.

**Table 3**

| Example No. | No. of Intermediate A | No. of Intermediate B | Compound name | NMR data | LC/MS data (ES+) |
|---|---|---|---|---|---|
| 33 | 21 | 40 | 2-(((R)-3-((3-((5-chloropyridin-2-yl)methoxy)ph enyl)amino)py rrolidin-1-yl)methyl)-1-(((S)-oxetan-2-yl)methyl)-1H-benzo[d]imida zole-6-carboxylic acid | ¹H NMR (400 MHz, CDCl₃): *δ* 8.57 (d, *J* =2.4 Hz, 1H), 8.05-8.03 (m, 2H), 7.81 (d, *J* =8.4 Hz, 1H), 7.70 (dd, *J* =8.4, 2.4 Hz, 1H), 7.50 (d, *J* =8.4 Hz, 1H), 7.07(t, J =8.0 Hz, 2H), 6.33-6.30 (m, 1H), 6.23-6.21 (m, 1H), 5.18-5.17 (m, 1H), 5.15 (s, 2H), 4.65-4.59 (m, 3H), 4.38-4.33 (m, 1H), 4.18 (s, 2H), 4.07 (brs, 1H), 3.10-3.08 (m, 1H), 3.06-2.96(m, 4H), 2.75-2.68 (m, 4H), 2.45-2.26 (m, 3H), 1.80-1.76 (m, 1H). | 548.4 (M+H) ⁺ |
| 34 | 22 | 40 | 2-((3-((6-((4-cyano-2-fluorobenzyl)o xy)pyridin-2-yl)amino)piper idin-1-yl)methyl)-1-(((S)-oxetan-2-yl)methyl)-1*H-*benzo[d]imida zole-6-carboxylic acid | ¹H NMR (400 MHz, CDCl₃): *δ* 8.13 (d, J=11.6 Hz, 1H), 8.02 (dd, J=8.6, 4.2 Hz, 1H), 7.79 (dd, J=8.4, 4.0 Hz, 1H), 7.62-7.41(m, 1H), 7.45 (d, *J* =7.6 Hz, 1H), 7.38 (d, J=9.2 Hz, 1H), 7.35-7.31 (m, 2H), 6.06 (dd, J=7.8, 3.4 Hz, 1H), 5.96 (d, J =8.0 Hz, 1H), 5.41-5.38(m, 1H), 5.24-5.20 (m, 2H), 4.82-4.78 (m, 1H), 4.68-4.60 (m, 3H), 4.40-4.37 (m, 1H), 4.03-3.85 (m, 3H), 2.92-2.73 (m, 2H), 2.63-2.38 (m, 4H). | 571.5 (M+H) ⁺ |
| 35 | 23 | 40 | 2-(((R)-3-((6-((4-cyano-2-fluorobenzyl)o xy)pyrazin-2-yl)amino)pyrro lidin-1-yl)methyl)-1-(((S)-oxetan-2-yl)methyl)-1H-benzo[d]imida zole-6-carboxylic acid | ¹H ¹H NMR (400 MHz, CDCl₃): *δ* 7.99 (s, 2H), 7.79 (brs, 1H), 7.56 (t, J =6.60 Hz, 1H), 7.51-7.49 (m, 2H), 7.46-7.42 (m, 1H), 7.36 (dd, J =8.6, 5.0 Hz, 1H), 6.11 (brs, 1H), 5.41 (s, 2H), 5.17 (brs, 1H), 4.61-4.57 (m, 3H), 4.32 (s, 2H), 4.17-4.09 (m, 1H), 3.9 (brs, 1H), 3.09 (brs, 2H), 2.90-2.82 (m, 2H), 2.38 (brs, 2H), 1.83 (brs, 1H). | 558 (M+H) ⁺ |
| 36 | 31 | 40 | (S)-2-((4-((6-((4-cyano-2-fluorobenzyl)o xy)pyridin-2-yl)(methyl)ami no)piperidin-1-yl)methyl)-1-(oxetan-2-ylmethyl)-1H-benzo[d]imida zole-6-carboxylic acid | ¹H NMR (400 MHz, DMSO-d₆): *δ* 8.26 (s, 1H), 7.91 (dd, J =10.0, 1.2 Hz, 1H), 7.80 (dd, *J*= 8.8, 1.2 Hz, 1H), 7.69 (dd, J = 8.0, 1.2, 1H), 7.63 (d, *J* = 8.4 Hz, 1H), 7.58 (t, *J* = 7.6 Hz, 1H), 7.44 (t, *J =* 7.8 Hz, 1H), 6.14 (d, J = 8.0 Hz, 1H), 6.07 (d, *J* = 7.6 Hz, 1H), 5.44 (s, 2H), 5.12-5.06 (m, 1H), 4.79 (dd, J = 15.4, 7.0 Hz, 1H), 4.67-4.63 (m, 1H), 4.53-4.47 (m, 1H), 4.40-4.35 (m, 1H), 4.22-4.16 (m, 1H), 3.93 (d, J = 13.6 Hz, 1H), 3.78 (d, J = 13.2 Hz, 1H), 2.87-2.75 (m, 2H), 2.74-2.73 (m, 1H), 2.46-2.39 (m, 1H), 2.18-2.06 (m, 2H), 1.70-1.59 (m, 2H), 1.43-1.42 (m, 2H) | 585 (M+H) ⁺ |
| 37 | 33 | 40 | 2-(((R)-3-((3-((4-cyano-2-fluorobenzyl)o xy)phenyl)ami no)pyrrolidin-1-yl)methyl)-1-(((S)-oxetan-2-yl)methyl)-*1H-*benzo[d]imida zole-6-carboxylic acid | ¹H NMR (400 MHz, DMSO-d₆): *δ* 8.25 (s, 1H), 7.91 (d, *J* =10.0 Hz, 1H), 7.81 (d, *J* =8.4 Hz, 1H), 7.75-7.69 (m, 2H), 7.65-7.63 (m, 1H), 6.96 (dd, *J* =8.0, 8.0 Hz, 1H), 6.20-6.15 (m, 3H), *5.82 (d, J* =6.0 Hz, 1H), 5.14 (s, 2H), 5.09-5.03 (m, 1H), 4.78-4.72 (m, 1H), 4.63-4.60 (m, 1H), 4.46 (d, *J* =8.8, 5.6 Hz, 1H), 4.34-4.29 (m, 1H), 4.08-4.04 (m, 1H), 3.91-3.88 (m, 2H), 2.87 (m, 1H), 2.71-2.63 (m, 2H), 2.57-2.55 (m, 1H), 2.39-2.34 (m, 1H), 2.22-2.19 (m, 1H), 1.61-1.59 (m, 1H). | 556 (M+H) ⁺ |
| 38 | 39 | 40 | 2-((3-((6-((4-chloro-2-fluorobenzyl)a mino)pyridin-2-yl)oxy)pyrrolid in-1-yl)methyl)-1-(((S)-oxetan-2-yl)methyl)-1H-benzo[d]imida zole-6-carboxylic acid | ¹H NMR (400 MHz, DMSO-d₆): *δ* 12.79 (brs, 1H), 8.25 (s, 1H), 7.80 (dd, J=8.4, 1.2 Hz, 1H), 7.64 (d, J=8.4 Hz, 1H), 7.35 (dd, J =10.2, 2.2 Hz, 1H), 7.31-7.27 (m, 1H), 7.20 (dd, J=8.4, 1.6 Hz, 1H), 7.13 (t, J =5.8 Hz, 1H), 6.08 (d, J=8.0 Hz, 1H), 5.86 (d, *J*=8.0 Hz, 1H), 5.14 (brs, 1H), 5.08-5.02 (m, 1H), 4.74 (dd, J =15.2, 7.2 Hz, 1H), 4.60 (dd, J =15.2, 2.4 Hz, 1H), 4.49-4.41 (m, 3H), 4.39-4.37 (m, 1H), 3.85-3.84 (brs, 1H), 2.78-2.64 (m, 4H), 2.41-2.34 (m, 1H), 2.12-1.99 (m, 1H), 1.72 (brs, 1H). | 566.1 (M+H) ⁺ |

### 3. Preparation Example C

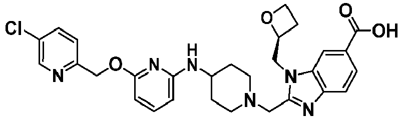

### (1) Synthesis of Example 39: (S)-2-((4-((6-((5-chloropyridin-2-yl)methoxy)pyridin-2-yl)amino)piperidin-1-yl)methyl)-1-(oxetan-2-ylmethyl)-1H-benzo[d]imidazole-6-carboxylic acid

### 1) Synthesis of methyl (S)-2-((4-((6-((5-chloropyridin-2-yl)methoxy)pyridin-2-yl)amino)piperidin-1-yl)methyl)-1-(oxetan-2-ylmethyl)-1H-benzo[d]imidazole-6-carboxylate

Intermediate 5 (crude), Intermediate 40 (47 mg), and potassium carbonate (90 mg) were placed in a round bottom flask and dissolved in CH₃CN (5 mL). The resulting mixture was stirred at 60°C for one day. After completion of the reaction as indicated by TLC, the solution was diluted with ethyl acetate and the organic layer was washed with saturated brine. The organic layer was dried over anhydrous magnesium sulfate and filtered under reduced pressure to obtain a filtrate. The filtrate was concentrated under reduced pressure, which was subjected to silica gel column chromatography eluting with hexane/ethyl acetate to obtain the target compound (86 mg) as a transparent syrup at a yield of 93%.

### 2) Synthesis of final compound

The compound (86 mg) synthesized in the step 1) was placed in a round bottom flask, dissolved in CH₃CN (10 mL), and stirred. TBD 1.0M aqueous solution (0.3 mL) was added dropwise and stirred. Then, purified water (1 mL) was added and the reaction mixture was stirred at room temperature for one day. After completion of the reaction as indicated by TLC, the solution was neutralized to pH 7 with 1*N* HCl aqueous solution. The compound was extracted using DCM/MeOH 10% solution, dried over anhydrous magnesium sulfate, and filtered under reduced pressure to obtain a filtrate. The filtrate was concentrated under reduced pressure, which was subjected to silica gel column chromatography eluting with DCM/MeOH to obtain the final compound (7 mg) as a white solid at a yield of 8%. ¹H NMR (DMSO-d₆): *δ* 8.57 (d, *J=* 2.4 Hz, 1H), 8.23 (s, 1H), 7.89 (dd, *J=* 8.4 Hz, *J* = 2.8 Hz, 1H), 7.79 (d, *J=* 8.8 Hz, 1H), 7.61 (d, *J=* 8.4 Hz, 1H), 7.35 (d, *J* = 8.4 Hz, 1H), 7.28 (t, *J* = 80 Hz, 1H), 6.41 (d, *J* = 7.2 Hz, 1H), 5.97 (dd, *J* = 16.4 Hz, *J=* 8.0 Hz, 2H), 5.31 (s, 2H), 5.09-5.04 (m, 1H), 4.76 (dd, *J=* 14.8 Hz, *J=* 7.2 Hz, 1H), 4.63-4.59 (m, 1H), 4.49-4.46 (m, 1H), 4.40-4.35 (m, 1H), 3.90 (d, *J=* 13.6, 1H), 3.72 (d, *J=* 13.2, 1H), 2.79 (d, *J=* 11.2 Hz, 1H), 2.72-2.66 (m, 2H), 2.46-2.39 (m, 2H), 2.13-2.02 (m, 2H), 1.66 (brs, 2H), 1.29-1.23 (m, 2H); LC-MS(ES⁺): 563 [M+H]⁺.

### (2) Synthesis of Examples 40 to 44

The compounds listed as Examples 40 to 44 in Table 4 below were prepared by using procedures identical or analogous to the synthesis of Example 39 from appropriate starting materials which are available commercially or prepared by preparation methods well known to those skilled in the art. The compounds were purified using methods well known to those skilled in the art, which may include silica gel chromatography, HPLC, or recrystallization from the reaction mixture. The final compounds could be isolated as neutrals or as acid addition or base addition salts. The compound names, NMR data, and LC-MS data of the Examples prepared above are shown in Table 4 below.

**[Table 4]**

| Example No. | No. of Intermediate A | No. of Intermediate B | Compound name | NMR data | LC/MS data (ES+) |
|---|---|---|---|---|---|
| 39 | 5 | 40 | (S)-2-((4-((6-((5-chloropyridin-2-yl)methoxy)py ridin-2-yl)amino)piper idin-1-yl)methyl)-1-(oxetan-2-ylmethyl)-1*H-*benzo[d]imida zole-6-carboxylic acid | ¹H NMR (400 MHz, DMSO-d₆): *δ*8.57 (d, *J*= 2.4Hz, 1H), 8.23 (s, 1H), 7.89 (dd,J= 8.4 Hz, *J =* 2.8 Hz, 1H), 7.79 (d, *J* = 8.8 Hz, 1H), 7.61 (d, *J* = 8.4 Hz, 1H), 7.35 (d, *J =* 8.4 Hz, 1H), 7.28 (t, *J=* 8.0 Hz, 1H), 6.41 (d, *J=* 7.2 Hz, 1H), 5.97 (dd, *J=* 16.4 Hz, *J=* 8.0 Hz, 2H), 5.31 (s, 2H), 5.09-5.04 (m, 1H), 4.76 (dd, *J* = 14.8 Hz, *J* = 7.2 Hz, 1H), 4.63-4.59 (m, 1H), 4.49-4.46 (m, 1H), 4.40-4.35 (m, 1H), 3.90 (d, *J =* 13.6, 1H), 3.72 (d, *J=* 13.2, 1H), 2.79 (d, *J* = 11.2 Hz, 1H), 2.72-2.66 (m, 2H), 2.46-2.39 (m, 2H), 2.13-2.02 (m, 2H), 1.66 (brs, 2H), 1.29-1.23 (m, 2H) | 563 (M+H) ⁺ |
| 40 | 10 | 40 | (S)-2-((4-((6-((5-cyanopyridin-2-yl)methoxy)py ridin-2-yl)amino)piper idin-1-yl)methyl)-1-(oxetan-2-ylmethyl)-1H-benzo[d]imida zole-6-carboxylic acid | ¹H NMR (400 MHz, DMSO-d₆): *δ* 9.0 (s, 1H), 8.27 (dd, *J* = 8.4 Hz, *J* = 2.4 Hz, 1H), 8.24 (s, 1H), 7.79 (dd,J= 8.4 Hz, *J* = 1.2 Hz, 1H), 7.62 (d, *J =* 8.4 Hz, 1H), 7.49 (d, *J* = 8.4 Hz, 1H), 7.30 (t, J= 8.0, 1H), 6.42 (d, *J=* 7.6 Hz, 1H), 6.01-5.98 (m, 2H), 5.40 (s, 1H), 5.09-5.06 (m, 1H), 4.75 (dd, *J* = 15.6 Hz, *J* = 7.2 Hz, 1H), 4.63-4.59 (m, 1H), 4.51-4.46 (m, 1H), 4.41-4.35 (m, 1H), 3.90 (d, *J* = 13.6 Hz, 1H), 3.73 (d, *J* = 13.6 Hz, 1H), 2.80-2.65 (m, 3H), 2.44-2.39 (m, 1H), 2.10-1.99 (m, 2H), 1.61-1.59 (m, 2H), 1.30-1.19 (m, 3H) | 554 (M+H) ⁺ |
| 41 | 14 | 40 | 2-(((R)-3-((6-((5-cyanopyridin-2-yl)methoxy)pyridin-2-yl)amino)pyrro lidin-1-yl)methyl)-1-(((S)-oxetan-2-yl)methyl)-1H-benzo[d]imida zole-6-carboxylic acid | ¹H NMR (400 MHz, DMSO-d₆): *δ* 8.98 (d, *J* = 1.6 Hz, 1H), 8.25 (dd, *J* = 8.4 Hz, *J* = 2.0 Hz, 1H), 8.23 (s, 1H), 7.79 (dd, *J* = 8.4Hz, J = 1.2Hz, 1H), 7.62 (d, *J* = 8.4 Hz, 1H), 7.49 (d, *J=* 8.0 Hz, 1H), 7.32 (t, J = 7.6 Hz, 1H), 6.68 (d, *J* =6.4 Hz, 1H), 6.02 (dd, *J* =10.8 Hz, *J* = 8.0 Hz, 2H), 5.41 (s, 2H), 5.06-5.02 (m, 1H), 4.73 (dd, *J* = 15.2 Hz, *J* = 7.2 Hz, 1H), 4.59 (dd, J = 15.2 Hz, J = 2.8 Hz, 1H), 4.49-4.43 (m, 1H), 4.33-4.28 (m, 1H), 3.96 (d, *J* = 13.6 Hz, 1H), 3.82 (d, J = 13.6 Hz, 1H), 3.29-3.25 (m, 1H), 2.68-2.59 (m, 3H), 2.49-2.40 (m, 1H), 2.38-2.33 (m, 2H), 2.03-1.98 (m, 1H), 1.53-1.49 (m, 1H). | 540 (M+H) ⁺ |
| 42 | 29 | 40 | (S)-2-((3-((6-((4-cyano-2-fluorobenzyl)o xy)pyridin-2-yl)amino)azeti din-1-yl)methyl)-1-(oxetan-2-ylmethyl)-1H-benzo[d]imida zole-6-carboxylic acid | ¹H NMR (400 MHz, DMSO-d₆): *δ* 8.24 (s, 1H), 7.85 (d, J =8 Hz, 1H), 7.79 (d, J=8 Hz, 1H), 7.71 (d, J=1.2 Hz, 1H), 7.69-7.62 (m, 2H), 7.33 (t, J =8 Hz, 1H), 7.03 (d, J =6.4 Hz, 1H), 6.02 (d, J =8 Hz, 1H), 5.98 (d, J=7.6 Hz, 1H), 5.38 (s, 2H), 5.07-5.05 (m, 1H), 4.75-4.70 (m, 1H), 4.62-4.58 (m, 1H), 4.48-4.46 (m, 1H), 4.32-4.26 (m, 2H), 3.99 (d, *J* =13.6 Hz, 1H), 3.89 (d, *J* =13.6 Hz, 1H), 3.59 (dt, *J* =6.4 Hz, *J* =20.8 Hz, 2H), 3.01-2.98 (m, 2H), 2.71-2.67 (m, 1H), 2.41-2.36 (m, 1H). | 543 (M+H) ⁺ |
| 43 | 35 | 40 | 2-(((*R*)-3-((4-((4-cyano-2-fluorobenzyl)o xy)pyrimidin-2-yl)amino)pyrro lidin-1-yl)methyl)-1-(((S)-oxetan-2-yl)methyl)-1H-benzo[d]imida zole-6-carboxylic acid | ¹H NMR (400 MHz, CDCl₃): *δ* 8.02-7.98 (m, 3H), 7.77 (d, *J =* 8.4 Hz, 1H), 7.59 (t, *J =* 7.6 Hz, 1H), 7.49 (d, *J* = 7.2 Hz, 1H), 7.41 (d, *J =* 8.8 Hz, 1H), 6.11 (d, *J=* 6.0 Hz, 1H), 5.52 (s, 2H), 5.05-5.03 (m, 2H), 4.67-4.58 (m, 2H), 4.47 (q, *J=* 6.8 Hz, 1H), 4.27-4.10 (m, 3H), 3.10-3.04 (m, 2H), 2.66-2.54 (m, 2H), 2.35-2.21 (m, 3H), 1.84 (brs, 1H) | 558 (M+H) ⁺ |
| 44 | 38 | 40 | 2-(((R)-3-((6-((5-chloropyridin-2-yl)methoxy)py ridin-2-yl)amino)pyrro lidin-1-yl)methyl)-1-(((S)-oxetan-2-yl)methyl)-1H-benzo[d]imida zole-6-carboxylic acid | ¹H NMR (400 MHz, DMSO-d₆): *δ*8.57 (d, *J*= 2.4 Hz, 1H), 8.21 (s, 1H), 7.88 (dd, *J* = 8.4Hz, J = 2.8Hz, 1H), 7.78 (dd, *J* = 8.8Hz, J = 1.6Hz, 1H), 7.59 (d, *J=* 8.4 Hz, 1H), 7.37 (d, *J=* 8.8 Hz, 1H), 7.30 (t, *J=* 8.0 Hz, 1H), 6.67 (d, *J* =6.0 Hz, 1H), 6.02 (d, J=8.0 Hz, 1H), 5.97 (d, J =8.0 Hz, 1H), 5.76 (s, 1H), 5.31 (s,2H), 5.06-5.03 (m, 1H), 4.73 (dd, J = 15.2 Hz, J = 7.2 Hz, 1H), 4.59 (dd, *J* = 14.8 Hz, J = 2.4 Hz, 1H), 4.46-4.43 (m, 1H), 4.32-4.30 (m, 1H), 4.08 (m ,1H), 3.99 (d, J =13.2 Hz, 1H), 3.82 (d, *J* =13.2 Hz, 1H), 2.73-2.62 (m, 3H), 2.41-2.37 (m, 2H), 2.07 (m, 1H), 1.56-1.54 (m, 1H) | 549 (M+H) ⁺ |

Structures and compound names of the compounds of Examples 1 to 44 prepared in Preparation Examples A, B, and C are shown in Table 5 below.

**Table 5**

| Example No. | Structure | Compound Name |
|---|---|---|
| 1 | 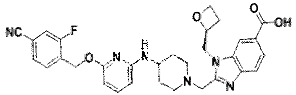 | (*S*)-2-((4-((6-((4-cyano-2-fluorobenzyl)oxy)pyridin- 2-yl)amino)piperidin-1-yl)methyl)-1-(oxetan-2-ylmethyl)- 1H-benzo[d]imidazole-6-carboxylic acid |
| 2 | 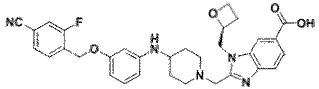 | (S)-2-((4-((3 -((4-cyano-2-fluorobenzyl)oxy)phenyl)amino)piperidin-1-yl)methyl)-1-(oxetan-2-ylmethyl)-1H-benzo[d]imidazole-6-carboxylic acid |
| 3 | 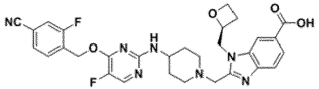 | (*S*)-2-((4-((4-((4-cyano-2-fluorobenzyl)oxy)-5-fluoropyrimidin-2-yl)amino)piperidin-1-yl)methyl)-1-(oxetan-2-ylmethyl)-1H-benzo[d]imidazole-6-carboxylic acid |
| 4 | 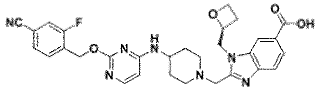 | (*S*)-2-((4-((2-((4-cyano-2-fluorobenzyl)oxy)pyrimidin-4-yl)amino)piperidin-1-yl)methyl)-1-(oxetan-2-ylmethyl)- 1H-benzo[d]imidazole-6-carboxylic acid |
| 5 | 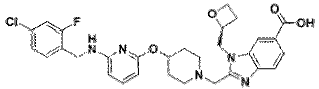 | (*S*)-2-((4-((6-((4-chloro-2-fluorobenzyl)amino)pyridin-2-yl)oxy)piperidin-1-yl)methyl)-1 -(oxetan-2-ylmethyl)- 1H-benzo[d]imidazole-6-carboxylic acid |
| 6 | 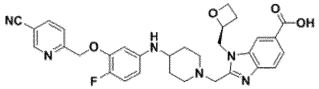 | (*S*)-2-((4-((3-((5-cyanopyridin-2-yl)methoxy)-4-fluorophenyl)amino)piperidin-1-yl)methyl)-1 -(oxetan-2-ylmethyl)- 1H-benzo[d]imidazole-6-carboxylic acid |
| 7 | 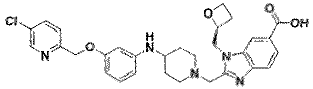 | (*S*)-2-((4-((3-((5-chloropyridin-2-yl)methoxy)phenyl)amino)piperi din- 1 - yl)methyl)-1-(oxetan-2-ylmethyl)-1H-benzo[d]imidazole-6-carboxylic acid |
| 8 | 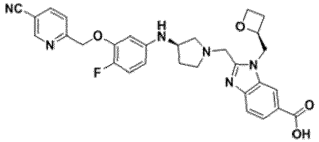 | 2-(((R)-3-((3-((5-cyanopyridin-2-yl)methoxy)-4-fluorophenyl)amino)pyrrolidin- 1 -yl)methyl)-1-(((S)-oxetan-2-yl)methyl)-1H-benzo[d]imidazole-6-carboxylic acid |
| 9 | 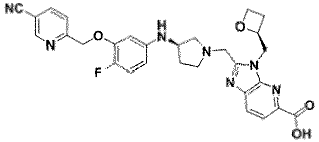 | 2-(((R)-3-((3-((5-cyanopyridin-2-yl)methoxy)-4-fluorophenyl)amino)pyrrolidin-1-yl)methyl)-3-(((S)-oxetan-2-yl)methyl)-3H-imidazo[4,5-b]pyridine-5-carboxylic acid |
| 10 | 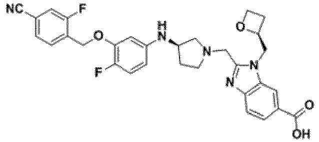 | 2-(((R)-3-((3-((4-cyano-2-fluorobenzyl)oxy)-4-fluorophenyl)amino)pyrrolidin-1-yl)methyl)-1-(((S)-oxetan-2-yl)methyl)-1H-benzo[d]imidazole-6-carboxylic acid |
| 11 | 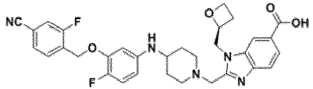 | (S)-2-((4-((3-((4-cyano-2-fluorobenzyl)oxy)-4-fluorophenyl)amino)piperidin-1-yl)methyl)-1-(oxetan-2-ylmethyl)-1H-benzo[d]imidazole-6-carboxylic acid |
| 12 | 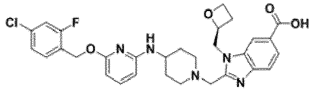 | (*S*)-2-((4-((6-((4-chloro-2-fluorobenzyl)oxy)pyridin- 2-yl)amino)piperidin-1-yl)methyl)-1-(oxetan-2-ylmethyl)- 1H-benzo[d]imidazole-6-carboxylic acid |
| 13 | 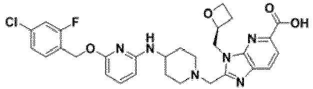 | (*S*)-2-((4-((6-((4-chloro-2-fluorobenzyl)oxy)pyri din - 2-yl)amino)piperidin-1-yl)methyl)-3 -oxetan-2-ylmethyl)-3H-imidazo[4,5-b]pyridine-5-carboxylic acid |
| 14 | 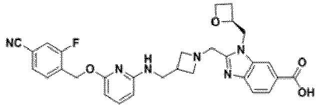 | (*S*)-2-((3-(((6-((4-cyano-2-fluorobenzyl)oxy)pyridin-2-yl)amino)methyl)azetidin-1-yl)methyl)-1-(oxetan-2-ylmethyl)-1*H*-benzo[d]imidazole-6-carboxylic acid |
| 15 | 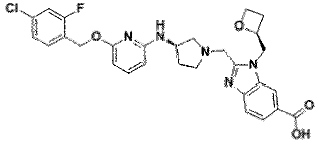 | 2-(((R)-3-((6-((4-chloro-2-fluorobenzyl)oxy)pyridin- 2-yl)amino)pyrrolidin-1-yl)methyl)-1-(((S)-oxetan-2-yl)methyl)-1H-benzo[d]imidazole-6-carboxylic acid |
| 16 | 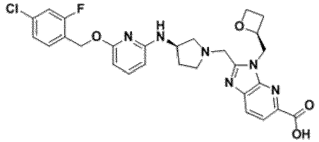 | 2-(((R)-3-((6-((4-chloro-2-fluorobenzyl)oxy)pyri din - 2-yl)amino)pyrrolidin-1-yl)methyl)-3-(((*S*)-oxetan-2-yl)methyl)-3H-imidazo[4,5-b]pyridine-5-carboxylic acid |
| 17 | 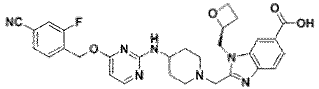 | (*S*)-2-((4-((4-((4-cyano-2-fluorobenzyl)oxy)pyrimidin-2-yl)amino)piperidin-1-yl)methyl)-1 -(oxetan-2-ylmethyl)- 1H-benzo[d]imidazole-6-carboxylic acid |
| 18 | 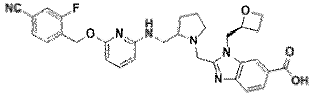 | 2-((2-(((6-((4-cyano-2-fluorobenzyl)oxy)pyridin- 2-yl)amino)methyl)pyrroli din- 1 -yl)m ethyl)-1 - (((S)-oxetan-2-yl)methyl)-1H-benzo[*d*]imidazole-6-carboxylic acid |
| 19 | 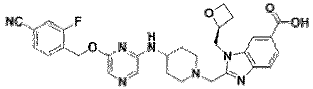 | (*S*)-2-((4-((6-((4-cyano-2-fluorobenzyl)oxy)pyrazin - 2-yl)amino)piperidin-1-yl)methyl)-1 -(oxetan-2-ylmethyl)- 1H-benzo[d]imidazole-6-carboxylic acid |
| 20 | 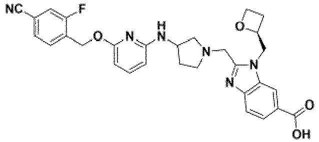 | 2-((3-((6-((4-cyano-2-fluorobenzyl)oxy)pyridin-2-yl)amino)pyrrolidin-1-yl)methyl)-1-(((S)-oxetan-2-yl)methyl)-1H-benzo[*d*]imidazole-6-carboxylic acid |
| 21 | 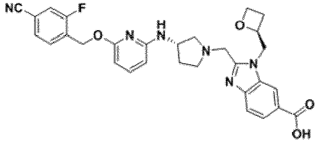 | 2-(((*S*)-3 -((6-((4-cyano-2-fluorobenzyl)oxy)pyri din - 2-yl)amino)pyrrolidin-1-yl)methyl)-1-(((S)-oxetan-2-yl)methyl)-1*H*-benzo[*d*]imidazole-6-carboxylic acid |
| 22 | 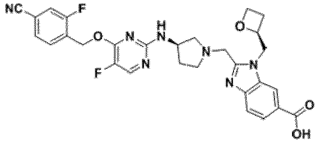 | 2-(((*R*)-3-((4-((4-cyano-2-fluorobenzyl)oxy-5-fluoropyrimidin-2-yl)amino)pyrrolidin-1-yl)methyl)-1-(((*S*)-oxetan-2-yl)methyl)-1*H-*benzo[d]imidazole-6-carboxylic acid |
| 23 | 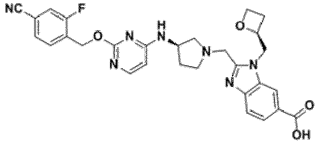 | 2-(((R)-3-((2-((4-cyano-2-fluorobenzyl)oxy)pyrimidin-4-yl)amino)pyrrolidin-1-yl)methyl)-1-(((S)-oxetan-2-yl)methyl)-1H-benzo[d]imidazole-6-carboxylic acid |
| 24 | 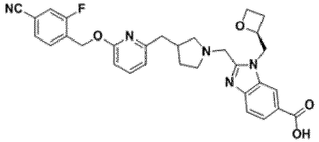 | 2-((3 -((6-((4-cyano-2-fluorobenzyl )oxy)pyri din- 2-yl)methyl)pyrrolidin-1-yl)methyl)-1-(((.S)-oxetan-2-yl)methyl)-1H-benzo[d]imidazole-6-carboxylic acid |
| 25 | 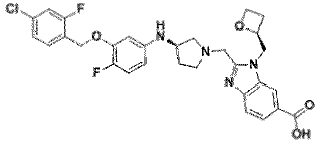 | 2-(((R)-3-((3-((4-chloro-2-fluorobenzyl)oxy)-4-fluorophenyl)amino)pyrrolidin-1-yl)m ethyl)-1-(((S)-oxetan-2-yl)m ethyl)-1H-benzo[d]imidazole-6-carboxylic acid |
| 26 | 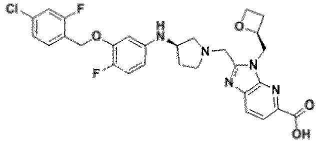 | 2-(((R)-3-((3-((4-chloro-2-fluorobenzyl)oxy)-4-fluorophenyl)amino)pyrrolidin-1-yl)methyl)-3 - (((S)-oxetan-2-yl)methyl)-3H-imidazo[4,5-b]pyridine-5-carboxylic acid |
| 27 | 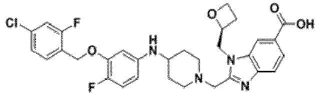 | (S)-2-((4-((3-((4-chloro-2-fluorobenzyl)oxy)-4-fluorophenyl)amino)piperidin-1-yl)methyl)- 1-(oxetan-2-ylmethyl)-1H-benzo[d]imidazole-6-carboxylic acid |
| 28 | 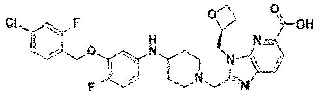 | (S)-2-((4-((3-((4-chloro-2-fluorobenzyl)oxy)-4-fluorophenyl)amino)piperidin-1-yl)methyl)-3-(oxetan-2-ylmethyl)-3H-imidazo[4,5-b]pyridine-5-carboxylic acid |
| 29 | 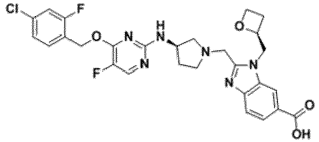 | 2-(((R)-3-((4-((4-chloro-2-fluorobenzyl)oxy)-5 - fluoropyrimidin-2-yl)amino)pyrrolidin-1-yl)methyl)-1-(((S)-oxetan-2-yl)methyl)-1H-benzo[d]imidazole-6-carboxylic acid |
| 30 | 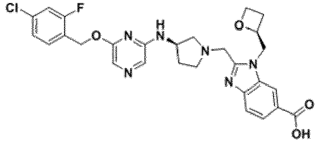 | 2-(((R)-3-((6-((4-chloro-2-fluorobenzyl )oxy)pyrazin -2-yl)amino)pyrrolidin-1-yl)methyl)-1-(((S)-oxetan-2-yl)methyl)-1H-benzo[d]imidazole-6-carboxylic acid |
| 31 | 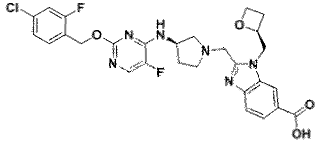 | 2-(((R)-3-((2-((4-chloro-2-fluorobenzyl)oxy)-5-fluoropyrimidin-4-yl)amino)pyrrolidin-1-yl)methyl)-1-(((S)-oxetan-2-yl)methyl)-1H-benzo[d]imidazole-6-carboxylic acid |
| 32 | 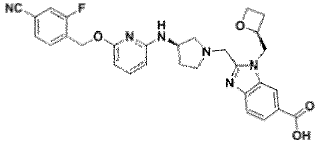 | 2-(((R)-3-((6-((4-cyano-2-fluorobenzyl)oxy)pyri din-2-yl)amino)pyrrolidin-1-yl)methyl)-1-(((S)-oxetan-2-yl)methyl)-1H-benzo[d]imidazole-6-carboxylicacid |
| 33 | 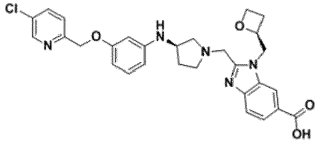 | 2-(((R)-3-((3-((5-chloropyridin-2-yl)methoxy)phenyl)amino)pyrrolidin-1-yl)methyl)-1-(((S)-oxetan-2-yl)methyl)-1H-benzo[d]imidazole-6-carboxylic acid |
| 34 | 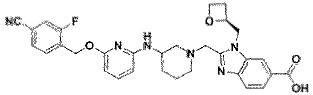 | 2-((3-((6-((4-cyano-2-fluorobenzyl)oxy)pyri din-2-yl)amino)piperidin-1-yl)methyl)-1-(((S)-oxetan-2-yl)methyl)-1H-benzo[d]imidazole-6-carboxylicacid |
| 35 | 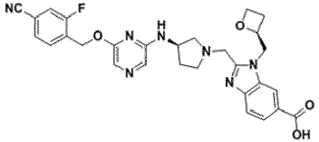 | 2-(((R)-3-((6-((4-cyano-2-fluorobenzyl)oxy)pyrazin-2-yl)amino)pyrrolidin-1-yl)methyl)-1-(((S)-oxetan-2-yl)methyl)-1H-benzo[*d*]imidazole-6-carboxylicacid |
| 36 | 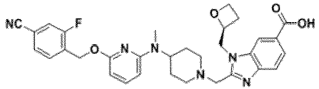 | (*S*)-2-((4-((6-((4-cyano-2-fluorobenzyl)oxy)pyridin-2-yl)(methyl)amino)piperidin-1-yl)methyl)-1-(oxetan-2-ylmethyl)-1H-benzo[d]imidazole-6-carboxylic acid |
| 37 | 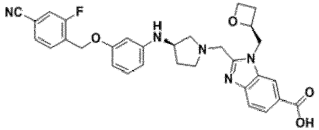 | 2-(((R)-3-((3-((4-cyano-2-fluorob enzyl)oxy)phenyl)amino)pyrroli din- 1 - yl)methyl)-1-(((*S*)-oxetan-2-yl)methyl)-1H-benzo[d]imidazole-6-carboxylic acid |
| 38 | 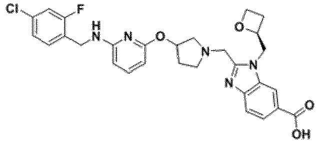 | 2-((3 -((6-((4-chloro-2-fluorobenzyl)amino)pyridin-2-yl)oxy)pyrrolidin-1-yl)methyl)-1-(((S)-oxetan-2-yl)methyl)-1H-benzo[d]imidazole-6-carboxylic acid |
| 39 | 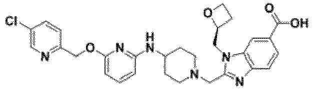 | (*S*)-2-((4-((6-((5-chioropyridin-2-yl)methoxy)pyridin-2-yl)amino)piperidin-1-yl)methyl)-1-(oxetan-2-ylmethyl)-1H-benzo[d]imidazole-6-carboxylic acid |
| 40 | 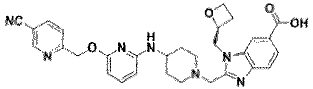 | (S)-2-((4-((6-((5-cyanopyridin-2-yl)methoxy)pyridin-2-yl)amino)piperidin-1-yl)methyl)-1-(oxetan-2-ylmethyl)-1H-benzo[d]imidazole-6-carboxylic acid |
| 41 | 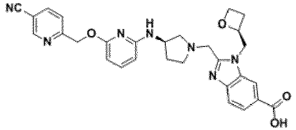 | 2-(((R)-3-((6-((5-cyanopyridin-2-yl)methoxy)pyndin-2-yl)amino)pyrrolidin-1-yl)methyl)-1-(((*S*)-oxetan-2-yl)methyl)-1H-benzo[d]imidazole-6-carboxylic acid |
| 42 | 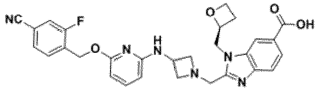 | (S)-2-((3 -((6-((4-cyano-2-fluorobenzyl)oxy)pyridin-2-yl)amino)azetidin-1 -yl)methyl)-1 -(oxetan-2-ylmethyl)- 1H-benzo[d]imidazole-6-carboxylic acid |
| 43 | 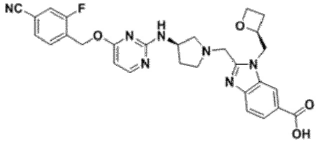 | 2-(((R)-3-((4-((4-cyano-2-fluorobenzyl)oxy)pyrimidin-2-yl)amino)pyrrolidin-1-yl)methyl)-1-(((S)-oxetan-2-yl)methyl)-1*H*-benzo[*d*]imidazole-6-carboxylic acid |
| 44 | 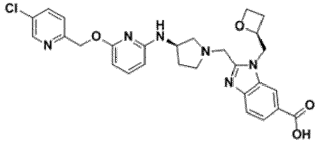 | 2-(((R)-3-((6-((5-chloropyridin-2-yl)methoxy)pyridin-2-yl)amino)pyrrolidin-1-yl)methyl)-1-(((*S*)-oxetan-2-yl)methyl)-1*H-*benzo[d]imidazole-6-carboxylic acid |

### Experimental Examples

### 1. Experimental Example 1: cAMP assay

The cAMP assay test was performed according to a method optimized based on a protocol provided by cAMP assay kit manufacturer (CISBIO). GLP-1 receptor CHO-K1 cells were dispensed into 96-well plates for cAMP measurement (low volume, white) at 6 × 10³ cells/well/5 µL. As a control substance, 5µL of Exendin-4 at the concentration of 0, 1, 10, 100, 1000, and 10000 pM was treated to each of the wells of one of the plates. 5µL of the compounds according to the Examples 1, 2, 5, 8, 12, 13, 15, 16, 19, 20, 23, 29, 32, 37, 39 and 43 at the concentration of 0, 1, 10, 100, 1000, 10000 nM was treated to each of the wells of the other plates, respectively. The cells were incubated at room temperature for 7 minutes. A cAMP-d₂ conjugate reagent was prepared by mixing a cAMP conjugate with an elution buffer at a ratio of 1 : 4. An anti-cAMP cryptate conjugate reagent was prepared by mixing a cGMP conjugate with an elution buffer at a ratio of 1 : 4. Then, 5 µL of the cAMP-d₂ conjugate reagent was added to each of the wells. Subsequently, 5 µL of the anti-cAMP cryptate conjugate reagent was added to each of the wells. After incubation of the cells at room temperature for 1 hour, HTRF signals at wavelengths of 665 nm and 620 nm of the culture were measured using a FlexStaton 3 (Molecular Devices) instrument. The ratio of 665/620 was calculated from the measured values at 665 nm and 620 nm with regard to Exendin-4 and the compounds of the Examples, respectively. By converting the ratio with regard to Exendin-4 to 100%, Emax values of the compounds of the Examples were calculated as the cAMP stimulation ratio of the compounds. The results are shown in Table 5 below. In the table, ++ means that EC₅₀ is smaller than 100 nM, and + means that EC₅₀ is 100~200 nM.

| Example No. | EC₅₀ (nM) | Eₘₐₓ(%) |
|---|---|---|
| 1 | ++ | 96.26 |
| 2 | + | 101.6 |
| 5 | + | 106.73 |
| 8 | ++ | 99.59 |
| 12 | ++ | 102.62 |
| 13 | ++ | 94.48 |
| 15 | ++ | 99.63 |
| 16 | ++ | 94.96 |
| 19 | + | 97.05 |
| 20 | ++ | 96.3 |
| 23 | + | 99.8 |
| 29 | ++ | 99.22 |
| 32 | ++ | 96.94 |
| 37 | ++ | 126.93 |
| 39 | + | 96.61 |
| 43 | ++ | 110.44 |

## Claims

1. A compound represented by the following Chemical Formula 1, an optical isomer of the compound, or a pharmaceutically acceptable salt of the compound or the optical isomer: 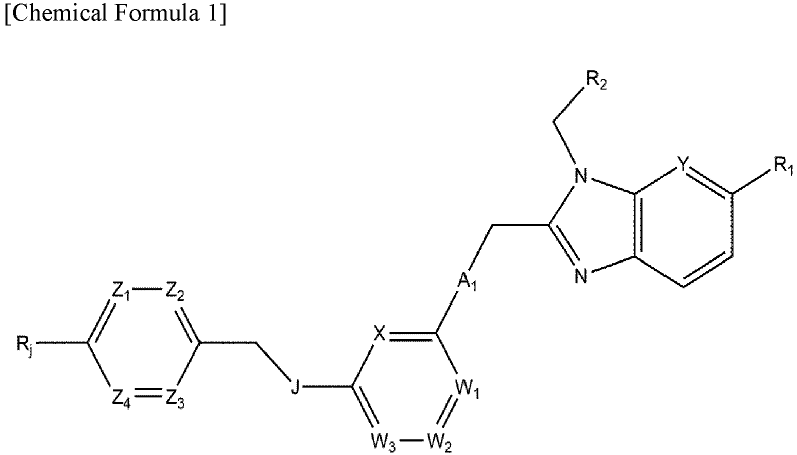
wherein R₁ is -C(=O)Rₐ;
Rₐ is -OH or -O-(C₁-C₄ alkyl);
Y is -CH- or -N-;
R₂ is one selected from the group consisting of substituted or unsubstituted C₆ to C₁₂ aryl, substituted or unsubstituted C₅ to C₁₂ heteroaryl, substituted or unsubstituted C₃ to C₈ heterocycloalkyl, and substituted or unsubstituted C₃ to C₈ cycloalkyl, where the substituted aryl, heteroaryl, heterocycloalkyl, and cycloalkyl include at least one substitution with -OH, -(C₁-C₄ alkyl), halogen, or -CN;
A₁ is 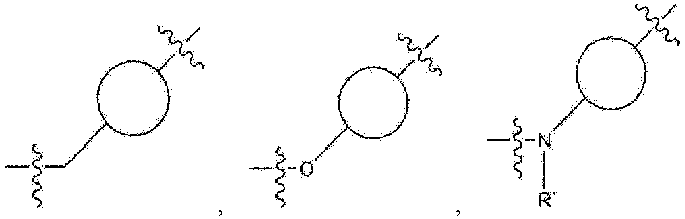 or 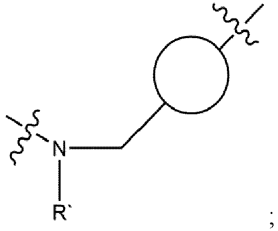 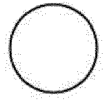 is substituted or unsubstituted C₃ to C₈ heterocycloalkyl containing at least one nitrogen, substituted or unsubstituted C₃ to C₁₂ spiroheterocycloalkyl containing at least one nitrogen, or substituted or unsubstituted C₃ to C₁₂ bridged heterobicycloalkyl containing at least one nitrogen, where the substituted heterocycloalkyl, spiroheterocycloalkyl, and heterobicycloalkyl include at least one substitution with -OH, -(C₁-C₄ alkyl), halogen, or -CN;
R' is hydrogen or -(C₁-C₄ alkyl);
X is -CR_{b}- or -N-;
R_{b} is one selected from the group consisting of -H, halogen, -CN, -OH, -O-(C₁-C₄ alkyl), -NH₂, -NO₂, and -C₁-C₄ haloalkyl;
W₁ is -CR_{c}- or -N-, where R_{c} is one selected from the group consisting of -H, halogen, -CN, -OH, -O-(C₁-C₄ alkyl), -NH₂, -NO₂, and -C₁-C₄ haloalkyl;
W₂ is -CR_{d}- or -N-, where R_{d} is one selected from the group consisting of -H, halogen, -CN, -OH, -O-(C₁-C₄ alkyl), -NH₂, -NO₂, and -C₁-C₄ haloalkyl;
W₃ is -CRₑ- or -N-, where Rₑ is one selected from the group consisting of -H, halogen, -CN, -OH, -O-(C₁-C₄ alkyl), -NH₂, -NO₂, and -C₁-C₄ haloalkyl;
J is -O- or -NR"-;
R" is hydrogen or -(C₁-C₄ alkyl);
Z₁ is -CR_{f}- or -N-, where R_{f} is one selected from the group consisting of -H, halogen, -CN, -OH, -O-(C₁-C₄ alkyl), -NH₂, -NO₂, and -C₁-C₄ haloalkyl;
Z₂ is -CR_{g}- or -N-, where R_{g} is one selected from the group consisting of -H, halogen, -CN, -OH, -O-(C₁-C₄ alkyl), -NH₂, -NO₂, and -C₁-C₄ haloalkyl;
Z₃ is -CRₕ- or -N-, where Rₕ is one selected from the group consisting of -H, halogen, -CN, -OH, -O-(C₁-C₄ alkyl), -NH₂, -NO₂, and -C₁-C₄ haloalkyl;
Z₄ is -CRi- or -N-, where Rᵢ is one selected from the group consisting of -H, halogen, -CN, -OH, -O-(C₁-C₄ alkyl), -NH₂, -NO₂, and -C₁-C₄ haloalkyl; and
Rⱼ is one selected from the group consisting of -H, halogen, -CN, -OH, -O-(C₁-C₄ alkyl), -NH₂, -NO₂, and -C₁-C₄ haloalkyl.

2. The compound, the optical isomer, or the pharmaceutically acceptable salt of claim 1,
wherein one of Z₁ to Z₄ is -N-.

3. The compound, the optical isomer, or the pharmaceutically acceptable salt of claim 1,
wherein Z₁ is -CR_{f}-, Z₂ is -CR_{g}-, Z₃ is -CRₕ-, and Z₄ is -CRᵢ-.

4. The compound, the optical isomer, or the pharmaceutically acceptable salt of claim 1,
wherein Rⱼ is halogen or -CN.

5. The compound, the optical isomer, or the pharmaceutically acceptable salt of claim 4,
wherein R_{g} is one selected from the group consisting of -H, halogen and -CN.

6. The compound, the optical isomer, or the pharmaceutically acceptable salt of claim 1,
wherein J is -O-.

7. The compound, the optical isomer, or the pharmaceutically acceptable salt of claim 1, wherein A₁ is

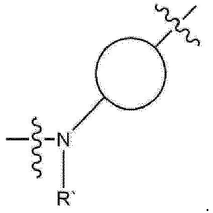

8. The compound, the optical isomer, or the pharmaceutically acceptable salt of claim 7, wherein 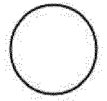 is substituted or unsubstituted C₃ to C₈ heterocycloalkyl containing at least one nitrogen, where the substituted heterocycloalkyl includes at least one substitution with -OH, -(C₁-C₄ alkyl), halogen, or -CN.

9. The compound, the optical isomer, or the pharmaceutically acceptable salt of claim 7, wherein 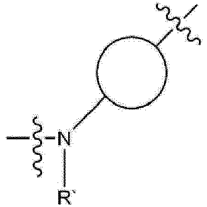 is one selected from the group consisting of substituted or unsubstituted 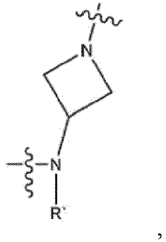 substituted or unsubstituted 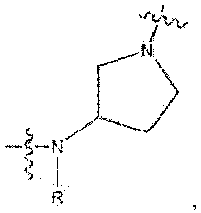 and substituted or unsubstituted 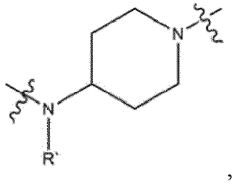 where the substituted 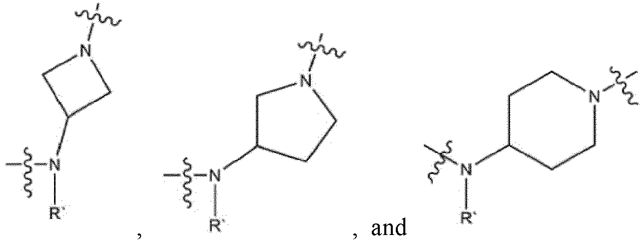 include at least one substitution with -OH, -(C₁-C₄ alkyl), halogen, or -CN.

10. The compound, the optical isomer, or the pharmaceutically acceptable salt of claim 1,
wherein R₂ is substituted or unsubstituted C₃ to C₅ heterocycloalkyl or substituted or unsubstituted C₃ to C₅ cycloalkyl, where the substituted heterocycloalkyl and cycloalkyl include at least one substitution with -OH, -(C₁-C₄ alkyl), halogen, or -CN.

11. The compound, the optical isomer, or the pharmaceutically acceptable salt of claim 10, wherein R₂ is substituted or unsubstituted 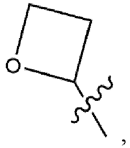 where the substituted 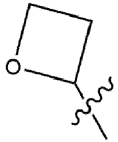 includes at least one substitution with -OH, -(C₁-C₄ alkyl), halogen, or -CN.

12. The compound, the optical isomer, or the pharmaceutically acceptable salt of claim 1,
wherein the compound represented by the Chemical Formula 1 is one compound selected from the group consisting of the following compounds:
(S)-2-((4-((6-((4-cyano-2-fluorobenzyl)oxy)pyridin-2-yl)amino)piperidin-1-yl)methyl)-1-(oxetan-2-ylmethyl)-1H-benzo[d]imidazole-6-carboxylic acid;
(S)-2-((4-((3-((4-cyano-2-fluorobenzyl)oxy)phenyl)amino)piperidin-1-yl)methyl)-1-(oxetan-2-ylmethyl)-1H-benzo[d]imidazole-6-carboxylic acid;
(S)-2-((4-((4-((4-cyano-2-fluorobenzyl)oxy)-5-fluoropyrimidin-2-yl)amino)piperidin-1-yl)methyl)-1-(oxetan-2-ylmethyl)-1H-benzo[d]imidazole-6-carboxylic acid;
(S)-2-((4-((2-((4-cyano-2-fluorobenzyl)oxy)pyrimidin-4-yl)amino)piperidin-1-yl)methyl)-1-(oxetan-2-ylmethyl)-1H-benzo[d]imidazole-6-carboxylic acid;
(S)-2-((4-((6-((4-chloro-2-fluorobenzyl)amino)pyridin-2-yl)oxy)piperidin-1-yl)methyl)-1-(oxetan-2-ylmethyl)-1H-benzo[d]imidazole-6-carboxylic acid;
(S)-2-((4-((3-((5-cyanopyridin-2-yl)methoxy)-4-fluorophenyl)amino)piperidin-1-yl)methyl)-1-(oxetan-2-ylmethyl)-1H-benzo[d]imidazole-6-carboxylic acid;
(S)-2-((4-((3-((5-chloropyridin-2-yl)methoxy)phenyl)amino)piperidin-1-yl)methyl)-1-(oxetan-2-ylmethyl)-1H-benzo[d]imidazole-6-carboxylic acid;
2-(((R)-3-((3-((5-cyanopyridin-2-yl)methoxy)-4-fluorophenyl)amino)pyrrolidin-1-yl)methyl)-1-(((S)-oxetan-2-yl)methyl)-1H-benzo[d]imidazole-6-carboxylic acid;
2-(((R)-3-((3-((5-cyanopyridin-2-yl)methoxy)-4-fluorophenyl)amino)pyrrolidin-1-yl)methyl)-3-(((S)-oxetan-2-yl)methyl)-3H-imidazo[4,5-b]pyridine-5-carboxylic acid;
2-(((R)-3-((3-((4-cyano-2-fluorobenzyl)oxy)-4-fluorophenyl)amino)pyrrolidin-1-yl)methyl)-1-(((S)-oxetan-2-yl)methyl)-1H-benzo[d]imidazole-6-carboxylic acid;
(S)-2-((4-((3-((4-cyano-2-fluorobenzyl)oxy)-4-fluorophenyl)amino)piperidin-1-yl)methyl)-1-(oxetan-2-ylmethyl)-1H-benzo[d]imidazole-6-carboxylic acid;
(S)-2-((4-((6-((4-chloro-2-fluorobenzyl)oxy)pyridin-2-yl)amino)piperidin-1-yl)methyl)-1-(oxetan-2-ylmethyl)-1H-benzo[d]imidazole-6-carboxylic acid;
(S)-2-((4-((6-((4-chloro-2-fluorobenzyl)oxy)pyridin-2-yl)amino)piperidin-1-yl)methyl)-3-oxetan-2-ylmethyl)-3H-imidazo[4,5-b]pyridine-5-carboxylic acid;
(S)-2-((3-(((6-((4-cyano-2-fluorobenzyl)oxy)pyridin-2-yl)amino)methyl)azetidin-1-yl)methyl)-1-(oxetan-2-ylmethyl)-1H-benzo[d]imidazole-6-carboxylic acid;
2-(((R)-3-((6-((4-chloro-2-fluorobenzyl)oxy)pyridin-2-yl)amino)pyrrolidin-1-yl)methyl)-1-(((S)-oxetan-2-yl)methyl)-1H-benzo[d]imidazole-6-carboxylic acid;
2-(((R)-3-((6-((4-chloro-2-fluorobenzyl)oxy)pyridin-2-yl)amino)pyrrolidin-1-yl)methyl)-3-(((S)-oxetan-2-yl)methyl)-3H-imidazo[4,5-b]pyridine-5-carboxylic acid;
(S)-2-((4-((4-((4-cyano-2-fluorobenzyl)oxy)pyrimidin-2-yl)amino)piperidin-1-yl)methyl)-1-(oxetan-2-ylmethyl)-1H-benzo[d]imidazole-6-carboxylic acid;
2-((2-(((6-((4-cyano-2-fluorobenzyl)oxy)pyridin-2-yl)amino)methyl)pyrrolidin-1-yl)methyl)-1-(((S)-oxetan-2-yl)methyl)-1H-benzo[d]imidazole-6-carboxylic acid;
(S)-2-((4-((6-((4-cyano-2-fluorobenzyl)oxy)pyrazin-2-yl)amino)piperidin-1-yl)methyl)-1-(oxetan-2-ylmethyl)-1H-benzo[d]imidazole-6-carboxylic acid;
2-((3-((6-((4-cyano-2-fluorobenzyl)oxy)pyridin-2-yl)amino)pyrrolidin-1-yl)methyl)-1-(((S)-oxetan-2-yl)methyl)-1H-benzo[d]imidazole-6-carboxylic acid;
2-(((S)-3-((6-((4-cyano-2-fluorobenzyl)oxy)pyridin-2-yl)amino)pyrrolidin-1-yl)methyl)-1-(((S)-oxetan-2-yl)methyl)-1H-benzo[d]imidazole-6-carboxylic acid;
2-(((R)-3-((4-((4-cyano-2-fluorobenzyl)oxy-5-fluoropyrimidin-2-yl)amino)pyrrolidin-1-yl)methyl)-1-(((S)-oxetan-2-yl)methyl)-1H-benzo[d]imidazole-6-carboxylic acid;
2-(((R)-3-((2-((4-cyano-2-fluorobenzyl)oxy)pyrimidin-4-yl)amino)pyrrolidin-1-yl)methyl)-1-(((S)-oxetan-2-yl)methyl)-1H-benzo[d]imidazole-6-carboxylic acid;
2-((3-((6-((4-cyano-2-fluorobenzyl)oxy)pyridin-2-yl)methyl)pyrrolidin-1-yl)methyl)-1-(((S)-oxetan-2-yl)methyl)-1H-benzo[d]imidazole-6-carboxylic acid;
2-(((R)-3-((3-((4-chloro-2-fluorobenzyl)oxy)-4-fluorophenyl)amino)pyrrolidin-1-yl)methyl)-1-(((S)-oxetan-2-yl)methyl)-1H-benzo[d]imidazole-6-carboxylic acid;
2-(((R)-3-((3-((4-chloro-2-fluorobenzyl)oxy)-4-fluorophenyl)amino)pyrrolidin-1-yl)methyl)-3-(((S)-oxetan-2-yl)methyl)-3H-imidazo[4,5-b]pyridine-5-carboxylic acid;
(S)-2-((4-((3-((4-chloro-2-fluorobenzyl)oxy)-4-fluorophenyl)amino)piperidin-1-yl)methyl)-1-(oxetan-2-ylmethyl)-1H-benzo[d]imidazole-6-carboxylic acid;
(S)-2-((4-((3-((4-chloro-2-fluorobenzyl)oxy)-4-fluorophenyl)amino)piperidin-1-yl)methyl)-3-(oxetan-2-ylmethyl)-3H-imidazo[4,5-b]pyridine-5-carboxylic acid;
2-(((R)-3-((4-((4-chloro-2-fluorobenzyl)oxy-5-fluoropyrimidin-2-yl)amino)pyrrolidin-1-yl)methyl)-1-(((S)-oxetan-2-yl)methyl)-1H-benzo[d]imidazole-6-carboxylic acid;
2-(((R)-3-((6-((4-chloro-2-fluorobenzyl)oxy)pyrazin-2-yl)amino)pyrrolidin-1-yl)methyl)-1-(((S)-oxetan-2-yl)methyl)-1H-benzo[d]imidazole-6-carboxylic acid;
2-(((R)-3-((2-((4-chloro-2-fluorobenzyl)oxy-5-fluoropyrimidin-4-yl)amino)pyrrolidin-1-yl)methyl)-1-(((S)-oxetan-2-yl)methyl)-1H-benzo[d]imidazole-6-carboxylic acid;
2-(((R)-3-((6-((4-cyano-2-fluorobenzyl)oxy)pyridin-2-yl)amino)pyrrolidin-1-yl)methyl)-1-(((S)-oxetan-2-yl)methyl)-1H-benzo[d]imidazole-6-carboxylic acid;
2-(((R)-3-((3-((5-chloropyridin-2-yl)methoxy)phenyl)amino)pyrrolidin-1-yl)methyl)-1-(((S)-oxetan-2-yl)methyl)-1H-benzo[d]imidazole-6-carboxylic acid;
2-((3-((6-((4-cyano-2-fluorobenzyl)oxy)pyridin-2-yl)amino)piperidin-1-yl)methyl)-1-(((S)-oxetan-2-yl)methyl)-1H-benzo[d]imidazole-6-carboxylic acid;
2-(((R)-3-((6-((4-cyano-2-fluorobenzyl)oxy)pyrazin-2-yl)amino)pyrrolidin-1-yl)methyl)-1-(((S)-oxetan-2-yl)methyl)-1H-benzo[d]imidazole-6-carboxylic acid;
(S)-2-((4-((6-((4-cyano-2-fluorobenzyl)oxy)pyridin-2-yl)(methyl)amino)piperidin-1-yl)methyl)-1-(oxetan-2-ylmethyl)-1H-benzo[d]imidazole-6-carboxylic acid;
2-(((R)-3-((3-((4-cyano-2-fluorobenzyl)oxy)phenyl)amino)pyrrolidin-1-yl)methyl)-1-(((S)-oxetan-2-yl)methyl)-1H-benzo[d]imidazole-6-carboxylic acid;
2-((3-((6-((4-chloro-2-fluorobenzyl)amino)pyridin-2-yl)oxy)pyrrolidin-1-yl)methyl)-1-(((S)-oxetan-2-yl)methyl)-1H-benzo[d]imidazole-6-carboxylic acid;
(S)-2-((4-((6-((5-chloropyridin-2-yl)methoxy)pyridin-2-yl)amino)piperidin-1-yl)methyl)-1-(oxetan-2-ylmethyl)-1H-benzo[d]imidazole-6-carboxylic acid;
(S)-2-((4-((6-((5-cyanopyridin-2-yl)methoxy)pyridin-2-yl)amino)piperidin-1-yl)methyl)-1-(oxetan-2-ylmethyl)-1H-benzo[d]imidazole-6-carboxylic acid;
2-(((R)-3-((6-((5-cyanopyridin-2-yl)methoxy)pyridin-2-yl)amino)pyrrolidin-1-yl)methyl)-1-(((S)-oxetan-2-yl)methyl)-1H-benzo[d]imidazole-6-carboxylic acid;
(S)-2-((3-((6-((4-cyano-2-fluorobenzyl)oxy)pyridin-2-yl)amino)azetidin-1-yl)methyl)-1-(oxetan-2-ylmethyl)-1H-benzo[d]imidazole-6-carboxylic acid;
2-(((R)-3-((4-((4-cyano-2-fluorobenzyl)oxy)pyrimidin-2-yl)amino)pyrrolidin-1-yl)methyl)-1-(((S)-oxetan-2-yl)methyl)-1H-benzo[d]imidazole-6-carboxylic acid; and
2-(((R)-3-((6-((5-chloropyridin-2-yl)methoxy)pyridin-2-yl)amino)pyrrolidin-1-yl)methyl)-1-(((S)-oxetan-2-yl)methyl)-1H-benzo[d]imidazole-6-carboxylic acid.

13. A pharmaceutical composition comprising the compound, the optical isomer, or the pharmaceutically acceptable salt according to any one of claims 1 to 12.

14. A pharmaceutical composition comprising the compound, the optical isomer, or the pharmaceutically acceptable salt according to any one of claims 1 to 12, and a pharmaceutically acceptable carrier.

15. A compound, the optical isomer, or the pharmaceutically acceptable salt according to any one of claims 1 to 12 for use in treatment and/or prevention of metabolic diseases.

16. A method for preventing or treating metabolic diseases, comprising administering to a subject in need the compound, the optical isomer, or the pharmaceutically acceptable salt according to any of claims 1 to 12.

17. Use of the compound, the optical isomer, or the pharmaceutically acceptable salt according to any one of claims 1 to 12 for prevention or treatment of metabolic diseases.

18. Use of the compound, the optical isomer, or the pharmaceutically acceptable salt according to any one of claims 1 to 12 for preparation of a medicament to prevent or treat metabolic diseases.

19. A pharmaceutical composition for preventing or treating a metabolic disease, the composition comprising the compound, the optical isomer, or the pharmaceutically acceptable salt according to any one of claims 1 to 12.

20. A GLP-1R agonist comprising the compound, the optical isomer, or the pharmaceutically acceptable salt according to any one of claims 1 to 12.
